# EUROPÄISCHE PATENTSCHRIFT

(11) **EP 1 311 471 B1**
(45) Veröffentlichungstag und Bekanntmachung des Hinweises auf die Patenterteilung: **12.04.2006**
(21) Anmeldenummer: 01971870.9
(22) Anmeldetag: 04.08.2001
(51) Int. Cl.: C07C 217/48, C07C 211/35, C07C 211/27, C07D 295/092, C07D 295/125, A61K 31/137, A61K 31/14, A61K 31/40, A61K 31/4453, A61P 9/06, A61P 25/28

(54) **PHENYL-UND PHENYLALKYL-SUBSTITUIERTE ETHANOLAMINE UND ETHYLENDIAMINE**
PHENYL- AND PHENYLALKYL-SUBSTITUTED ETHANOLAMINES AND ETHYLENEDIAMINES
ETHANOLAMINES ET ETHYLENEDIAMINES A SUBSTITUTION PHENYLE ET PHENYLALKYLE

(30) Priorität: 18.08.2000 DE 10040901
(43) Veröffentlichungstag der Anmeldung: 21.05.2003
(73) Patentinhaber: Boehringer Ingelheim Pharma GmbH & Co. KG, 55216 Ingelheim am Rhein (DE)
(72) Erfinder: FUCHS, Klaus, 88433 Schemmerhofen (DE); STRANSKY, Werner, 55435 Gau-Algesheim (DE); GRAUERT, Matthias, 88400 Biberach (DE); CARTER, Adrian, 55411 Bingen am Rhein (DE); GAIDA, Wolfram, 55218 Ingelheim am Rhein (DE); WEISER, Thomas, 55268 Nieder-Olm (DE); ENSINGER, Helmut, 55218 Ingelheim am Rhein (DE)
(74) Vertreter: Weymann, Markus
(86) Internationale Anmeldenummer: PCT/EP2001/009036
(87) Internationale Veröffentlichungsnummer: WO 2002/016308

(56) Entgegenhaltungen:
- WO-A-94/24116
- WO-A-97/27169
- CHEMICAL ABSTRACTS, vol. 113, no. 5, 30. Juli 1990 (1990-07-30) Columbus, Ohio, US; abstract no. 40077, CESARE GENNARI ET AL.: "Auxiliary structure and asymmetric induction in the Mukaiyama-aldol reactions of chiral silyl ketene acetals" XP002185088 & TETRAHEDRON LETT., Bd. 30, Nr. 38, 1989, Seiten 5163-6,
- CHEMICAL ABSTRACTS, vol. 121, no. 19, 7. November 1994 (1994-11-07) Columbus, Ohio, US; abstract no. 221698, S. J. HONG ET AL: "Inhibition of the sodium channel by SK&F 96365, an inhibitor of the receptor-operated calcium channel, in mouse diaphragm" XP002185089 & J. BIOMED. SCI. , Bd. 1, Nr. 3, 1994, Seiten 172-8,

## Beschreibung

Die vorliegende Patentanmeldung betrifft neue Verbindungen der allgemeinen Formel **1**, 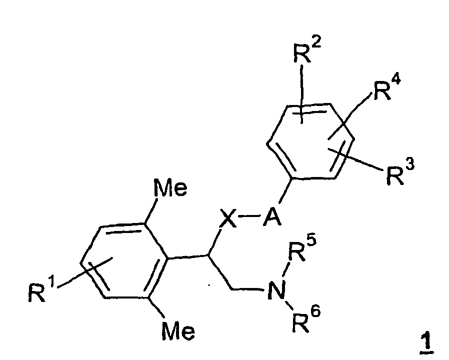 worin die Reste A, X, R¹, R², R³, R⁴, R⁵ und R⁶ die in der Beschreibung und die in den Ansprüchen genannte Bedeutung haben können, Verfahren zu ihrer Herstellung und ihre Verwendung als Arzneimittel, insbesondere als Arzneimittel zur Vorbeugung oder Therapie von Erkrankungen, deren Ursache auf eine durch Übererregung bedingte Funktionsstörung beruht.

### Hintergrund der Erfindung

Es ist Aufgabe der vorliegenden Erfindung, neue Verbindungen bereitzustellen, die als Blocker des spannungsabhängigen Natriumkanals Verwendung finden können. Solche Verbindungen können bei Erkrankungen, deren Ursache auf eine durch Übererregung bedingte Funktionsstörung beruhen eingesetzt werden. Darunter fallen Erkrankungen wie Arrhythmien, Spasmen, kardiale und Gehimischämien, Schmerzen sowie neurodegenerative Erkrankungen verschiedener Genese. Als Beispiele seien genannt: Epilepsie, Hypoglykämie, Hypoxie, Anoxie, Gehirntrauma, Gehirnoedem, Gehirn-Schlaganfall, perinatale Asphyxie, Degenerationen des Cerebellums, amyotrophe laterale Sklerose, Morbus Huntington, Morbus Alzheimer, Morbus Parkinson, Zyklophrenie, Hypotonie, Herzinfakt, Herzrhythmusstörungen, Angina pectoris, chronischer Schmerz, neuropathischer Schmerz sowie Lokalanaesthesie.

### Detaillierte Beschreibung der Erfindung

Die vorstehend genannte Aufgabe wird durch die in der nachstehenden Beschreibung offenbarten Verbindungen der allgemeinen Formel **1** gelöst.

Die vorliegende Patentanmeldung betrifft neue Verbindungen der allgemeinen Formel **1**, 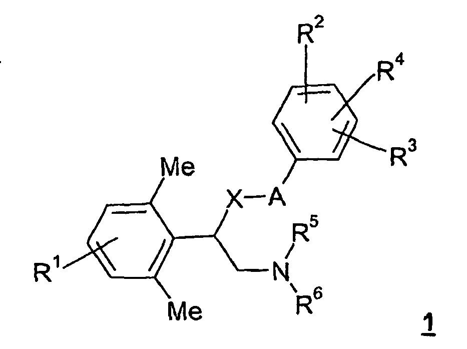 worin
- R¹: Wasserstoff, Hydroxy, CF₃, NO₂, CN, Halogen, C₁-C₈-Alkyl oder C₁-C₈-Alkoxy;
- R², R³ und R⁴: unabhängig voneinander Wasserstoff, C₁-C₈-Alkyl, Hydroxy, NO₂ CN, C₁-C₈-Alkyloxy, CF₃ oder Halogen;
- R⁵ und R⁶: unabhängig voneinander Wasserstoff oder ein Rest ausgewählt aus der Gruppe bestehend aus C₁-C₈-Alkyl, C₂-C₈-Alkenyl, C₃-C₈-Alkinyl, C₃-C₈-Cycloalkyl, C₃-C₈-Cycloalkyl-C₁-C₆-alkylen, C₅-C₈-Cycloalkenyl, C₅-C₈-Cycloalkenyl-C₁-C₆-alkylen, C₆-C₁₀-Aryl und
C₆-C₁₀-Aryl-C₁-C₆-alkylen, der gegebenenfalls durch einen Rest ausgewählt aus der Gruppe bestehend aus C₁-C₆-Alkyl, C₂-C₆-Alkenyl, Halogen, C₁-C₆-Alkyloxy, -NH₂, -NH(C₁-C₄-Alkyl), -N(C₁C₄-Alkyl)₂, Hydroxy, =O, -COOH, -CO-OC₁-C₄-alkyl, -CONH₂, -CONH(C₁-C₄-Alkyl), -CON(C₁-C₄-alkyl)₂ und CF₃ substituiert sein kann, oder
R⁵ und R⁶ gemeinsam mit dem Stickstoffatom einen gesättigten oder ungesättigten 5-, 6-, 7- oder 8-gliedrigen Heterocyclus, der gegebenenfalls ein oder zwei weitere Heteroatome ausgewählt aus Schwefel, Sauerstoff und Stickstoff enthält und gegebenenfalls ein-, zwei- oder dreifach durch einen Rest ausgewählt aus C₁-C₄-Alkyl, Hydroxy, =O, -COOH, -CO-OC₁-C₄-alkyl, -CONH₂, -CONH(C₁-C₄-Alkyl), -CON(C₁-C₄-alkyl)₂, Halogen und Benzyl substituiert sein kann;
- X: Sauerstoff, -NH-, -N(CHO)-, -N(CO-C₁-C₆-Alkyl)-, -N(C₁-C₆-Alkyl)- oder -N(C₃-C₆-Cycloalkyl-C₁-C₄-alkylen)-, bevorzugt Sauerstoff oder -NH-;
- A: ein Rest ausgewählt aus C₁-C₆-Alkylen, C₂-C₆-Alkenylen und C₃-C₆-Alkinylen, der gegebenenfalls durch einen Rest ausgewählt aus Halogen, =O und Hydroxy substituiert sein kann, bedeuten.

Bevorzugt sind Verbindungen der allgemeinen Formel **1** , worin
- R¹: Wasserstoff, Halogen, C₁-C₆-Alkyl, CF₃ oder Methoxy;
- R², R³ und R⁴: unabhängig voneinander Wasserstoff, C₁-C₆-Alkyl, C₁-C₆-Alkyloxy, CF₃ oder Halogen;
- R⁵ und R⁶: unabhängig voneinander Wasserstoff oder ein Rest ausgewählt aus der Gruppe bestehend aus C₁-C₆-Alkyl, C₂-C₆-Alkenyl, C₃-C₆-Alkinyl, C₃-C₆-Cycloalkyl, C₃-C₆-Cycloalkyl-C₁-C₆-alkylen, C₅-C₆-Cycloalkenyl, C₅-C₆-Cycloalkenyl-C₁-C₆-alkylen, Phenyl und Phenyl-C₁-C₆-alkylen, der gegebenenfalls durch einen Rest ausgewählt aus der Gruppe C₁-C₄-Alkyl, C₂-C₄-Alkenyl, Halogen, C₁-C₄-Alkyloxy, Hydroxy, -CONH₂, =O und CF₃ substituiert sein kann
oder
R⁵ und R⁶ gemeinsam mit dem Stickstoffatom einen gesättigten oder ungesättigten 5-, 6- oder 7-gliedrigen Heterocyclus, der gegebenenfalls ein oder zwei weitere Heteroatome ausgewählt aus Schwefel, Sauerstoff und Stickstoff enthält und gegebenenfalls ein-, zwei- oder dreifach durch C₁-C₄-Alkyl, -CONH₂ oder Hydroxy substituiert sein kann;
- X: Sauerstoff, -NH-, -N(CHO)-, -N(CO-C₁-C₅-Alkyl)-, -N(C₁-C₅-Alkyl)- oder -N(C₃-C₆-Cycloalkyl-C₁-C₄-alkylen)-, bevorzugt Sauerstoff oder -NH-;
- A: C₁-C₅-Alkylen, C₂-C₄-Alkenylen oder C₃-C₄-Alkinylen, bevorzugt C₁-C₅-Alkylen, bedeuten

Besonders bevorzugt sind Verbindungen der allgemeinen Formel **1**, worin
- R¹: Wasserstoff, C₁-C₄-Alkyl oder CF₃;
- R², R³ und R⁴: unabhängig voneinander Wasserstoff, C₁-C₄-Alkyl, CF₃ oder Halogen;
- R⁵ und R⁶: unabhängig voneinander Wasserstoff, C₁-C₆-Alkyl, CF₃-C₁-C₆-Alkylen, bevorzugt ausgewählt aus -CH₂-CF₃ und -CH₂CH₂-CF₃, C₂-C₆-Alkenyl, C₃-C₆-Alkinyl, C₃-C₆-Cycloalkyl, C₃-C₆-Cycloalkyl-C₁-C₆-alkylen, bevorzugt Cyclopropylmethyl oder Cyclohexerimethyt, Cyclohexenyl, Cyclohexenyl-C₁-C₆-alkylen, Propenyl-cyclohexenylen-C₁-C₆-alkylen, Phenyl, Phenyl-C₁-C₆-alkylen oder
R⁵ und R⁶ gemeinsam mit dem Stickstoffatom einen gesättigten oder ungesättigten 5-, 6- oder 7-gliedrigen Heterocyclus, der gegebenenfalls ein weiteres Stickstoffatom enthält und gegebenenfalls ein-, zwei-oder dreifach durch C₁-C₄-Alkyl, -CONH₂ oder Hydroxy substituiert sein kann;
- X: Sauerstoff, -NH-, -N(CHO)-, -N(CO-Methyl)-, -N(CO-Ethyl)-, -N(C₁-C₅-Alkyl)- oder -N(C₃-C₆-cycloalkyl-methylen)-, bevorzugt Sauerstoff oder -NH-;
- A: -CH₂-, -CH₂-CH₂- oder -CH₂-CH₂-CH₂- bedeuten.

Besonders bevorzugt sind ferner Verbindungen der allgemeinen Formel **1**, worin
- R¹: Wasserstoff oder Methyl;
- R² und R³: unabhängig voneinander Wasserstoff, Methyl, Fluor, Chlor oder Brom;
- R⁴: Wasserstoff, Fluor, Chlor oder Brom;
- R⁵ und R⁶: unabhängig voneinander Wasserstoff, C₁-C₆-Alkyl, CF₃-C₁-C₆-Alkylen, bevorzugt -CH₂CH₂-CF₃, C₂-C₆-Alkenyl, bevorzugt Butenyl und Pentenyl, C₃-C₆-Cycloalkyl, bevorzugt Cyclohexyl, C₃-C₆-Cycloalkyl-C₁-C₆-alkylen, bevorzugt Cyclopropylmethyl oder Cyclohexenmethyl, Cyclohexenyl, Cyclohexenyl-C₁-C₆-alkylen, bevorzugt Cyclohexenyl-CH₂-,
oder
R⁵und R⁶ gemeinsam mit dem Stickstoffatom einen Heterocyclus ausgewählt aus der Gruppe bestehend aus Pyrrolidin, Piperidin, 1.2.3.6-Tetrahydropyridin und Azepan;
- X: Sauerstoff, -NH-, -N(CHO)-, -N(CO-Methyl)-, -N(CO-Ethyl)-, -N(Methyl)-, -N(Ethyl)-, -N(Propy)-, -N(Butyl)-, -N(Pentyl)- oder -N(Cyclopropylmethyl)-, bevorzugt Sauerstoff oder -NH-;
- A: -CH₂-, -CH₂-CH₂- oder -CH₂-CH₂-CH₂- bedeuten.

Erfindungsgemäß von besonderer Bedeutung sind Verbindungen der allgemeinen Formel **1**, worin
- R¹: Wasserstoff oder Methyl;
- R² und R³: unabhängig voneinander Wasserstoff, Methyl, Fluor, Chlor oder Brom;
- R⁴: Wasserstoff, Fluor, Chlor oder Brom;
- R⁵ und R⁶: unabhängig voneinander Wasserstoff, Methyl, Propyl, Butyl, Hexyl, Cyclopropylmethyl oder Cyclohexenmethyl, oder
R⁵ und R⁶ gemeinsam mit dem Stickstoffatom einen Heterocyclus ausgewählt aus der Gruppe bestehend aus Pyrrolidin, Piperidin, 1.2.3.6-Tetrahydropyridin und Azepan;
- X: Sauerstoff, -NH-, -N(CHO)-, -N(CO-Methyl)-, -N(CO-Ethyl)-, -N(Ethyl)-, -N(Propyl)-, -N(Butyl)-, -N(Pentyl)- oder -N(Cyclopropylmethyl)-, bevorzugt Sauerstoff oder -NH-;
- A: -CH₂-, -CH₂-CH₂- oder -CH₂-CH₂-CH₂- bedeuten.

Erfindungsgemäß von herausragender Bedeutung sind Verbindungen der allgemeinen Formel **1**, worin
- R¹: Wasserstoff oder Methyl;
- R² und R³: unabhängig voneinander Wasserstoff oder Fluor;
- R⁴: Wasserstoff;
- R⁵ und R⁶: unabhängig voneinander Wasserstoff, Methyl, Butyl, Hexyl oder Cyclohexenmethyl, oder
R⁵ und R⁶ gemeinsam mit dem Stickstoffatom Piperidin und 1.2.3.6-Tetrahydropyridin;
- X: Sauerstoff oder -NH-;
- A: -CH₂-CH₂- oder -CH₂-CH₂-CH₂- bedeuten.

Verbindungen der allgemeinen Formel **1**, in denen R¹ Wasserstoff bedeutet, in denen R² und R³ in ortho-Position angeordnet sind und in denen X, A, R², R³, R⁴, R⁵ und R⁶ die vorstehend genannten Bedeutungen haben können, entsprechen der allgemeinen Formel **1'**.

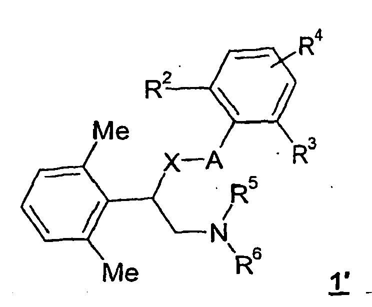

Diese Verbindungen sind erfindungsgemäß besonders bedeutsam.

Verbindungen der allgemeinen Formel **1**, in denen R¹ Methyl bedeutet und in para-Position angeordnet ist, in denen R² und R³ in ortho-Position angeordnet sind und in denen X, A, R², R³, R⁴, R⁵ und R⁶ die vorstehend genannten Bedeutungen haben können, entsprechen der allgemeinen Formel **1"**.

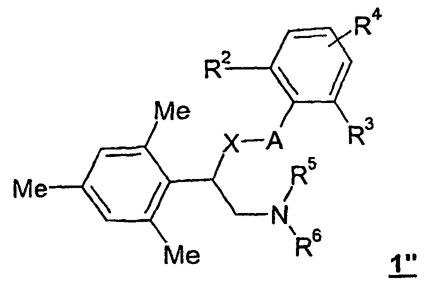

Diese Verbindungen sind erfindungsgemäß besonders bedeutsam. Von besonderer Bedeutung sind die Verbindungen der allgemeinen Formeln **1**, **1'** und **1"**, bei denen R⁴ für Wasserstoff steht.

Gegenstand der Erfindung sind die jeweiligen Verbindungen der Formel **1** gegebenenfalls in Form der einzelnen optischen Isomeren, Mischungen der einzelnen Enantiomeren oder Racemate sowie in Form der freien Basen oder der entsprechenden Säureadditionssalze mit pharmakologisch unbedenklichen Säuren - wie beispielsweise Säureadditionssalze mit Halogenwasserstoffsäuren - beispielsweise Chlor- oder Bromwasserstoffsäure - oder organische Säuren - wie z.B. Oxal-, Fumar- oder Diglycolsäure oder Methansulfonsäure.

Die vorliegende Erfindung betrifft ferner quartäre Ammoniumverbindungen wie sie von den Verbindungen der Formel **1** mit Alkylhalogeniden der Formel R⁷-X gebildet werden können. Entsprechend sind erfindungsgemäß ferner von Bedeutung die quartären Ammoniumverbindungen der Formel **1-Y** 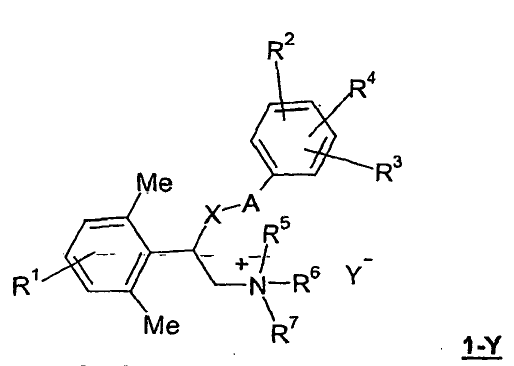 worin die Reste A, X, R¹, R², R³ und R⁴die vorstehend genannten Bedeutungen haben können, R⁵ und R⁶ die vorstehend genannten Bedeutungen, aber nicht Wasserstoff, haben können, R⁷ C₁-C₄-Alkyl, bevorzugt Methyl oder Ethyl bedeutet und Y für ein Halogenid ausgewählt aus der Gruppe Chlor, Brom und Jod steht.

Verbindungen der allgemeinen Formel **1-Y**, in denen R¹ Wasserstoff bedeutet, in denen R² und R³ in ortho-Position angeordnet sind und in denen X, A, R², R³, R⁴, R⁵, R⁶ und R⁷ die vorstehend genannten Bedeutungen haben können, entsprechen der allgemeinen Formel **1'-Y**.

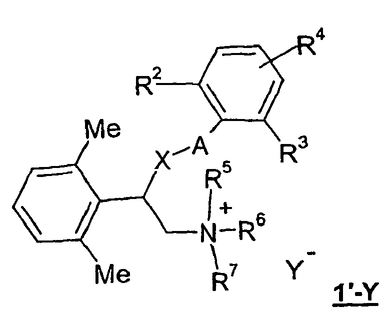

Diese Verbindungen sind erfindungsgemäß besonders bedeutsam.

Verbindungen der allgemeinen Formel **1-Y**, in denen R¹ Methyl bedeutet und in para-Position angeordnet ist, in denen R² und R³ in ortho-Position angeordnet sind und in denen X, A, R², R³, R⁴, R⁵ und R⁶ die vorstehend genannten Bedeutungen haben können, entsprechen der allgemeinen Formel **1"-Y**.

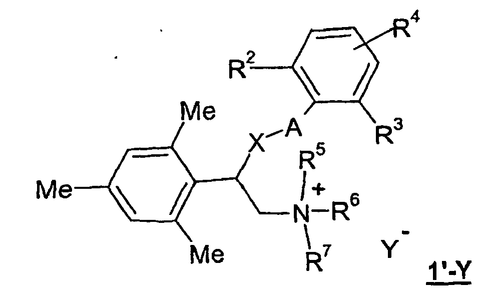

Diese Verbindungen sind ebenfalls erfindungsgemäß bedeutsam.

Von besonderer Bedeutung sind die Verbindungen der allgemeinen Formeln **1-Y**, **1'-Y** und **1"-Y**, bei denen R⁴ für Wasserstoff steht.

Als erfindungsgemäß von besonderem Interesse seien u.a. die folgenden Verbindungen genannt:
- N-[2-[3-(2,6-Difluorphenyl)-propoxy]-2-(2,6-dimethylphenyl)-ethyl]-N-n-butyl-amin;
- N-[[2-[3-(2,6-Difluorphenyl)-propoxy]-2-(2,6-dimethylphenyl)-ethyl]-N-(2-ethylbutyl)-N,N-dimethyl-ammoniumiodid;
- 1-[2-[3-(2,6-Difluorphenyl)-propoxy]-2-(2,6-dimethylphenyl)-ethyl]-pyrrolidin;
- N-[2-[3-(2,6-Difluorphenyl)-propoxy]-2-(2,6-dimethylphenyl)-ethyl]-N-(4-penten-1-yl)-amin;
- N-[2-[3-(2,6-Difluorphenyl)-propoxy]-2-(2,6-dimethylphenyl)-ethyl]-N-n-propyl-amin;
- N-[2-[3-(2,6-Difluorphenyl)-propoxy]-2-(2,4,6-trimethylphenyl)-ethyl]-N,N-dimethyl-amin;
- 1-[2-[3-(2,6-Difluorphenyl)-propoxy]-2-(2,6-dimethylphenyl)-ethyl]-piperidin;
- N-[2-[3-(2,6-Difluorphenyl)-propoxy]-2-(2,6-dimethylphenyl)-ethyl]-N-n-butyl-N,N-dimethyl-ammoniumiodid;
- N-[2-[3-(2,6-Difluorphenyl)-propoxy]-2-(2,6-dimethylphenyl)-ethyl]-N-(1-cyclohexen-4-yl-methyl)-N,N-dimethyl-ammoniumiodid;

Soweit nicht im einzelnen abweichende Angaben gemacht werden, werden die allgemeinen Definitionen im folgenden Sinn gebraucht:

Als Alkylgruppen (auch soweit sie Bestandteil anderer Reste sind) werden, soweit nicht anders definiert, verzweigte und unverzweigte Alkylgruppen mit 1 bis 8, bevorzugt 1 bis 6, besonders bevorzugt 1 bis 4 Kohlenstoffatomen betrachtet, die in einigen Resten wie in den Ansprüchen definiert gegebenenfalls mit einem oder mehreren Halogenatom(en) - vorzugsweise Fluorsubstituiert sein können. Als Beispiele seien folgende Kohlenwasserstoffreste genannt:
Methyl, Ethyl, Propyl, 1-Methylethyl (Isopropyl), n-Butyl, 1-Methylpropyl, 2-Methylpropyl, 1,1-Dimethyfethyl, Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl,-1,1,2-Trimethylpropyl, 1,2,2-Trimethylpropyl, 1-Ethyl-1-methylpropyl und 1-Ethyl-2-methylpropyl. Bevorzugt sind - sofern nicht anders angegeben - Niederalkylreste mit 1 bis 4 Kohlenstoffatomen, wie Methyl, Ethyl, n-Propyl, iso-Propyl, n-Butyl, 1-Methylpropyl, 2-Methylpropyl oder 1,1-Dimethylethyl. Dabei sind von den Definitionen Propyl, Butyl, Pentyl etc. die jeweiligen isomeren Reste stets mit umfaßt. Gegebenenfalls werden für die vorstehend genannten Alkylgruppen auch die gängigen Abkürzungen Me für Methyl, Et für Ethyl, Prop für Propyl, But für Butyl etc. verwendet.

Als Alkylengruppen werden verzweigte und unverzweigte Alkylenbrücken mit 1 bis 6, bevorzugt 1 bis 4 Kohlenstoffatomen betrachtet. Beispielsweise werden genannt: Methylen, Ethylen, Propylen und Butylen etc. Sofern nicht anders genannt, sind von den vorstehend genannten Bezeichnungen Propylen, Butylen etc. sämtliche der möglichen isomeren Formen umfaßt. Dementsprechend umfaßt die Bezeichnung Propylen die isomeren Brücken n-Propylen, Methylethylen und Dimethylmethylen und die Bezeichnung Butylen die isomeren Brücken n-Butylen, 1-Methylpropylen, 2-Methylpropylen, 1.1-Dimethylethylen und 1.2-Dimethylethylen.

Cycloalkyl steht im allgemeinen für einen gesättigten cyclischen Kohlenwasserstoffrest mit 3 bis 8 Kohlenstoffatomen, der in einigen Resten wie in den Ansprüchen definiert gegebenenfalls mit einem Halogenatom oder mehreren Halogenatomen - vorzugsweise Fluor - substituiert sein kann, die untereinander gleich oder verschieden sein können. Bevorzugt sind cyclische Kohlenwasserstoffe mit 3 bis 6 Kohlenstoffatomen. Als Beispiele seien Cyclopropyl, Cyclobutyl, Cyclopentyl, Cyclohexyl, Cycloheptyl und Cyclooctyl genannt.

Alkenyl verkörpert im allgemeinen einen verzweigten oder unverzweigten Kohlenwasserstoffrest mit 2 bis 8, bevorzugt 2 bis 6, besonders bevorzugt 2 bis 4 Kohlenstoffatomen, der eine oder mehrere Doppelbindungen aufweisen kann und der in einigen Resten wie in den Ansprüchen definert gegebenenfalls durch ein oder mehrere Halogenatome - vorzugsweise Fluor - substituiert sein kann, wobei die Halogene untereinander gleich oder verschieden sein können. Folgende Alkenylreste seien beispielhaft genannt:
Vinyl, 2-Propenyl (Allyl), 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-Pentenyl, 4-Methyl-2-pentenyl, 1-Methyl-3-pentenyl, 2-Methyl-3-pentenyl, 3-Methyl-3-Pentenyl, 4-Methyl-3-pentenyl, 1-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-2-butenyl, 1,2-Dimethyl-3-butenyl, 1,3-Dimethyl-2-butenyl, 1,3-Dimethyl-3-butenyl, 2,2-Dimethyl-3-butenyl, 2,3-Dimethyl-2-butenyl, 2,3-Dimethyl-3-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl und 1-Ethyl-2-methyl-2-propenyl etc.

Cycloalkenyl steht im allgemeinen für einen cyclischen Kohlenwasserstoffrest mit 5 bis 8 Kohlenstoffatomen, der wenigstens eine Doppelbindung enthält und der in einigen Resten wie in den Ansprüchen definiert gegebenenfalls mit einem Halogenatom oder mehreren Halogenatomen - vorzugsweise Fluor - substituiert sein kann, die untereinander gleich oder verschieden sein können. Bevorzugt sind im allgemeinen Cyclopentenyl oder Cyclohexenyl, wobei diese Reste, wenn nicht anders angegeben durch C₁-C₄-Alkyl, C₂-C₄-Alkenyl substituiert sein können.

Alkinyl verkörpert im allgemeinen einen verzweigten oder unverzweigten Kohlenwasserstoffrest mit 3 bis 8, bevorzugt 3 bis 6, besonders bevorzugt 3 bis 5 Kohlenstoffatomen, der eine oder mehrere Dreifachbindungen aufweisen kann und der in einigen Resten wie in den Ansprüchen definiert gegebenenfalls durch ein oder mehrere Halogenatome - vorzugsweise Fluor - substituiert sein kann, wobei die Halogene untereinander gleich oder verschieden sein können. Folgende Alkinylreste seien beispielhaft genannt:
Ethinyl, 2-Propinyl (Propargyl), 2-Butinyl, 3-Butinyl, 1-Methyl-2-propinyl, 2-Methyl-2-propinyl, 2-Pentinyl, 3-Pentinyl, 4-Pentinyl, 1-Methyl-2-butinyl, 2-Methyl-2-butinyl, 3-Methyl-2-butinyl, 1-Methyl-3-butinyl, 2-Methyl-3-butinyl, 3-Methyl-3-butinyl, 1,1-Dimethyl-2-propinyl, 1,2-Dimethyl-2-propinyl-, 1-Ethyl-2-propinyl, 2-Hexinyl, 3-Hexinyl, 4-Hexinyl, 5-Hexinyl, 1-Methyl-2-pentinyl, 2-Methyl-2-pentinyl, 3-Methyl-2-Pentinyl, 4-Methyl-2-pentinyl, 1-Methyl-3-pentinyl, 2-Methyl-3-pentinyl, 3-Methyl-3-Pentinyl, 4-Methyl-3-pentinyl, 1-Methyl-4-pentinyl, 3-Methyl-4-pentinyl, 4-Methyl-4-pentinyl, 1,1-Dimethyl-2-butinyl, 1,1-Dimethyl-3-butinyl, 1,2-Dimethyl-2-butinyl, 1,2-Dimethyl-3-butinyl, 1,3-Dimethyl-2-butinyl, 1,3-Dimethyl-3-butinyl, 2,2-Dimethyl-3-butinyl, 1-Ethyl-2-butinyl, 1-Ethyl-3-butinyl, 2-Ethyl-1-butinyl, 2-Ethyl-2-butinyl, 2-Ethyl-3-butinyl, 1,1,2-Trimethyl-2-propinyl, 1-Ethyl-1-methyl-2-propinyl etc.

Alkyloxy, welches gegebenenfalls auch als Alkoxy bezeichnet werden kann, steht im allgemeinen für einen über ein Sauerstoffatom gebundenen geradkettigen oder verzweigten Kohlenwasserstoffrest mit 1 bis 6 Kohlenstoffatmen - bevorzugt ist ein Niederalkoxyrest mit 1 bis 4 Kohlenstoffatom(en). Besonders bevorzugt ist die Methoxygruppe.

Der Begriff Aryl steht für ein aromatisches Ringsystem mit 6 bis 10 Kohlenstoffatomen. Bevorzugter Arylrest ist, soweit nicht anders beschrieben, Phenyl.

Unter Cycloalkyl-alkylen werden im Sinne der Erfindung Cycloalkylgruppen verstanden, die über eine Alkylenbrücke verknüpft sind. Unter Cycloalkenyl-alkylen werden im Sinne der Erfindung Cycloalkenylgruppen verstanden, die über eine Alkylenbrücke verknüpft sind. Unter Aryl-alkylen werden im Sinne der Erfindung Arylgruppen verstanden, die über eine Alkylenbrücke verknüpft sind.

Als Beispiele für N-verknüpfte 5-, 6-, 7- oder 8-gliedrige, gesättigte oder ungesättigte heterocyclische Reste die gemeinsam mit dem Stickstoffatom durch die Reste R⁵ und R⁶ gebildet werden können, werden genannt: Pyrrol, Pyrrolin, Pyrrolidin, 1,2,3,6-Tetrahydropyridin, Piperidin, Piperazin, Morpholin, Thiomorpholin, Imidazol, Imidazolin, Imidazolidin, Pyrazol, Pyrazolin, Pyrazolidin, Azepan, Azepin, Diazepin, etc., bevorzugt Pyrrolidin, Piperidin, 1,2,3,6-Tetrahydropyridin und Azepan.

Die beanspruchten Verbindungen sind Blocker des spannungsabhängigen Natriumkanals. Es handelt sich dabei um Verbindungen, die Batrachotoxin (BTX) mit hoher Affinität (Kᵢ < 1000 nM) kompetitiv oder nicht-kompetitiv von der Bindungsstelle am Natriumkanal verdrängen. Solche Substanzen zeigen eine "usedependency" bei der Blockade der Natriumkanäle, d.h. für die Bindung der Substanzen an den Natriumkanal müssen die Natriumkanäle zunächst aktiviert werden. Die maximale Blockade der Natriumkanäle wird erst nach wiederholter Stimulation der Natriumkanäle erreicht. Demzufolge binden die Substanzen bevorzugt an Natriumkanäle, die vermehrt aktiviert werden. Dadurch sind die Substanzen in der Lage, bevorzugt in den Körperregionen wirksam zu werden, die pathologisch überstimuliert sind. Die erfindungsgemäßen Verbindungen der allgemeinen Formel **1** können somit bei Erkrankungen, deren Ursache auf eine durch Übererregung bedingte Funktionsstörung beruhen, eingesetzt werden. Darunter fallen Erkrankungen wie Arrhythmien, Spasmen, kardiale und Gehimischämien, Schmerzen sowie neurodegenerative Erkrankungen verschiedener Genese. Als Beispiele seien genannt: Epilepsie, Hypoglykämie, Hypoxie, Anoxie, Gehirntrauma, Gehirnoedem, Gehirn-Schlaganfall, perinatale Asphyxie, Degenerationen des Cerebellums, amyotrophe laterale Sklerose, Morbus

Huntington, Morbus Alzheimer, Morbus Parkinson, Zyklophrenie, Hypotonie, Herzinfakt, Herzrhythmusstörungen, Angina pectoris, chronischer Schmerz, neuropathischer Schmerz sowie Lokalanaesthesie.

Als Testsystem für den Nachweis der Natriumkanal blockierenden Wirkung dient die BTX-Bindung am Natriumkanal [S.W. Postma & W.A. Catterall, Mol. Pharmacol. 25, 219-227 (1984)] sowie patch-clamp Experimente, in denen gezeigt werden kann, daß die erfindungsgemäßen Verbindungen den elektrisch stimulierten Natriumkanal in einer "use-dependent" Art und Weise blockieren [W.A. Catterall, Trends Pharmacol. Sci., 8, 57-65 (1987)]. Durch die Auswahl des Zellsystems (z.B. neuronale, kardiale, DRG Zellen) kann die Wirkung der Substanzen auf verschiedene Natriumkanalsubtypen untersucht werden.
Die Natriumkanal blockierende Eigenschaft der erfindungsgemäßen Verbindungen kann durch die Blockade des Veratridin induzierten Glutamat-releases nachgewiesen werden [S. Villauneva, P. Frenz, Y. Dragnic, F. Orrego, Brain Res. 461, 377-380 (1988)]. Veratridin ist ein Toxin, das den Natriumkanal permanent öffnet. Dadurch kommt es zu einem erhöhten Einstrom von Natriumionen in die Zelle. Über die oben beschriebene Kaskade führt dieser Natriumeinstrom in neuronalem Gewebe zu einer gesteigerten Freisetzung von Glutamat. Durch die erfindungsgemäßen Verbindungen läßt sich diese Glutamatfreisetzung antagonisieren.
Antikonvulsive Eigenschaften der erfindungsgemäßen Substanzen wurden durch eine protektive Wirkung gegen Krämpfe, die durch einen maximalen Elektroschock in Mäusen ausgelöst wurden, belegt [M. A. Rogawski & R.J. Porter, Pharmacol. Rev. 42, 223-286 (1990)].
Neuroprotektive Eigenschaften wurden durch protektive Wirkung in einem Ratten-MCAO-Modell [U. Pschorn & A. J. Carter, J. Stroke, Cerebrovascular Diseases, 6, 93-99 (1996)] sowie einem Malonat induziertem Läsionsmodel [M. F. Beal, Annals of Neurology, 38, 357-366 (1995) und J.B. Schulz, R.T. Matthews, D.R. Henshaw und M.F. Beal, Neuroscience, 71, 1043-1048 (1996)] belegt.
Analgetische Wirkung lassen sich in Modellen der diabetischen Neuropathie sowie in einem Ligatur-Modell belegen [C. Courteix, M. Bardin, C. Chantelauze, J. Lavarenne, A. Eschalier, Pain 57, 153-160 (1994); C. Courteix, A. Eschalier, J. Lavarenne, Pain 53, 81-88 (1993); G. J. Bennett & Y.-K. Xie, Pain 33, 87-107(1988)].
Ferner wurde beschrieben, daß Natriumkanalblocker zur Therapie der Zyklophrenie (manisch depressive Erkrankung) eingesetzt werden können [J. R. Calabrese, C. Bowden, M.J. Woyshville; in: Psychopharmacology: The Fourth Generation of Progress (Eds.: D. E. Bloom and D. J. Kupfer) 1099-1111. New York: Raven Press Ltd.].

Die beanspruchten Verbindungen **1** lassen sich nach an sich aus dem Stand der Technik bekannten Verfahren herstellen. Exemplarische Synthesewege werden nachfolgend beschrieben.

Ausgehend von den Benzaldehyd-Derivaten der Formel **2** gelingt ein Zugang zu den Verbindungen der allgemeinen Formel **6** (entspricht Verbindungen der Formel **1**, in denen X für O und die Reste R⁵ und R⁶ für Wasserstoff stehen), nach der in Schema 1 dargestellten Vorgehensweise. 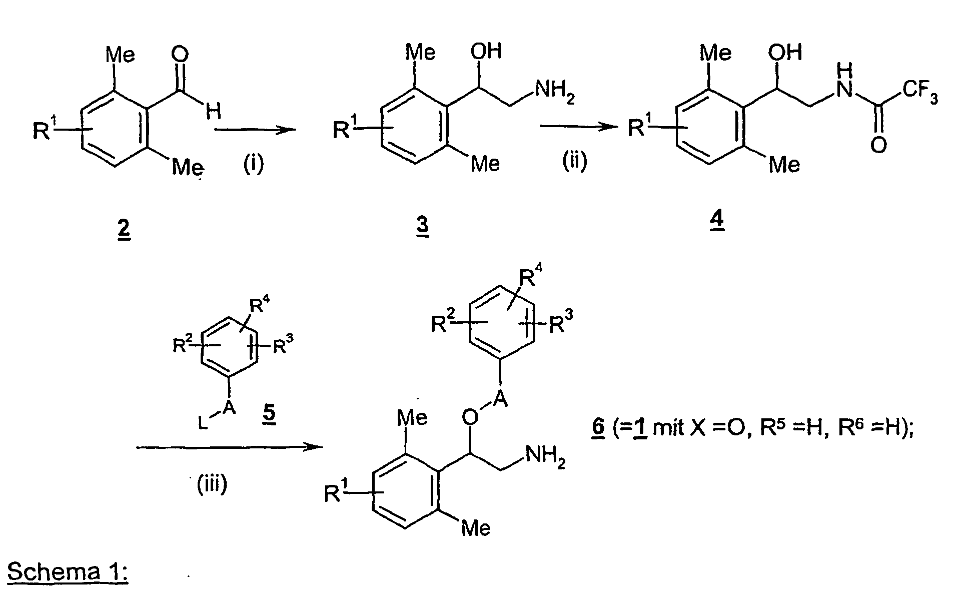 Ausgehend von den 2,6-Dimethylbenzaldehyd-Derivaten **2** gelingt gemäß Stufe (i) die Darstellung der 2-Amino-ethanole **3** zunächst durch Aufnehmen von **2** in Trimethylsilylcyanid in Gegenwart einer Lewissäure, bevorzugt in Gegenwart von Zinkiodid. Nach Mischen der vorstehend genannten Reaktanden, bevorzugt bei Raumtemperatur, wird mit einem wasserfreien organischen Lösemittel, bevorzugt mit einem etherischen organischen Lösemittel, besonders bevorzugt mit Diethylether, Tetrahydrofuran oder Dioxan verdünnt. Sodann erfolgt die Zugabe eines Reduktionsmittels, bevorzugt eines Metallhydrids, besonders bevorzugt eines Hydrids ausgewählt aus Lithiumaluminiumhydrid oder Natrium-bis-(2-methoxyethoxy)-aluminiumhydrid (Red-Al®). Zur Vervollständigung der Reaktion wird bei erhöhter Temperatur, besonders bevorzugt unter Rückfluß des verwendeten Lösemittels 0,5 bis 4, bevorzugt 2 Stunden gerührt. Die Aufarbeitung erfolgt auf üblichem Wege. Die Reinigung der Produkte erfolgt je nach Kristallisationsneigung durch Kristallisation oder mittels chromatographischer Methoden.

Auf der Stufe des Aminoalkohols **3** kann das Racemat gegebenenfalls in die Enantiomeren gespalten werden. Dabei kann die anschließende Aufspaltung des so erhaltenen Gemisches der enantiomeren Aminoalkohole vom Typ **3** auf den an sich aus dem Stand der Technik bekannten Wegen zur Enantiomerentrennung - beispielsweise durch Umsetzung mit Äpfelsäure, Weinsäure, Mandelsäure oder Campfersulfonsäure, worunter Weinsäure besonders bevorzugt ist, erfolgen.

Aus den so gegebenenfalls enantiomerenrein erhaltenen Verbindungen **3** gelingt die Darstellung der Trifluoracetate **4** (Stufe (ii)) wie folgt. Die Alkohole **3** werden in einem organischen Lösemittel, bevorzugt in einem wasserfreien organischen Lösemittel, besonders bevorzugt in einem Lösemittel ausgewählt aus der Gruppe Toluol, Ether, Dichlormethan, DMF und Essigsäurethylester gelöst und in Gegenwart einer organischen oder anorganischen Base bei Raumtemperatur oder unter Eiskühlung mit Trifluoressigsäureanhydrid versetzt und 1 bis 8, bevorzugt 2 bis 6, besonders bevorzugt ca. 4 Stunden gerührt. Als anorganische Base kommen die Alkali- oder die Erdalkalicarbonate des Lithiums, Natriums, Kaliums, Calziums wie Natriumcarbonat, Lithiumcarbonat, Kaliumcarbonat, Calziumcarbonat und bevorzugt Kaliumcarbonat in Betracht. Als organische Base kommen bevorzugt organische Amine, besonders bevorzugt Diisopropylethylamin, Triethylamin, cyclische Amine wie DBU, oder Pyridin in Betracht. Die vorstehend genannten Amine können gegebenenfalls auch als Lösungsmittel verwendet werden. Die Aufarbeitung erfolgt auf üblichem Wege. Die Reinigung der Produkte erfolgt je nach Kristallisationsneigung durch Kristallisation oder mittels chromatographischer Methoden.
Zur Darstellung der Verbindungen der Formel **6** (entspricht Verbindungen der Formel **1**, in denen X für O und die Reste R⁵ und R⁶ für Wasserstoff stehen) wird eine Verbindung **4** gemäß Stufe (iii) in einem organischen Lösemittel, bevorzugt in einem wasserfreien organischen Lösemittel, besonders bevorzugt in einem Lösemittel ausgewählt aus der Gruppe Toluol, Ether, Dichlormethan, DMF und Essigsäurethylester gelöst und in Gegenwart einer organischen Base, bevorzugt ausgewählt aus Diisopropylethylamin, Triethylamin, cyclischen Aminen wie DBU, und Pyridin bei Raumtemperatur oder in Gegenwart einer anorganischen Base, bevorzugt in Gegenwart von Alkali- oder Erdalkalicarbonaten des Lithiums, Natriums, Kaliums, Calziums wie Natriümcarbonat, Lithiumcarbonat, Kaliumcarbonat, Calziumcarbonat, oder in Gegenwart der Alkali- und Erdalkalihydride wie Natriumhydrid, Calcumhydrid oder Kaliumhydrid, oder in Gegenwart der Alkalialkoholate, bevorzugt Kaliumtertbutylat, Natriummethanolat oder Natriumethanolat, bei Raumtemperatur oder bevorzugt bei Temperaturen zwischen - 20°C und Raumtemberatur, besonders bevorzugt bei ca. 0°C mit einer Verbindung der Formel **5**, gegebenenfalls gelöst in einem der vorstehend genannten organischen Lösemittel, versetzt. Bei Verwendung von Natriumhydrid als Base kann die Verwendung chelatisierender Agentien wie Kronenethern, bevorzugt von 15-Krone-5 hilfreich sein. Zur Vervollständigung der Reaktion wird bei Raumtemperatur oder erhöhter Temperatur, bevorzugt bei Siedetemperatur des verwendeten Lösemittels 2 bis 24, bevorzugt 4 bis 12, besonders bevorzugt 6 bis 7 Stunden gerührt. Die Aufarbeitung erfolgt auf üblichem Wege. Die Reinigung der Produkte erfolgt je nach Kristallisationsneigung durch Kristallisation oder mittels chromatographischer Methoden.

Eine alternativer Zugang zu Verbindungen der allgemeinen Formel **6** (entspricht **1** in denen X für Sauerstoff steht und R⁵ und R⁶ Wasserstoff bedeuten), ausgehend von den Benzaldehyd-Derivaten der Formel **2** gelingt nach der in Schema 2 dargestellten Vorgehensweise. 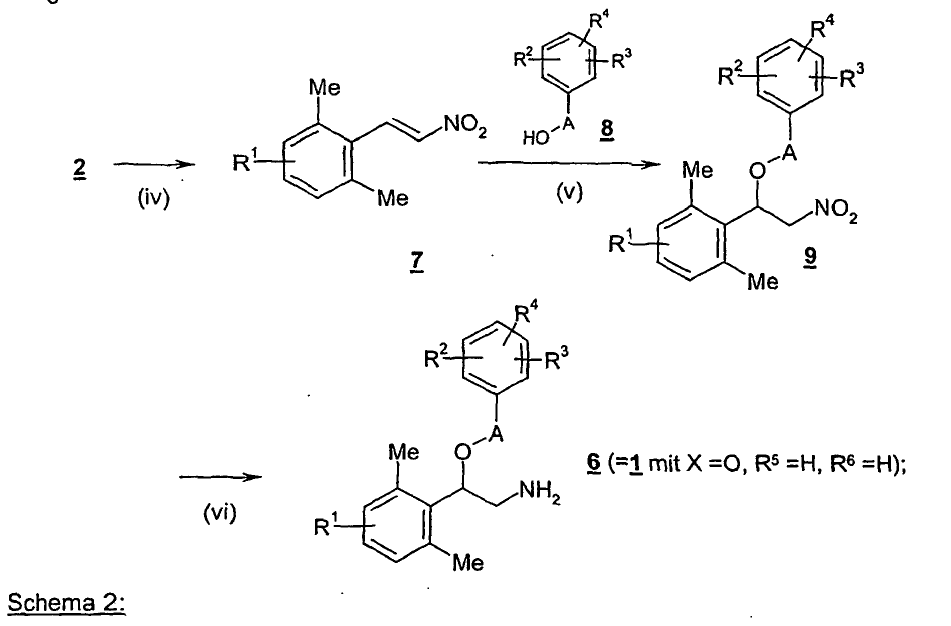 Ausgehend von den 2,6-Dimethylbenzaldehyd-Derivaten (**2**) gelingt gemäß Stufe (iv) die Umsetzung zu den α,β-ungesättigten Nitroverbindungen **7** mittels Nitromethan in Eisessig bei erhöhter Temperatur, bevorzugt bei mehr als 60°C, besonders bevorzugt mehr als 100°C, vorzugsweise bei etwa 120°C über einen Zeitraum von 2 bis 8, bevorzugt 3 bis 6, besonders bevorzugt ca. 4 Stunden. Die Aufarbeitung erfolgt auf üblichem Wege. Die Reinigung der Produkte erfolgt je nach Kristallisationsneigung durch Kristallisation oder mittels chromatographischer Methoden.

Aus den Nitroverbindungen **7** lassen sich durch Umsetzung mit den Alkoholen **8** die Ether **9** erhalten. Hierzu wird wie folgt verfahren. In einem organischen Lösemittel, bevorzugt in einem wasserfreien organischen Lösemittel ausgewählt aus der Gruppe Methylenchlorid, Tetrahydrofuran, Diethylether und Dioxan wird der Alkohol **8** gelöst und mit einer Base, ausgewählt aus den Alkalialkoholaten wie Natriumethanolat, Natriummethanolat oder Kaliumtertbutylat und den Alkali- oder Erdalkalimetallhydriden, vorzugsweise Natriumhydrid, versetzt. Es wird 6 bis 24, vorzugsweise etwa 10 bis 14 Stunden bei Raumtemperatur, gegebenenfalls auch bei leicht erhöhter Temperatur gerührt und anschließend eine Lösung der Nitroverbindung **7**, vorzugsweise in einem der vorstehend genannten Lösemittel gelöst, zugegeben. Es wird bis zur Vervollständigung der Reaktion bei konstanter Temperatur weiter gerührt. Die Aufarbeitung erfolgt auf üblichem Wege. Die Reinigung der Produkte erfolgt je nach Kristallisationsneigung durch Kristallisation oder mittels chromatographischer Methoden.

Die abschließende Reduktion von **9** führt zu den Verbindungen der Formel **6** (entspricht Verbindungen der Formel **1**, in denen X für O und die Reste R⁵ und R⁶ für Wasserstoff stehen). Diese Reduktion erfolgt vorzugsweise durch katalytische Hydrierung, bevorzugt an Palladiumkatalysatoren oder an Raney-Nickel in alkoholischen Lösemitteln, vorzugsweise in Methanol, bei Raumtemperatur. Die Aufarbeitung erfolgt auf üblichem Wege. Die Reinigung der Produkte erfolgt je nach Kristallisationsneigung durch Kristallisation oder mittels chromatographischer Methoden.

Die Darstellung der Verbindungen **1** ausgehend von den Aminen **6** gelingt wie auch die Synthese der Ammoniumsalze **1-Y** über Standardverfahren (Schema 3).

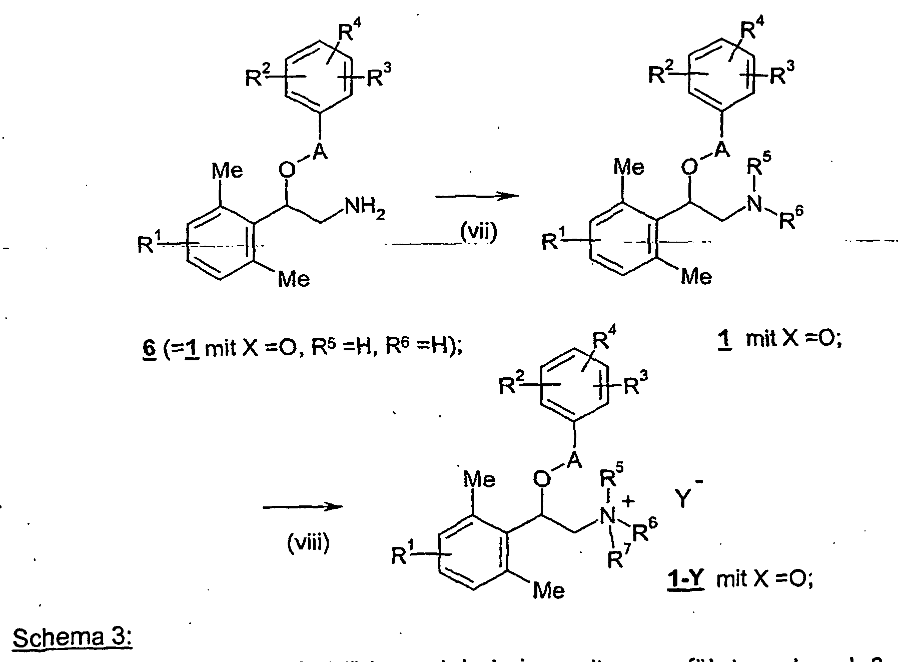

Die Umsetzung nach Stufe (vii) kann dabei einerseits so geführt werden, daß direkt tertiäre Amine der Formel **1**, bei denen sowohl R⁵ als auch R⁶ nicht Wasserstoff bedeuten, erhalten werden, oder kann durch Wahl der Reaktionsbedingungen zu sekundären Aminen der Formel **1** führen, bei denen entweder R⁵ oder R⁶ für Wasserstoff stehen. Letztere können dann einerseits durch eine wiederholte Durchführung von Stufe (vii) zu tertiären Aminen alkyliert werden, oder direkt der Stufe (viii) unterworfen werden, um so einen Zugang zu den Amoniumsalzen **1-Y** zu eröffnen.

Zur Durchführung des Verfahrens nach Stufe (vii) wird ein Amin der allgemeinen Formel **6** in einem organischen Lösemittel wie Dimethylformamid, Dimethylacetamid, Methylenchlorid, Tetrahydrofuran, bevorzugt Dimethylformamid und besonders bevorzugt wasserfreies, gegebenenfalls absolutes Dimethylformamid oder Methylenchlorid gelöst. Die so erhaltene Lösung wird mit einer anorganischen oder organischen Base und einem entsprechenden Alkylierungsmittel versetzt. Als Base kommen die Alkali- oder die Erdalkalicarbonate des Lithiums, Natriums, Kaliums, Catziums wie Natriumcarbonat, Lithiumcarbonat, Kaliumcarbonat, Calziumcarbonat und bevorzugt Kaliumcarbonat in Betracht. Ferner können die Hydrogencarbonate des Lithiums, Natriums und Kaliums eingesetzt werden. Ferner sind die Alkali- oder Erdalkalihydroxide des Lithiums, Natriums, Kaliums, Magnesiums, Calziums, bevorzugt jedoch Natriumhydroxid, Kaliumhydroxid, Lithiumhydroxid und Calziumhydroxid in Alkoholen oder Wasser einsetzbar. Als weitere Basen kommen Alkoholate der Alkali- und Erdalkalimetalle, bevorzugt die Ethylate des Natriums und Kaliums in Betracht. Weiterhin können Alkali- und Erdalkalihydride, vorzugsweise des Kaliums oder Natriums, bevorzugt in inerten Lösungsmitteln wie Dimethylformamid, Dimethylacetamid, Methylenchlorid, Ethern, Tetrahydrofuran und Toluol eingesetzt werden. Als organische Base kommen bevorzugt organische Amine, besonders bevorzugt Diisopropylethylamin, Triethylamin, cyclische Amine wie DBU, oder Pyridin in Betracht. Als Alkylierungsmittel kommen Alkylhalogenide, wie Alkylchlorid, Alkylbromid, besonders Alkyljodid sowie Alkyltosylate, -Mesylate, -Triflate, -Dialkylsulfate in Betracht. Die Alkylreste der Alkylierungsmittel entsprechen den zuvor getroffenen Definitionen für R⁵ und R⁶. Das Reaktionsgemisch wird 0,5 bis 4 Tage, bevorzugt 1 bis 2 Tage bei Raumtemperatur gerührt und zur Trockne eingeengt. Die Aufarbeitung erfolgt auf üblichem Wege. Die Reinigung der Produkte erfolgt je nach Kristallisationsneigung durch Kristallisation oder mittels chromatographischer Methoden.
Zur Darstellung der Ammoniumsalze **1-Y** ausgehend von den Aminen **1** (Stufe viii) kann wie vorstehend für Stufe (vii) beschrieben vorgegangen werden.

Alternativ zur vorstehend beschriebenen Vorgehensweise gelingt die Darstellung der Verbindungen der Formel **1** nach Stufe (vii) auch durch reduktive Aminierung der Amine **6** mit Carbonylverbindungen in Gegenwart eines Reduktionsmittels. Die Umsetzung der Amine **6** mit den Carbonylverbindungen zu den intermediär gebildeten Schiffschen Basen wird in Solventien wie Toluol, Dichlormethan, Essigsäureethylester, Ether, Tetrahydrofuran etc. vorzugsweise bei Raumtemperatur geführt. Sie kann in Gegenwart einer Säure, vorzugsweise in Gegenwart von Essigsäure durchgeführt werden. Die anschließende Reduktion kann mit mit komplexen Hydriden wie beispielsweise LiAlH₄, Li-Alkoxyhydriden, NaBH₄, NaBHCN₃, NaBH(OAc)₃, etc. erfolgen. Für die Reaktion mit primären Aminen verwendet man vorzugsweise NaBH₄, für sekundäre Amine NaBH(OAc)₃. Zur Darstellung der Methylverbindungen durch Umsetzung mit Formalin bietet sich als Lösemittel die Verwendung von Ameisensäure an. Die Aufarbeitung erfolgt auf üblichem Wege. Die Reinigung der Produkte erfolgt je nach Kristallisationsneigung durch Kristallisation oder mittels chromatographischer Methoden.

Alternativ zur vorstehend beschriebenen Vorgehensweise gelingt die Darstellung der Verbindungen der Formel **1** auch gemäß der in Schema 4 dargestellten Vorgehensweise. 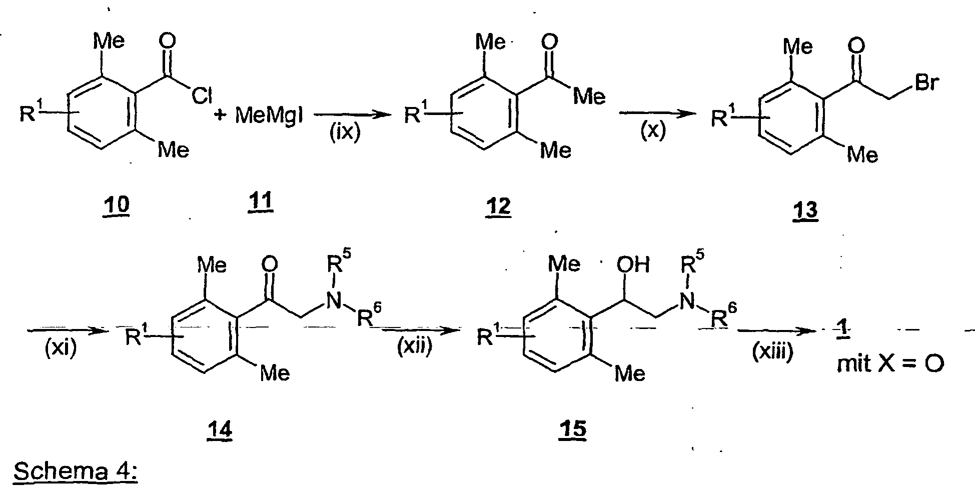 Ausgehend von geeignet substituierten Benzoesäuren lassen sich gemäß literaturbekannter Verfahren (Recl.Trav.Chim.Pays-Bas **61**,1942,539,544) durch Grignard-Reaktion mit den entsprechenden Säurechloriden **10** die erwünschten Acetophenonzwischenstufen **12** herstellen (Stufe ix). Diese werden bevorzugt in Ether zu den Verbindungen **13** bromiert (Stufe x) und zweckmäßigerweise ohne weitere Reinigung über die Aminoketone **14** und deren sofortige Reduktion (Stufe xii), bevorzugt mit Natriumboranat in Isopropanol oder mit Lithiumalanat in Diethylether oder Tetrahydrofuran, in die Aminoethanolzwischenstufen **15** umgewandelt. Der Zugang zu optisch aktiven Aminoethanolzwischenstufen **15** erschließt sich stereospezifisch durch asymmetrische Hydrierung gemäß literaturbekannter Verfahren z.B. mittels Rhodiumkatalysatoren unter Verwendung von (S.S)- bzw. (R.R)-BCPM (Chem.Pharm.Bull.43,738 (1995)).
Die Veretherung zu den Verbindungen **1** (bei denen X für Sauerstoff steht) mit Variation der Kettenlänge von A erfolgt z.B. durch Verwendung von Benzylhalogeniden unter bevorzugtem Einsatz von Kalium-tert.-butylat als Hilfsbase (A = C₁), durch Reppe-Reaktion mittels gegebenenfalls substituierten Phenylacetylenen und anschließender Hydrierung der entstehenden Z/E-Olefine (A = C₂) sowie durch Williamson-Veretherungen mittels Phenylalkylhalogeniden unter bevorzugter Verwendung von Kronenethern (z.B. A = C₃). An dieser Stelle sei ferner auf die allgemeinen Ausführungen zu Stufe (iii) gemäß Schema 1 verwiesen, die an dieser Stelle analog anwendbar sind.

Ausgehend von den Verbindungen der Formel **3** gelingt ein Zugang zu den Verbindungen der allgemeinen Formel **1**, in denen X für -NH- steht, nach der in Schema 5 dargestellten Vorgehensweise. 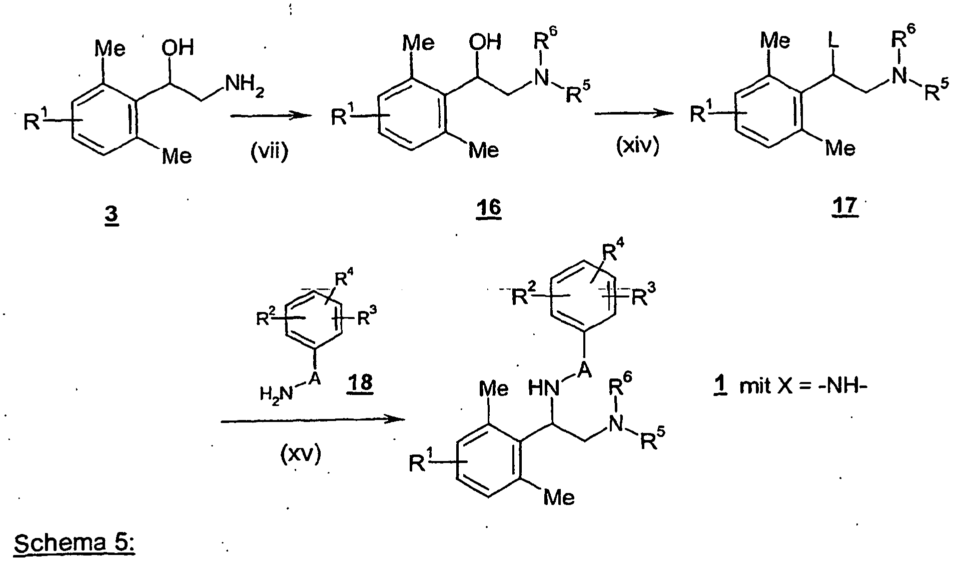 Zur Durchführung des Verfahrens nach Stufe (vii) wird ein Amin der allgemeinen Formel **3** in einem organischen Lösemittel wie Dimethylformamid, Dimethylacetamid, Methylenchlorid, Tetrahydrofuran, bevorzugt Dimethylformamid und besonders bevorzugt wasserfreies, gegebenenfalls absolutes Dimethylformamid oder Methylenchlorid gelöst. Die so erhaltene Lösung wird mit einer anorganischen oder organischen Base und einem entsprechenden Alkylierungsmittel versetzt. Als Base kommen die Alkali- oder die Erdalkalicarbonate des Lithiums, Natriums, Kaliums, Calziums wie Natriumcarbonat, Lithiumcarbonat, Kaliumcarbonat, Calziumcarbonat und bevorzugt Kaliumcarbonat in Betracht. Ferner können die Hydrogencarbonate des Lithiums, Natriums und Kaliums eingesetzt werden. Ferner sind die Alkali- oder Erdalkalihydroxide des Lithiums, Natriums, Kaliums, Magnesiums, Calziums, bevorzugt jedoch Natriumhydroxid, Kaliumhydroxid, Lithiumhydroxid und Calziumhydroxid in Alkoholen oder Wasser einsetzbar. Als weitere Basen kommen Alkoholate der Alkali- und Erdalkalimetalle, bevorzugt die Ethylate des Natriums und Kaliums in Betracht. Weiterhin können Alkali- und Erdalkalihydride, vorzugsweise des Kaliums oder Natriums, bevorzugt in inerten Lösungsmitteln wie Dimethylformamid, Dimethylacetamid, Methylenchlorid, Ethern, Tetrahydrofuran und Toluol eingesetzt werden. Als organische Base kommen bevorzugt organische Amine, besonders bevorzugt Diisopropylethylamin, Triethylamin, cyclische Amine wie DBU, oder Pyridin in Betracht. Als Alkylierungsmittel kommen Alkylhalogenide, wie Alkylchlorid, Alkylbromid, besonders Alkyljodid sowie Alkyltosylate, -Mesylate, -Triflate, -Dialkylsulfate in Betracht. Die Alkylreste der Alkylierungsmittel entsprechen den zuvor getroffenen Definitionen für R⁵ und R⁶. Das Reaktionsgemisch wird 0,5 bis 4 Tage, bevorzugt 1 bis 2 Tage bei Raumtemperatur gerührt und zur Trockne eingeengt. Die Aufarbeitung erfolgt auf üblichem Wege. Die Reinigung der Produkte **16** erfolgt je nach Kristallisationsneigung durch Kristallisation oder mittels chromatographischer Methoden.

Aus den Verbindungen der Formel **16** lassen sich durch Umsetzung gemäß Stufe (xiv) die Verbindungen der Formel **17** erhalten, in denen L für eine Abgangsgruppe, ausgewählt aus Chlor, Brom, lod, Methansulfonat, Trifluormethansulfonat oder para-Toluolsulfonat steht. Im Falle von R⁵ oder R⁶ gleich Wasserstoff sind Schutzgruppen gemäß dem Stand der Technik zu verwenden. Steht L für Chlor oder Brom, gelingt die Umsetzung unter Verwendung gängiger Halogenierungsreagenzien. Steht L für Methansulfonat, Trifluormethansulfonat oder para-Toluolsulfonat gelingt die Umsetzung der Verbindungen **16** mit den entsprechenden Sulfonsäurechloriden oder -anhydriden zu den Verbindungen **17** in inerten Lösungsmitteln wie Dimethylformamid, Dimethylacetamid, Methylenchlorid, Ethern, Tetrahydrofuran und Toluol in Gegenwart organischer Amine, wie bevorzugt Diisopropylethylamin, Triethylamin, cyclischen Aminen wie DBU, oder Pyridin.

Aus den Verbindungen **17** lassen sich durch Reaktion mit den Aminen **18** unter den Reaktionsbedingungen die bereits für Stufe (vii) beschrieben wurden, die Verbindungen der Formel **1** erhalten, in denen X für -NH- steht. Aus diesen lassen sich ferner die Verbindungen der Formel **1** darstellen, in denen X für -N(C₁-C₆-Alkyl)- oder -N(C₃-C₆-Cycloalkyl-C₁-C₄-alkylen)- steht. Diese Umsetzung gelingt unter den Reaktionsbedingungen, die für Stufe (vii) beschrieben worden sind durch Alkylierung der Verbindungen der Formel **1** mit X=-NH- mit Alkylierungsreagentien C₁-C₆-Alkyl-L beziehungsweise C₃-C₆-cycloalkyl-C₁-C₄-alkyl-L, wobei L die vorstehend genannten Bedeutungen haben kann.

Ausgehend von den Verbindungen der Formel **1** mit X = NH gelingt ein Zugang zu den Verbindungen der allgemeinen Formel **1**, in denen X für -N(CHO)- oder -N(CO-C₁-C₆-Alkyl)- steht, nach der in Schema 6 dargestellten Vorgehensweise.

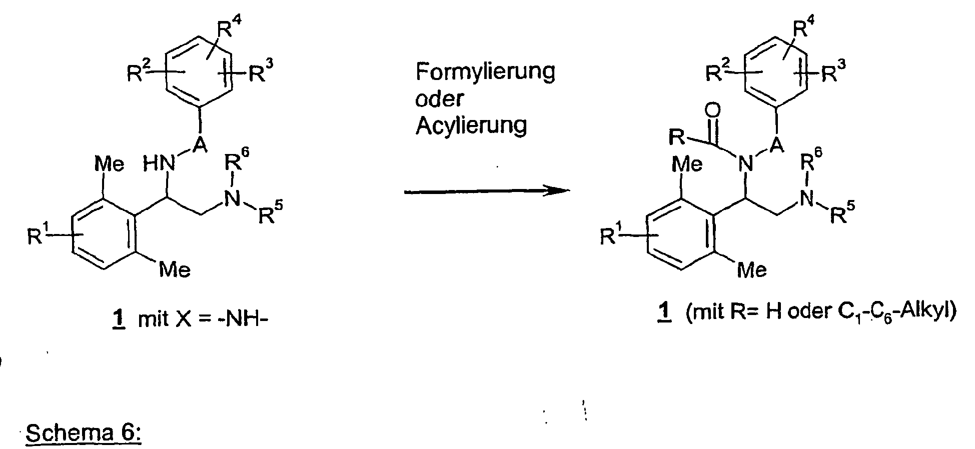

Die Umsetzungen gemäß Schema 6 können dabei in Analogie zu an sich bekannten Formylierungs- und Acylierungsverfahren durchgeführt werden.

Die nachfolgenden Beispiele dienen lediglich der exemplarischen Erläuterung ohne den Gegenstand der Erfindung auf selbige zu beschränken.

### Beispiel 1: 2-[3-(2,6-Difluorphenyl-propoxy]-2-(2,6-dimethylphenyl)-ethylamin

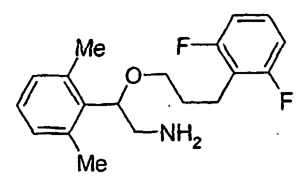

### Synthese der Vorstufen 2, 5 und 8:

### 1.1: 2,6-Dimethylbenzaldehyd (entspricht Verbindung der Formel 2)

Zu einer auf - 65°C gekühlten Lösung von 100 g (0,54 mol) 2-Brom-1,3-dimethylbenzol in 690 ml THF werden innerhalb 1 h 336 ml (0,54 mol) einer 1,6 M Lösung von n-Butyllithium in Hexan getropft. Anschließend wird 1 h bei gleicher Temperatur gerührt. Danach tropft man 100 ml DMF bei - 65°C zu und lässt 30 min bei dieser Temperatur reagieren. Der Ansatz wird auf 500 ml Eis/135 ml konz. Salzsäure gegeben. Die organische Phase wird abgetrennt und die wässrige Phase mit Essigester extrahiert. Die vereinigten organischen Phasen werden getrocknet und eingeengt. Ausbeute: 85,0 g hellgelbes Öl.

### 1.2: 3-(2,6-Difluorphenyl)-propan-1-ol (entspricht Verbindung der Formel 8)

### 1.2.1: 2-(2,6-Difluorbenzyl)-malonsäurediethylester:

Man legt 19,3 g (121 mmol) Malonsäurediethylester in 700 ml THF vor und gibt 14,9 g (132 mmol) Kaliumtertiärbutylat zu. Man rührt 1 h und versetzt mit 25 g (121 mmol) 2,6-Difluorbenzylbromid (**3**). Nach 3 h Rühren bei Raumtemperatur wird über Kieselgur abgesaugt und eingeengt. Ausbeute: 33,1 g, hellgelbes Öl.

### 1.2.2: 2-(2,6-Difluorbenzyl)-malonsäuremonoethylester.

33,1 g (116 mmol) 2-(2,6-Difluorbenzyl)-malonsäurediethylester (Beispiel 1.2.1) werden in 120 ml Ethanol gelöst und unter Eiskühlung tropfenweise mit 15 ml einer 40proz. Natriumhydroxidlösung versetzt. Man rührt 4 h bei Raumtemperatur, destilliert dann das organische Lösungsmittel ab und wäscht den Rückstand mit Wasser aus. Die wässrige Lösung wird mit Dichlormethan gewaschen und mit Salzsäure sauer gestellt. Man extrahiert mit Dichlormethan, engt ein und erhält ein langsam kristallisierendes, braunes Öl. Ausbeute 21,5 g, Schmp.: 60°C.

### 1.2.3: 3-(2,6-Difluorphenyl)-propionsäureethylester:

21,5 g (83,3 mmol) 2-(2,6-Difluorbenzyl)-malonsäuremonoethylester (Beispiel 1.2.2) werden ohne Lösungsmittel für 4h unter Rühren erhitzt (Badtemperatur 160°C). Man erhält eine hellbraune Flüssigkeit. Ausbeute: 17,6 g.

### 1.2.4: 3-(2,6-Difluorphenyl)-propan-1-ol (entspricht Verbindung der Formel.8):

8,80 g (41,0 mmol) 3-(2,6-Difluorphenyl)-propionsäureethylester (Beispiel 1.2.3) gelöst in 60 ml THF werden unter Eiskühlung zu einer Suspension von 1,71 g (41,1 mmol) Lithiumaluminiumhydrid in 40 ml THF getropft. Man rührt zunächst 1 h bei Raumtemperatur, dann 2 h bei 75°C. Man versetzt mit Diammoniumtartratlösung und Magnesiumsulfat, trennt die Lösung ab und wäscht den Rückstand mit Essigester aus. Nach Trocken und Einengen erhält man eine klare Flüssigkeit. Ausbeute: 5,67 g.

### 1.2.5: 2-(3-Brom-propyl)-1,3-difluorbenzol (Verbindung der Formel 5):

6,86 g (39,8 mmol) 3-(2,6-Difluorphenyl)-propan-1-ol (Beispiel 1.2.4) werden in 50 ml Toluol gelöst und mit 19,1 g (92,0 mmol) Thionylbromid versetzt. Man rührt 3 h unter Rückfluss, entfernt das Lösungsmittel und destilliert überschüssiges Thionylbromid ab. Anschließend wird flash-chromatographisch (Cyclohexan) gereinigt.
Ausbeute: 6,98 g.

### Synthesevariante nach Schema 1:

### 1.3: 2-Amino-1-(2,6-dimethylphenyl)-ethanol (entspricht Verbindung der Formel 3):

### Verfahren nach Stufe (i):

Zu 14,4 g (107 mmol) 2,6-Dimethylbenzaldehyd (Beispiel 1.1) gibt man 15,7 ml (117 mmol) Trimethylsilylcyanid und 10,0 g (110 mmol) Zinkdiiodid. Man rührt 30 min bei Raumtemperatur und gibt 150 ml Ether zu. Dann werden 8,10 g (213 mmol) Lithiumaluminiumhydrid so zugegeben, dass der Ansatz gelinde siedet. Nach beendeter Zugabe wird 2 h unter Rückfluss gerührt. Danach wird unter Eiskühlung vorsichtig mit Diammoniumtartratlösung hydrolysiert. Das Gemisch wird über Magnesiumsulfat abgesaugt und mit Ether nachgewaschen. Man engt ein und erhält das Produkt als gelbe Kristalle. Ausbeute: 7,0 g, MS: m/z 166 [(M+H)⁺].

### 1.4: N-[2-(2,6-Dimethylphenyl)-2-hydroxyethyl]-trifluoracetamid (entspricht Verbindung der Formel 4):

### Verfahren nach Stufe (ii):

2,00 g (12,1 mmol) 2-Amino-1-(2,6-dimethylphenyl)-ethanol (Beispiel 1.3) werden in 100 ml Dichlormethan gelöst, mit 3,68 g (17,5 mmol) Trifluoressigsäureanhydrid und 1,60 g (15,8 mmol) Triethylamin versetzt und 4 h im Eisbad gerührt. Es wird eingeengt, der Rückstand in 100 ml Dichlormethan aufgenommen und mit 30 ml gesättigter Natriumhydrogencarbonatlösung gewaschen. Die organische Phase wird getrocknet und eingeengt und das Rohprodukt flash-chromatographisch (DichlormethanlEthanol 90:10) gereinigt. Ausbeute: 3,11 g, Schmp.: 96°C.

### 1.5: 2-[3-(2,6-Difluorphenyl)-propoxy]-2-(2,6-dimethylphenyl)-ethylamin (entspricht Verbindung der Formel 6):

### Verfahren nach Stufe (iii):

261 mg (1,00 mmol) *N*-[2-(2,6-Dimethylphenyl)-2-hydroxyethyl]-trifluoracetamid (Beispiel 1.4) werden in 3 ml THF gelöst und unter Eiskühlung mit 70,0 mg (1,46 mmol) Natriumhydrid (50proz. Suspension in Mineralöl) versetzt. Nach 5 min gibt man 230 µL (1,13 mmol) 15-Krone-5 hinzu und rührt weitere 5 min. Man versetzt mit 236 mg (1,00 mmol) 2-(3-Bromo-propyl)-1,3-difluorbenzol (Beispiel 1.2) und rührt 2 h bei Raumtemperatur. Danach kocht man 7 h unter Rückfluss. Der Ansatz wird flashchromatographisch gereinigt (DichlormethanlEthanöl 95:5). Ausbeute: 120 mg, Schmp.: 166°C.

### Synthesevariante nach Schema 2:

### 1.6: 1,3-Dimethyl-2-(2-nitrovinyl)benzol (entspricht Verbindung der Formel 7):

### Verfahren nach Stufe (iv):

Zu 40,0 g (300 mmol) 2,6-Dimethylbenzaldehyd (Beispiel 1.1) in 160 ml Eisessig gibt man 27,2 g Ammoniumacetat und 48 ml (900 mmol) Nitromethan. Man erhitzt 4 h auf 120°C und gibt auf 500 ml Eis. Nach dem Auftauen wird mit Essigester extrahiert, die organische Phase mit gesättigter Natriumchloridlösung gewaschen, getrocknet und eingeengt. Das Rohprodukt wird flash-chromatographisch (Cyclohexan/Essigester 80:20) gereinigt. Ausbeute: 15,0 g, gelbes, langsam kristallisierendes Öl.

### 1.7: 2-(1-(2,6-Difluorphenylpropyloxy)-2-nitro-ethyl)-1,3-dimethyl-benzol (entspricht Verbindung der Formel 9):

### Verfahren nach Stufe (v):

Zu 4,30 g (24,3 mmol) 3-(2,6-Difluorphenyl)-propan-1-ol (Beispiel 1.2.4) gelöst in 20 ml THF werden 1,20 g (25,0 mmol) Natriumhydrid (50proz. Suspension in Mineralöl) gegeben. Man rührt 14 h bei Raumtemperatur und tropft dann 4,30 g (24,1 mmol) 1,3-Dimethyl-2-(2-nitrovinyl)-benzol (Beispiel 1.6) gelöst in 40 ml THF zu. Anschließend wird 6 h bei Raumtemperatur gerührt, mit Eisessig sauer gestellt, mit Wasser verdünnt und mit Essigester extrahiert. Die organische Phase wird mit gesättigter Natriumchloridlösung gewaschen, getrocknet und eingeengt. Das Rohprodukt wird flash-chromatographisch (Cyclohexan/Essigester 90:10) gereinigt. Ausbeute: 3,20 g.

### 1.8: 2-[3-(2,6-Difluorphenyl)-propoxy]-2-(2,6-dimethylphenyl)-ethylamin (entspricht Verbindung der Formel 6):

### Verfahren nach Stufe (vi):

1,50 g (4,29 mmol) 2-(1-(2,6-Difluorphenylpropyloxy)-2-nitro-ethyl)-1,3-dimethylbenzol (Beispiel 1.7) gelöst in 30 ml Methanol werden über 1,0 g Raney-Nickel 6 h bei Atmosphärendruck hydriert. Man trennt vom Katalysator ab, engt ein und reinigt flash-chromatographisch (Dichlormethan/Ethanol 95:5). Ausbeute: 745 mg, gelbes Öl.

### Beispiel 2: N-[2-[3-(2,6-Difluorphenyl)-propoxy]-2-(2,6-dimethylphenyl)-ethyl]-N,N-bis-(2-ethylbutyl)-amin

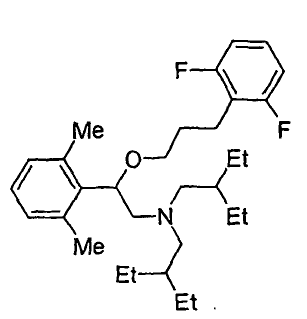

### Verfahren nach Stufe (vii):

126 mg (0,39 mmol) 2-[3-(2,6-Difluorphenyl)-propoxy]-2-(2,6-dimethylphenyl)-ethylamin (Beispiel 1) werden in 4 ml Dichlormethan vorgelegt und mit 24 mg (0,39 mmo) Eisessig und 39,5 mg (0,39 mmol) 2-Ethylbutyraldehyd versetzt. Nach 7 min werden 117 mg (0,55 mmol) Natriumtriacetoxyborhydrid zugesetzt. Der Ansatz wird 2 h bei Raumtemperatur gerührt, dann mit 10proz. Natriumhydrogencarbonatlösung versetzt und weitere 30 min bei Raumtemperatur gerührt. Die Phasen werden getrennt, die organische Phase mit gesättigter Natriumchloridlösung gewaschen, getrocknet, eingeengt und das Rohprodukt flash-chromatographisch (Dichlormethan/Ethanol 95:5) getrennt, wobei [2-[3-(2,6-Difluorphenyl)-propoxy]-2-(2,6-dimethylphenyl)-ethyl]-bis-(2-ethylbutyl)-amin als erstes eluiert.
Ausbeute: 72 mg, farbloses Öl, MS: m/z 488 [(M+H)⁺];

### Beispiel 3: N-[2-[3-(2,6-Difluorphenyl)-propoxy]-2-(2,6-dimethylphenyl)-ethyl]-N-(2-ethylbutyl)-amin

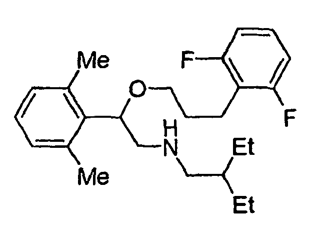

Herstellung analog Beispiel 2, wobei die Titelverbindung bei der chromatographischen Trennung als zweites eluiert;
Ausbeute: 61 mg, farbloses Öl, MS: m/z 404 [(M+H)⁺].

### Beispiel 4: N-[2-[3-(2,6-Difluorphenyl)-propoxy]-2-(2,6-dimethylphenyl)-ethyl]-N-(2-ethylbutyl)-N-methyl-amin

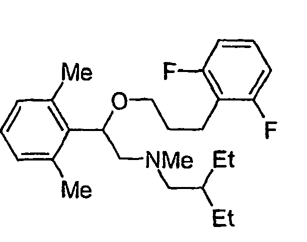

### Verfahren nach Stufe (vii):

45 mg (0,11 mmol) [2-[3-(2,6-Difluorphenyl)-propoxy]-2-(2,6-dimethylphenyl)-ethyl]-(1-ethylpropyl)-amin (Beispiel 3) werden in 2 ml Ameisensäure gelöst und mit 1 ml 37proz. Formalinlösung versetzt. Man rührt 4 h bei einer Badtemperatur von 120°C, kühlt ab, verdünnt mit Wasser und extrahiert mit Dichlormethan. Der nach Trocknen und Einengen erhaltene Rückstand wird flash-chromatographisch gereinigt (Dichlormethan/Ethanol 95:5). Ausbeute: 15 mg, MS: m/z 418 [(M+H)⁺].

### Beispiel 5: N-[2-[3-(2,6-Difluorphenyl)-propoxy]-2-(2,6-dimethylphenyl)-ethyl]-N-(2-ethylbutyl)-N,N-dimethyl-ammoniumiodid

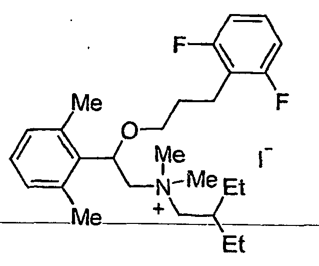

### Verfahren nach Stufe (viii):

30,0 mg (74 µmol) [2-[3-(2,6-Difluorphenyl)-propoxy]-2-(2,6-dimethylphenyl)-ethyl]-(2-ethylbutyl)-amin (Beispiel 3) werden in 3 ml Acetonitril gelöst und mit 20,5 mg (148 µmol) Kaliumcarbonat, 6,0 mg (37 µM) Kaliumiodid und 22,0 mg (155 µmol) Methyliodid versetzt. Man rührt 3 h bei Raumtemperatur, entfernt das Lösungsmittel und reinigt den Rückstand flash-chromatographisch (Dichlormethan/Ethanol 95:5). Ausbeute: 33 mg, MS: m/z 432 [(M+H)⁺].

### Beispiel 6: 1-[2-[3-(2,6-Difluorphenyl)-propoxy]-2-(2,6-dimethylphenyl)-ethyl]-pyrrolidin

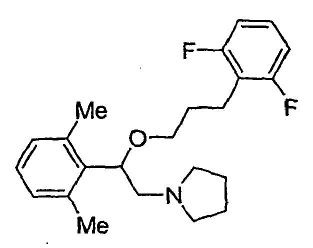

### Verfahren nach Stufe (vii):

163 mg (0,51 mmol) 2-[3-(2,6-Difluorphenyl)-propoxy]-2-(2,6-dimethylphenyl)-ethylamin (Beispiel 1), 110 mg (0,51 mmol) 1,4-Dibrombutan, 200 mg Kaliumcarbonat und 50 mg Kaliumiodid gelöst in 20 ml Acetonitril werden 4 h unter Rückfluss erhitzt. Anschließend wird eingeengt und der Rückstand flashchromatographisch (Dichlormethan/Ethanol 95:5) gereinigt, um das Produkt als weißen, kristallinen Feststoff zu erhalten. Ausbeute: 58 mg, Schmp.: 173-175°C.

### Beispiel 7: 1-[2-[3-(2,6-Difluorphenyl)-propoxy]-2-(2,6-dimethylphenyl)-ethyl]-1-methyl-pyrrolidiniumiodid

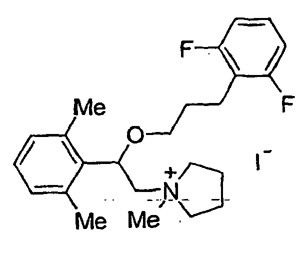

### Verfahren nach Stufe. (viii):

25,0 mg (70 µmol) 1-[2-[3-(2,6-Difluorphenyl)-propoxy]-2-(2,6-dimethylphenyl)-ethyl]-pyrrolidin (Beispiel 6), 10,0 mg (70 µmol) Methyliodid, 20,0 mg Kaliumcarbonat und 6,0 mg Kaliumiodid werden in 2 ml Acetonitril 14 h bei Raumtemperatur gerührt. Man engt ein und reinigt den Rückstand flash-chromatographisch (Dichlormethan/Ethanol 95:5). Ausbeute: 20,0 mg, MS: m/z 389 [(M+H)⁺].

### Beispiel 8: N-[2-[3-(2,6-Difluorphenyl)-propoxy]-2-(2,6-dimethylphenyl)-ethyl]-N,N-dimethyl-amin

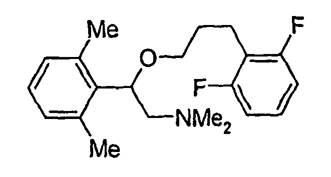

### Verfahren nach Stufe (vii):

200 mg (0,63 mmol) 2-[3-(2,6-Difluorphenyl)-propoxy]-2-(2,6-dimethylphenyl)-ethylamin (Beispiel 1) werden in 12 ml Ameisensäure gelöst und mit 6 ml einer 37proz. Formalinlösung versetzt. Man rührt 5 h bei 120°C, gibt den Ansatz auf Eis und stellt mit konz. Natriumhydroxidlösung auf pH 13-14. Anschließend wird mit Ether extrahiert, die organische Phase getrocknet und eingeengt, wodurch man das Produkt als gelbliches Öl erhält. Ausbeute: 170 mg, MS: m/z 348 [(M+H)⁺].

### Beispiel 9: N-]2-[3-(2,6-Difluorphenyl)-Propoxy]-2-(2,6-dimethylphenyl)-ethyl]-N-cyclopropylmethyl-amin

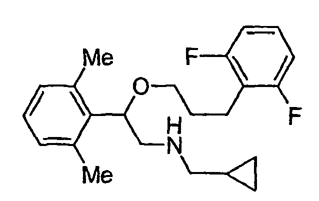

Herstellung analog Beispiel 3; Öl; MS: m/z 374 [(M+H)⁺].

### Beispiel 10: N-[2-[3-(2,6-Difluorphenyl)-propoxy]-2-(2,6-dimethylphenyl)-ethy]-N-cyclopropylmethyl-N-methyl-amin

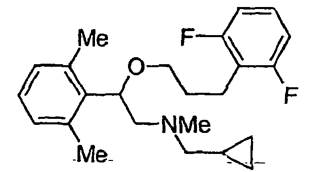

Herstellung analog Beispiel 4, ausgehend von Beispiel 9; Schmp.: 78°C; MS: m/z 388 [(M+H)⁺].

### Beispiel 11: N-[2-[3-(2,6-Difluorphenyl)-propoxyl]-2-(2,6-dimethylphenyl)-ethyl]-N,N-bis-(1-cyclohexen-4-yl-methyl)-amin

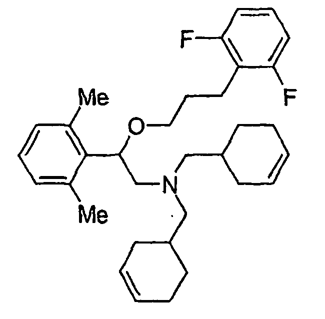

Herstellung analog Beispiel 2; Öl; MS: m/z 508 [(M+H)⁺].

### Beispiel 12: N-[2-[3-(2,6-Difluorphenyl)-propoxy]-2-(2,6-dimethylphenyl)-ethyl]-N-(1-cyclohexen-4-yl-methyl)-amin

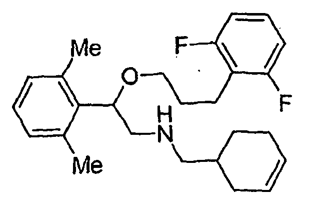

Herstellung analog Beispiel 3; Öl; MS: m/z 414 [(M+H)⁺].

### Beispiel 13: N-[2-[3-(2,6-Difluorphenyl)-propoxy]-2-(2,6-dimethylphenyl)-ethyl]-N-n-butyl-amin

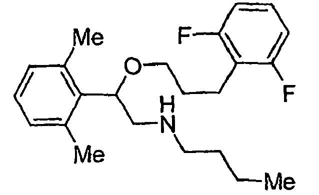

Herstellung analog Beispiel 3; Öl; MS: m/z 376 [(M+H)⁺].

### Beispiel 14: 1-[2-[3-(2,6-Difluorphenyl)-propoxy]-2-(2,6-dimethylphenl)-ethyl]-piperidin

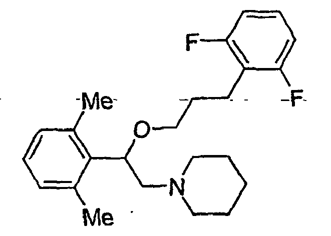

Herstellung analog Beispiel 6; Schmp.(Hydrochlorid): >200°C; MS: m/z 388 [(M+H)⁺]. Zur Darstellung des Hydrochlorids der Titelverbindung wird die freie Base in wenig Ether aufgenommen und tropfenweise solange mit etherischer Salzsäure versetzt, bis die Fällung vollständig ist. Anschließend wird vom Lösemittel abgetrennt, vorsichtig mit Ether nachgewaschen und getrocknet.

### Beispiel 15: N-[2-[3-(2,6-Difluoruhenyl)-propoxy]-2-(2,6-dimethylphenyl)-ethyl]-N-(1-cyclohexen-4-yl-methyl)·N,N-dimethyl-ammoniumiodid

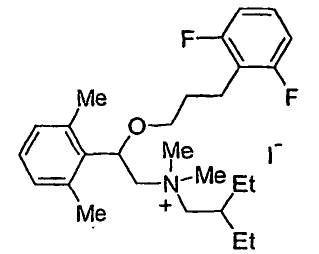

Herstellung analog Beispiel 5 ausgehend von Beispiel 12; Schmp.: 105°C; MS: m/z 432 [(M+H)⁺].

### Beispiel 16: N-[2-[3-(2,6-Difluorphenyl)-propoxy]-2-(2,6-dimethylphenyl)-ethyl]-N-n-butyl-N,N-dimethyl-ammoniumiodid

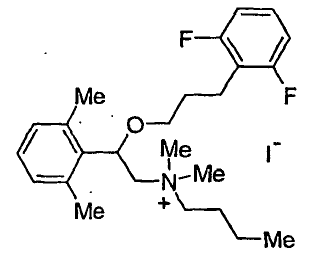

Herstellung analog Beispiel 5 ausgehend von Beispiel 13; MS: m/z 404 [(M+H)⁺].

### Beispiel 17: N-[2-[3-(2,6-Difluorphenyl)-propoxy]-2-(2,6-dimethylphenyl)-ethyl]-N-iso-propyl-amin

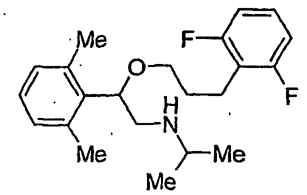

Herstellung analog Beispiel 3; Öl; MS: m/z 362 [(M+H)⁺].

### Beispiel 18: 1-[2-[3-(2,6-Difluorphenyl)-propoxy]-2-(2,6-dimethylphenyl)-ethyl]-azepan

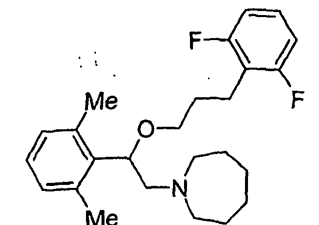

Herstellung analog Beispiel 6; Öl; MS: m/z 402 [(M+H)⁺].

### Beispiel 19: N-[2-F3-(2,6-Difluorphenyl)-Propoxy]-2-(2,6-dimethylphenyl)-ethyl]-N-neo-Pentyl-amin

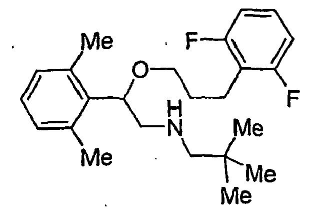

Herstellung analog Beispiel 3; Öl; MS: m/z 390 [(M+H)⁺].

### Beispiel 20: N-[2-[3-(2,6-Difluorphenyl)-propoxy]-2-(2,6-dimethytphenyl)-ethyl]-N,N-diethyl-amin

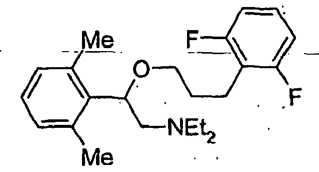

Herstellung analog Beispiel 2; Öl; MS; m/z 376 [(M+H)⁺]

### Beispiel 21: N-[2-[3-(2,6-Difluorphenyl)-propoxyl]-2-(2,6-dimethylphenyn)-ethyl]-N-cyclohexylmethyl-amin

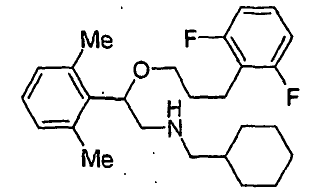

Herstellung analog Beispiel 3; Schmp.(Hydrochlorid): 187°C; MS: m/z 416 [(M+H)⁺]. Zur Darstellung des Hydrochlorids der Titelverbindung wird die freie Base in wenig Ether aufgenommen und tropfenweise solange mit etherischer Salzsäure versetzt, bis die Fällung vollständig ist. Anschließend wird vom Lösemittel abgetrennt, vorsichtig mit Ether nachgewaschen und getrocknet.

### Beispiel 22: N-[2-[3-(2,6-Difluorphenyl)-propoxy]-2-(2,6-dimethylphenyl)-ethyt]-N-iso-butyl-amin

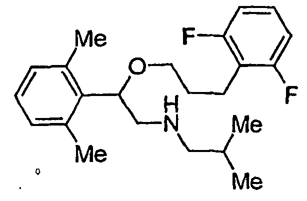

Herstellung analog Beispiel 3; Öl; MS: m/z 376 [(M+H)⁺].

### Beispiel 23: N-[2-[3-(2,6-Difluorphenyl)-propoxy]-2-(2,6-dimethylphenyl)-ethyl]-N-benzyl-amin

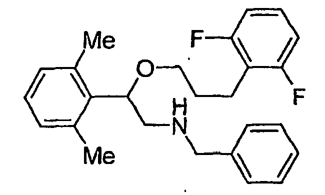

Herstellung analog Beispiel 3; Schmp.(Hydrochlorid): 176°C; MS: m/z 410 [(M+H)⁺]. Zur Darstellung des Hydrochlorids der Titelverbindung wird die freie Base in wenig Ether aufgenommen und tropfenweise solange mit etherischer Salzsäure versetzt, bis die Fällung vollständig ist. Anschließend wird vom Lösemittel abgetrennt, vorsichtig mit Ether nachgewaschen und getrocknet.

### Beispiel 24: N-[2-[3-(2,6-Difluorphenyl)-propoxy]-2-(2,6-dimethylphenyl)-ethyl]-N,N-bis(benzyl)-amin

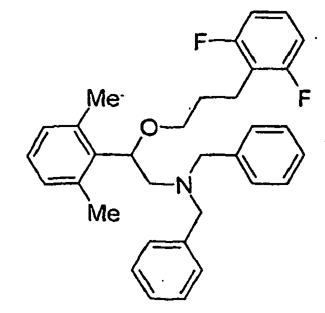

Herstellung analog Beispiel 2; Schmp. (Hydrochlorid): 108°C; MS: m/z 500 [(M+H)⁺]. Zur Darstellung des Hydrochlorids der Titelverbindung wird die freie Base in wenig Ether aufgenommen und tropfenweise solange mit etherischer Salzsäure versetzt, bis die Fällung vollständig ist. Anschließend wird vom Lösemittel abgetrennt, vorsichtig mit Ether nachgewaschen und getrocknet.

### Beispiel 25: N-[2-[3-(2,6.Difluorphenyl)-propoxy]-2-(2,6-dimethylahenyl)-ethyl]-N-(4-isopropenyl-cyclohexen-1-yl-methyl)-amin

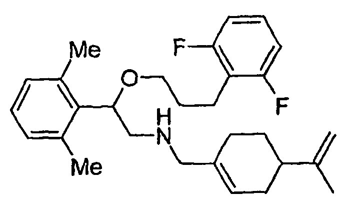

Herstellung analog Beispiel 3; Schmp. (Hydrochlorid): 155°C; MS: m/z 454 [(M+H)⁺]. Zur Darstellung des Hydrochlorids der Titelverbindung wird die freie Base in wenig Ether aufgenommen und tropfenweise solange mit etherischer Salzsäure versetzt, bis die Fällung vollständig ist. Anschließend wird vom Lösemittel abgetrennt, vorsichtig mit Ether nachgewaschen und getrocknet.

### Beispiel 26: N-[2-[3-(2,6-Difluorphenyl)-propoxy]-2-(2,6-dimethylphenyl)-ethyl]-N-(2-methylbutyl)-amin

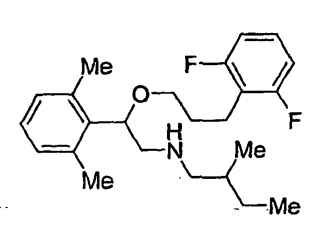

Herstellung analog Beispiel 3; Öl; MS: m/z 390 [(M+H)⁺].

### Beispiel 27: N-[2-[3-(2,6-Difluolphenyl)-Propoxy]-2-(2,6-dimethylphenyl)-ethyl]-N,N-bis-(2-methylbututyl-amin

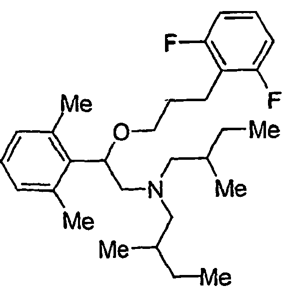

Herstellung analog Beispiel 2; Öl; MS: m/z 460 [(M+H)⁺].

### Beispiel 28: N-[2-[3-(2,6-Difluorphenyl)-propoxy]-2-(2,6-dimethylphenyl)-ethyl]-N-neo-hexyl-amin

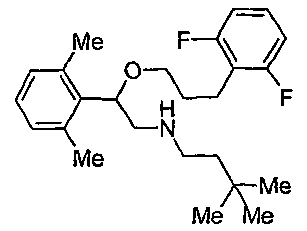

Herstellung analog Beispiel 3; Öl; MS: m/z 404 [(M+H)⁺].

### Beispiel 29: N-[2-[3-(2.6-Difluorphenyl)-propoxy]-2-(2,6-dimethylphenyl)-ethyl]-N,N-bis-(neo-hexyl)-amin

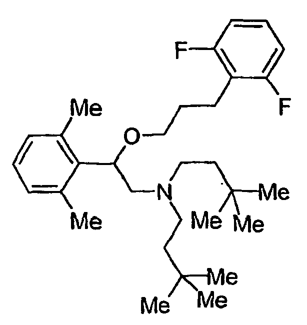

Herstellung analog Beispiel 2; Öl; MS: m/z 488 [(M+H)⁺].

### Beispiel 30: N-[2-[3-(2,6-Difluorphenyl)-propoxy]-2-(2,6-dimethylphenyl)-ethyl]-N-(2-trifluormethyl-ethyl)-amin

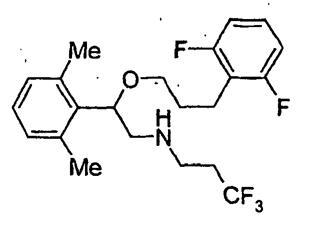

Herstellung analog Beispiel 3; ÖI; MS: m/z 416 [(M+H)⁺].

### Beispiel 31: N-[2-[3-(2,6-Difluorphenyl)-propoxy]-2-(2,6-dimethylphenyl)-ethyl]-N-cyclohexyl-amin

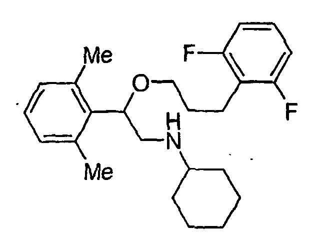

Herstellung analog Beispiel 3; Öl; MS: m/z 402 [(M+H)⁺].

### Beispiel 32: N-[2-[3-(2,6-Diftuorphenyl)-propoxyl]-2-(2,6-dimethylphenyl)-ethyl]-N-iso-pentyl-amin

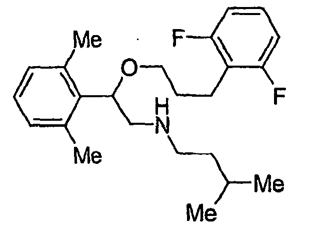

Herstellung analog Beispiel 3; Öl; MS: m/z 390 [(M+H)⁺].

### Beispiel 33: N-[2-[3-(2,6-Difluorphenyl)-propoxy]-2-(2,6-dimethylphenyl)-ethyl]-N,N,N-trimethyl-ammoniumiodid

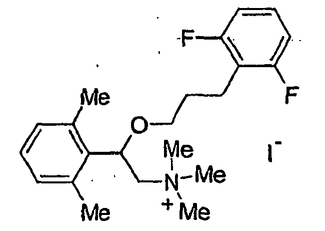

Herstellung analog Beispiel 5 ausgehend von Beispiel 1; Schmp.: 190°C.

### Beispiel 34: [3-(2,6-Difluorphenyl)-propyl]-[1-(2,6-dimethyl-phenyl)-2-pyrrolidin-1-yl-ethyl]-amin

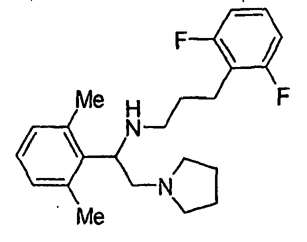

### 34.1: 3-(2 6-Difluorphenyl)-propylamin (entspricht Verbindung der Formel 18):

3,20 g (13,6 mmol) 2-(3-Brompropyl)-1,3-difluorbenzol (entspricht Beispiel 1.2.5) werden im Autoklaven 20 h bei 80°C mit einer Lösung von 5 g Ammoniak in 30 ml THF umgesetzt. Anschließend wird eingeengt und der Rückstand flashchromatographisch gereinigt. Ausbeute: 1,01 g, MS: m/z 172 [(M+H)⁺].

### 34.2: 1-(2 6-Dimethylphenyl)-2-pyrrolidin-1-yl-ethanol (entspricht Verbindung der Formel 16):

### Verfahren nach Stufe (vii):

Zu einer Lösung von 500 mg (3,00 mmol) 2-Amino-1-(2,6-dimethylphenyl)-ethanol (Beispiel 1.3) in 30 ml Acetonitril gibt man 647 mg (3,00 mmol) 1,4-Dibrombutan, 1,0 g Kaliumcarbonat und 250 mg Kaliumiodid. Man kocht 3 h unter Rückfluss. Anschließend wird das Lösungsmittel entfernt und der Rückstand in Dichlormethan aufgenommen. Man filtriert, engt ein und reinigt das Rohprodukt durch Flash-Chromatogaphie (Dichlormethan/Ethanol 90:10). Ausbeute 360 mg, Schmp.:126°C, MS: m/z 220 [(M+H)⁺].

### 34.3: Methansulfonsäure-1-(2,6-dimethyl-phenyl)-2-pyrrolidin-1-yl-ethylester (entspricht Verbindung der Formel 17):

### Verfahren nach Stufe (xiv):

219 mg (1,00 mmol) 1-(2,6-Dimethylphenyl)-2-pyrrolidin-1-yl-ethanol (Beispiel 34.2) werden in 3 ml Dichlormethan gelöst und mit 250 µl (1,80 mmol) Triethylamin und 105 µl (1,30 mmol) Methansulfonsäurechlorid versetzt. Man rührt 14 h bei Raumtemperatur, gibt 2 ml gesättigte Natriumhydrogencarbonatlösung hinzu, extrahiert mit Dichlormethan, trocknet und engt ein. Ausbeute: 192 mg braunes Öl.

### 34.4: [3-(2,6-Difluorphenyl)-propyl]-[1-(2,6-dimethyl-phenyl)-2-pyrrolidin-1-yl-ethyl]-amin:

### Verfahren nach Stufe (xv):

Zu einer Lösung von 190 mg (0,63 mmol) Methansulfonsäure-1-(2,6-dimethylphenyl)-2-pyrrolidin-1-yl-ethylester (Beispiel 34.3) in 2 ml Acetonitril gibt man 140 mg (0,81 mmol) 3-(2,6-Difluorphenyl)-propylamin (Beispiel 34.1) und 2 ml Diisopropylethylamin. Man rührt 6 h bei 80°C, engt ein und reinigt den Rückstand flash-chromatographisch. Ausbeute: 13 mg gelbes Öl, MS: m/z 373 [(M+H)⁺].

### Beispiel 35: [3-(2,6-Difluorphenyl)-propyl]-[1-(2,6-dimethyl-phenyl)-2-piperidin-1-yl-ethyl]-amin

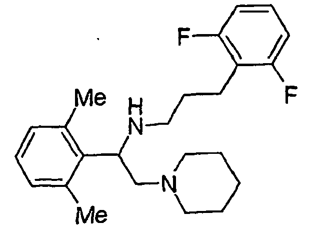

Herstellung analog Beispiel 34; Schmp.(Hydrochlorid): 145°C, MS: m/z 387 [(M+H)⁺]. Zur Darstellung des Hydrochlorids der Titelverbindung wird die freie Base in wenig Ether aufgenommen und tropfenweise solange mit etherischer Salzsäure versetzt, bis die Fällung vollständig ist. Anschließend wird vom Lösemittel abgetrennt, vorsichtig mit Ether nachgewaschen und getrocknet.

### Beispiel 36: 1-[2-[2-(2-Fluorphenyl)-ethoxy]-2-(2,6-dimethylphenyl)-ethyl]-piperidin

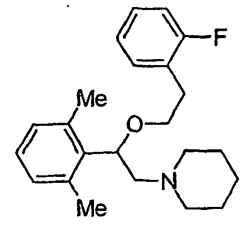

### 36.1: 2.6-Dimethyl-α-brom-acetophenon (entspricht Verbindung der Formel 13):

### Verfahren nach Stufe (x):

23.3 g (0.15 mol) 2.6-Dimethylacetophenon, hergestellt gemäß Rec.Trav.Chim.Pays-Bas 61 539, 544 (1942), werden in 250 ml absolutem Ether gelöst, innerhalb ca. 30 Minuten mit 7.5 ml (0.15 mol) Brom zur Reaktion gebracht und weitere 2 Stunden bei Raumtemperatur nachgerührt. Die etherische Lösung wird 2 x mit ca. 100 ml Wasser gewaschen, bis zum Erreichen von ph 6 mit NaHCO₃ versetzt und getrocknet. Die etherische Lösung wird direkt weiter umgesetzt.

### 36.2: α-N-Piperidino-2 6-dimethyl-acetophenon (entspricht Verbindung der Formel 14):

### Verfahren nach Stufe (xi):

Zu einer Lösung von 30 ml (0.3 mol) Piperidin in 50 ml absolutem Ether wird die etherische Lösung nach Beispiel 36.1 zugetropft und noch weitere 3 Stunden bei Raumtemperatur nachgerührt. Der entstandene Niederschlag wird abgesaugt und von der Etherphase das Solvens entfernt. Der Rückstand wird sofort reduziert.

### 36.3: 1-(2.6-Dimethylphenyl)-2-piperidinoethanol (entspricht Verbindung der Formel 15):

### Verfahren nach Stufe (xii):

Das Rohprodukt gemäß Beispiel 36.2 wird in 300 ml Isopropanol aufgenommen, mit 2.3 g (0.06 mol) NaBH₄ versetzt, 3 Stunden zum Rückfluß erhitzt, zur Vervollständigung der Reaktion nochmals mit der gleichen Menge NaBH₄ versetzt und weitere 3 Stunden zum Rückfluß erhitzt. Anschließend wird das Solvens abgezogen, der Rückstand von ca. 30 g zwischen Wasser und Methylenchlorid verteilt und die organische Phase nach dem Trocknen eingedampft. Nach Flash-Chromatographie an Kieselgel mit Laufmittel Methylenchlorid/Methanol (95 : 5) bis (90 : 10) erhält man 15.1 g der Verbindung c) als helles Öl.
Alternativ gelingt die Reduktion mit LiAlH₄ in THF bevorzugt bei - 60°C; auf die chromatographische Reinigung kann hierbei verzichtet werden.

### 36.4: 1-[2-[2-(2-Fluorphenyl)-ethoxy]-2-(2,6-dimethylphenyl)-ethyl]-piperidin:

### Verfahren nach Stufe (xiii):

2.3 g (0.01 mol) des Ethanolamins nach Beispiel 36.3 und 0.6 g (0.01 mol) fein gepulvertes KOH werden 15 Minuten in DMSO gerührt, anschließend mit 1.2 g (0.01 mol) 2-Fluorphenylacetylen 4 Stunden bei 70°C gerührt, mit Wasser versetzt und extraktiv mit Methylenchlorid aufgearbeitet. Nach dem Abziehen des Solvens wird der Rückstand an Kieselgel mit Methylenchlorid/Methanol (95 : 5) als Eluens flash-chromatographiert. Die so erhaltene Hauptfraktion von 2.5 g wird in 30 ml Methanol bei Raumtemperatur mit 0.5 g Pd/BaSO₄ als Katalysator bei 5 bar hydriert. Das Solvens wird abgezogen und der Rückstand an Kieselgel mit EssigesterlCyclöhexan , (25 : 75) flash-chromatographiert. Die saubere Fraktion wird mit der berechneten Menge Fumarsäure versetzt. Es erfolgt keine Salzbildung mit Fumarsäure, man erhält nach dem Abziehen des Solvens die Titelverbindung als amorphe Verbindung.

### Beispiel 37: 1-[2-(2,6-Dimethylbenzyloxy)-2-(2,6-dimethylphenyl)-ethyl]-piperidin

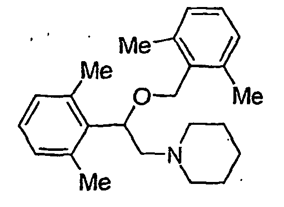

### Verfahren nach Stufe (xiii):

1.5 g (0.006 mol) des Ethanolamins nach Beispiel 36.3 werden in 30 ml absolutem THF gelöst, mit 0.9 g (0.0077 mol) Kaliumtertiärbutylat versetzt und 30 Minuten gerührt. Nach Zugabe von 1.5 g (0.0077 mol) 2.6-Dimethylbenzylbromid wird das Reaktionsgemisch weitere 60 Minuten bei Raumtemperatur gerührt, anschließend das Solvens abgezogen und der Rückstand zwischen Wasser und Methylenchlorid verteilt. Die organische Phase wird nach dem Waschen und Trocknen eingedampft und der ölige Rückstand von 2.2 g mittels Flash-Chromatographie an Kieselgel und Essigester/Cyclohexan (25 : 75) als Eluens aufgereinigt. Das erhaltene Öl wird in das Hydrochlorid überführt (Schmp. 185 -186 °C).

### Beispiel 38: 1-[2-Benzyloxy-2-(2,6-dimethylphenyl)-ethyl]-piperidin

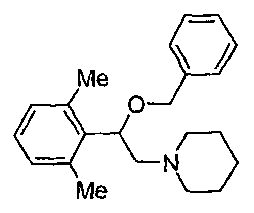

Herstellung analog Beispiel 37; Schmp.(Hydrochlorid): 197-199°C;

### Beispiel 39: 1-[2-(4-Brombenzyloxy)-2-(2,6-dimethylphenyl)-ethyl]-piperidin

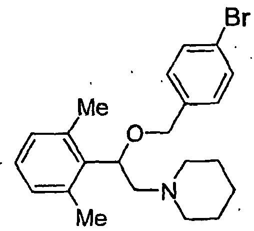

Herstellung analog Beispiel 37; Schmp.(Hydrochlorid): 152-154°C;

### Beispiel 40: 1-[2-(4-Chlorbenzyloxy)-2-(2,6-dimethylphenyl)-ethyl]-piperidin

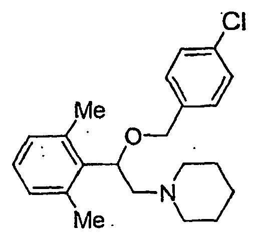

Herstellung analog Beispiel 37; Schmp.(Hydrochlorid): 134-135°C;

### Beispiel 41: 1-[2-(2,6-Dichlorbenzyloxy)-2-(2,6-dimethylphenyl)-ethyl]-piperidin

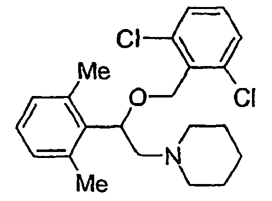

Herstellung analog Beispiel 37; Schmp.(Hydrochlorid): 225-227°C;

### Beispiel 42: 1-[2-(2,6-Difluorbenzyloxy)-2-(2,6-dimethylphenyl)-ethyl]-piperidin

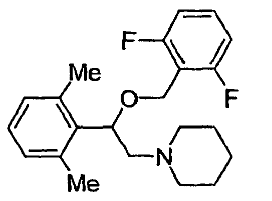

Herstellung analog Beispiel 37; Schmp.(Hydrochlorid): 183-185°C;

### Beispiel 43: 1-[2-(2-Chlor-4-brombenzyloxy)-2-(2,6-dimethylphenyl)-ethyl]-piperidin

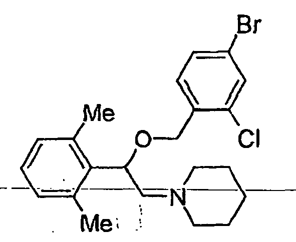

Herstellung analog Beispiel 37; Schmp.(Hydrochlorid): 222-224°C;

### Beispiel 44: 1-[2-[3-(2,6-Difluorphenyl)-propoxy]2-(2,6-dimethylnhenyl)-ethyl]-imidazol

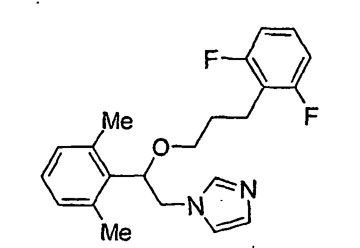

Herstellung analog Beispiel 37 unter Verwendung von Natriumhydrid als Hilfsbase; Schmp.(Hydrochlorid): 184-185°C;

### Beispiel 45: N-[3-(2,6-Difluorphenyl)-propyl]-[1-(2,6-dimethyl-phenyl)-2-piperidin-1-yl-ethyl]-N-cyclopropylmethyl-amin

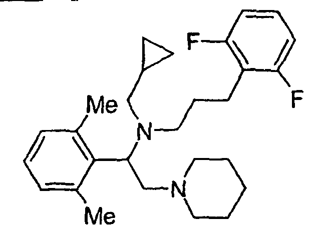

150 mg (0,39 mmol) [3-(2,6-Difluörphenyl)-propyl]-[1-(2,6-dimethyl-phenyl)-2-piperidin-1-yl-ethyl]-amin (entspricht Beispiel 35) werden in 3 ml Acetonitril/DMF 1:1 vorgelegt und mit 107 mg Kaliumcarbonat und 20 mg Kaliumiodid versetzt. Anschließend gibt man 0,04 ml (0,39 mmol) Brommethylcyclopropan hinzu und rührt 16 h bei 75°C. Danach wird über Kieselgur abfiltriert, die Lösung eingeengt und der Rückstand flashchromatographisch (Cyclohexan/Essigester 7:3) gereinigt.
MS: m/z 441 [(M+H)⁺].

### Beispiel 46: N-[3-(2,6-Difluorphenyl)-propyl]-[1-(2,6-dimethyl-phenyl)-2-piperidin-1-yl-ethy]-N-ethyl-amin

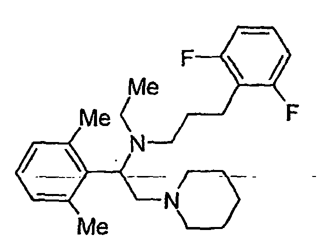

Herstellung analog Beispiel 45 ausgehend von Beispiel 35; MS: m/z 416 [(M+H)⁺].

### Beispiel 47: N-[3-(2,6-Difluorophenyl)-propyl]-[1-(2,6-dimethyl-phenyl)-2-piperidin-1-yl-ethyl]-N-isopentyl-amin

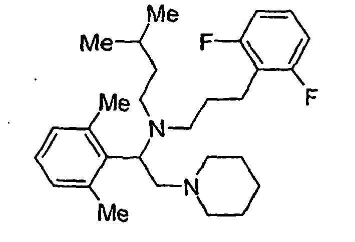

Herstellung analog Beispiel 45 ausgehend von Beispiel 35; MS: m/z 457 [(M+H)⁺].

### Beispiel 48: N-[3-(2,6-Difluorphenyl)-propyl]-[1-(2,6-dimethyl-phenyl)-2-piperidin-1-yl-ethyl]-N-isobutyl-amin

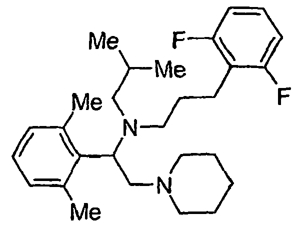

Herstellung analog Beispiel 45 ausgehend von Beispiel 35; MS: m/z 443 [(M+H)⁺].

### Beispiel 49: N-[3-(2,6-Difluorphenyl)-propyl]-[1-(2,6-dimethyl-phenyl)-2-pyrrolidin-1-yl-ethyl]-N-isobutyl-amin

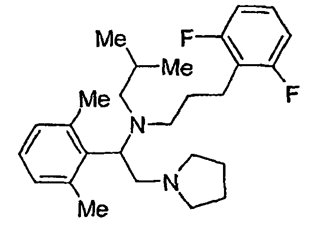

Herstellung analog Beispiel 45 ausgehend von Beispiel 34; MS: m/z 429 [(M+H)⁺].

### Beispiel 50: N-[3-(2,6-Difluorphenyl)-propyl]-[1-(2,6-dimethyl-phenyl)-2-pyrrolidin-1-yl-ethyl]-N-isopentyl-amin

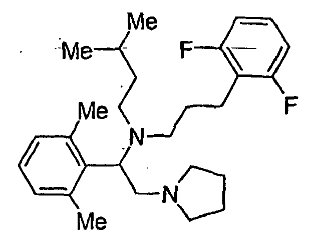

Herstellung analog Beispiel 45 ausgehend von Beispiel 34; MS: m/z 443 [(M+H)⁺].

### Beispiel 51: N-[3-(2,6-Difluorphenyl)-propyl]-[1-(2,6-dimethyl-phenyl)-2-pyrrolidin-1-yl-ethyl]-N-cyclopropylmethyl-amin

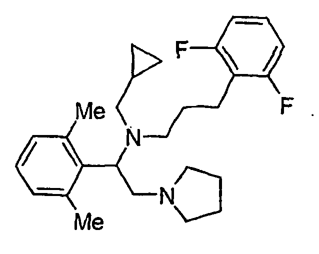

Herstellung analog Beispiel 45 ausgehend von Beispiel 34; MS: m/z 427 [(M+H)⁺].

### Beispiel 52: N-[3-(2,6-Difluorphenyl)-propyl]-[1-(2,6-dimethyl-phenyl)-2-piperidin-1-yl-ethyl]-N-acetyl-amin

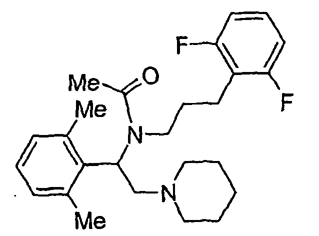

150 mg (0,39 mmol) [3-(2,6-Difluorphenyl)-propyl]-[1-(2,6-dimethyl-phenyl)-2-piperidin-1-yl-ethyl]-amin (entspricht Beispiel 35) werden in 3 ml THF vorgelegt. Danach werden 0,07 ml DIPEA und 0,02 ml (0,39 mmol) Essigsäure zugesetzt und zuletzt 124,6 mg (0,39 mmol) TBTU zugegeben. Dann wird 6 h bei RT gerührt; das Lösungsmittel entfernt, mit Essigester und ges. NaHCO₃ - Lsg. versetzt und 15 min rühren lassen. Die Phasen werden getrennt und die org. Phase noch 2 mal mit NaHCO₃-Lsg. und 2 mal mit ges. NaCI-Lösung gewaschen. Die organische Phase wird getrocknet und eingeengt und Rückstand flashchromatographisch (Cyclohexan/Essigester 7:3) gereinigt.
MS: m/z 429 [(M+H)⁺].

### Beispiel 53: N-[3-(2,6-Difluorphenyl)-propyl]-[1-(2,6-dimethyl-phenyl)-2-piperidin-1-yl-ethyl]-N-propionyl-amin

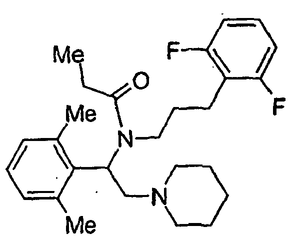

Herstellung analog Beispiel 52 ausgehend von Beispiel 35; MS: m/z 443 [(M+H)⁺].

### Beispiel 54: N-[3-(2,6-Difluorphenyl)-propyl]-[1-(2,6-dimethyl-phenyl)-2-piperidin-1-yl-ethyl]-N-formyl-amin

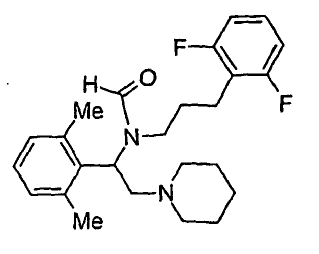

Herstellung analog Beispiel 52 ausgehend von Beispiel 35; MS: m/z 415 [(M+H)⁺].

### Beispiel 55: N-[3-(2,6-Difluorphenyl)-Propyl]-[1-(2,6-dimethyl-phenyl)-2-pyrrolidin-1-yl-ethyl]-N-formyl-amin

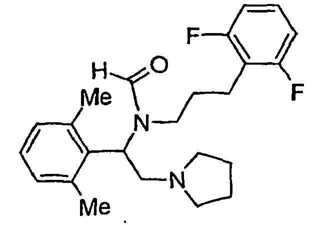

Herstellung analog Beispiel 52 ausgehend von Beispiel 34; MS: m/z 401 [(M+H)⁺].

### Beispiel 56: N-[3-(2,6-Difluorohenyl)-propyl]-[1-(2,6-dimethyl-phenyl)-2-pyrrolidin-1-yl-ethyl]-N-acetyl-amin

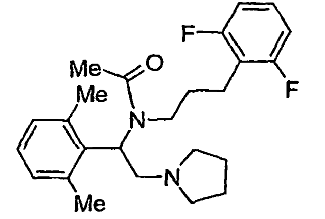

Herstellung analog Beispiel 52 ausgehend von Beispiel 34; MS: m/z 416 [(M+H)⁺].

### Beispiel 57: N-[3-(2,6-Difluorphenyl)-propyl]-[1-(2,6-dimethyl-phenyl)-2-pyrrolidin-1-yl-ethyl]-N-propionyl-amin

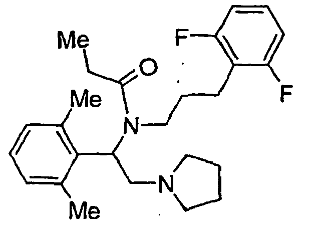

Herstellung analog Beispiel 52 ausgehend von Beispiel 35; MS: m/z 430 [(M+H)⁺].

### Beispiel 58: [3-(2,6-Difluorphenyl)-propyl]-[1-(2,6-dimethyl-phenyl)-2-cyclohexylamin-ethyl]-amin

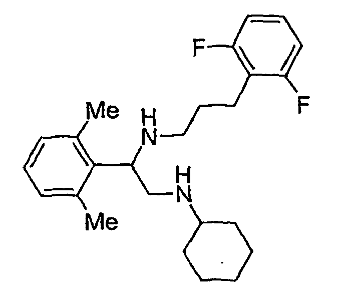

Herstellung analog Beispiel 34.4, jedoch zuvor reduktive Aminierung mit Cyclohexanon analog Beispiel 2; MS: m/z 400 [(M+H)⁺].

### Beispiel 59: N-[2-[3-(2,6-Difluorphenyl)-propoxy]-2-(2,6-dimethylphenyl)-ethyl]-N-methyl-N-(1-cyclohexen-4-yl-methyl)-amin

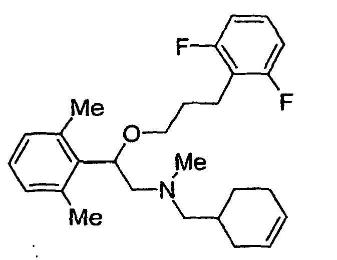

Herstellung analog Beispiel 5. Es wurde die Monomethylverbindung erhalten; Öl; MS: m/z 428 [(M+H)⁺].

### Beispiel 60: 1-[2-[3-(2,6-Difluorphenyl)-propoxy]-2-(2,4,6-trimethylphenyl)-ethyl]-piperidin

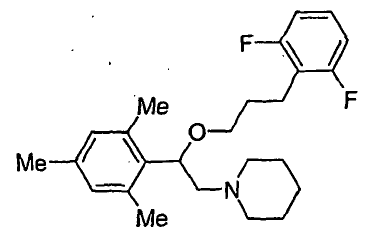

Herstellung analog Beispiel 6; Öl; MS: m/z 401 [(M+H)⁺].

### Beispiel 61: 1-[2-[3-(2,6-Difluorphenyl)-propoxy]-2-(2,4,6-trimethylphenyl)-ethyl]-1-methyl-piperidiniumiodid

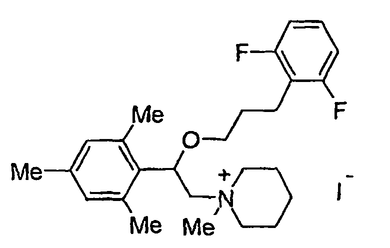

Herstellung analog Beispiel 7; Öl.

### Beispiel 62: [2-[3(2,6-Difluorphenyl)-propoxy]-2-(2,4,6-trimethylphenyl)-ethyl]-dimethyl-amin

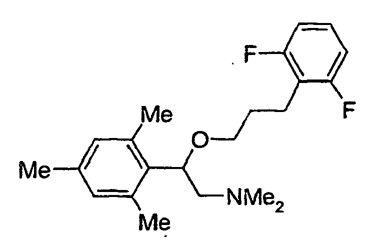

Herstellung analog Beispiel 8; Öl; MS: m/z 401 [(M+H)⁺].

### Beispiel 63: [2-[3-(2,6-Difluorphenyl)-propoxy]-2-(2,4,6-dimethylphenyl)-ethyl]-amin

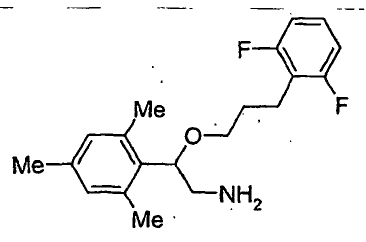

Herstellung analog Beispiel 1; Öl; MS: m/z 334 [(M+H)⁺].

### Beispiel 64: [2-[3-(2,6-Difluorphenyl)-propoxy]-2-(2,4,6-dimethylphenyl)-ethyl]-trimethylammonium iodid

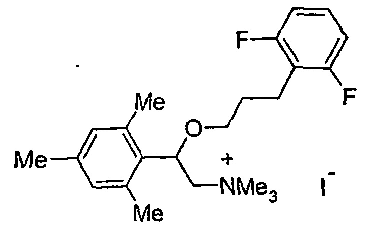

Herstellung analog Beispiel 5; gelbe Kristalle.

### Beispiel 65: N-[2-[3-(2,6-Difluorphenyl)-propoxy]-2-(2,6-dimethylphenyl)-ethyl]-N-(3-phenylpropyl)-N,N-dimethyl-ammonium iodid

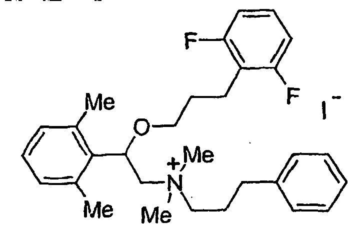

Herstellung analog Beispiel 5; Öl; MS: m/z 466 [(M+H)⁺].

### Beispiel 66: N-[2-[3-(2,6-Difluorphenyl)-propoxy]-2-(2,6-dimethylphenyl)-ethyl]-N-methyl-N-cyclohexyl-amin

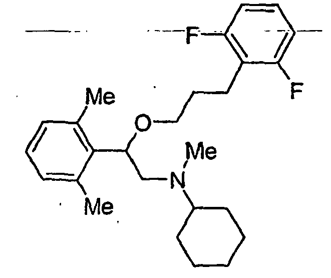

Herstellung analog Beispiel 4; Öl; MS: m/z 416 [(M+H)⁺]

### Beispiel 67: N-[2-[3-(2,6-Difluorphenyl)-propoxy]-2-(2,6-dimethylphenyl)-ethyl]-N-n-propyl-amin

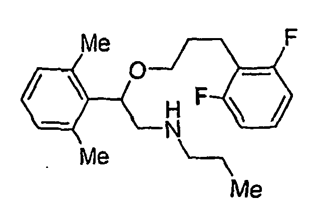

Herstellung analog Beispiel 3; Öl; MS: m/z 362 [(M+H)⁺].

### Beispiel 68: N-[2-[3-(2,6-Difluorphenyl)-propoxy]-2-(2,6-dimethylphenyl)-ethyl]-N-(3-phenyl-ethyl)-amin

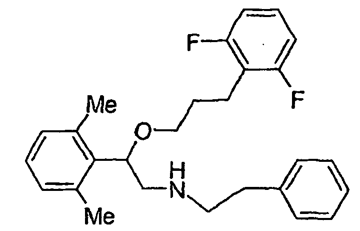

Herstellung analog Beispiel 3; Öl; MS: m/z 424 [(M+H)⁺].

### Beispiel 69: N-[2-[3-(2,6-Difluorphenyl)-propoxy]-2-(2,6-dimethylphenyl)-ethyl]-N,N-bis(3-phenyl-propyl)-amin

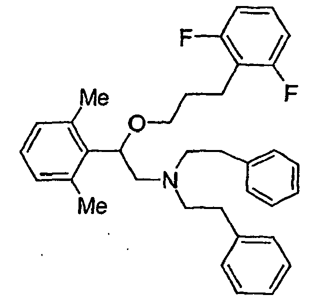

Herstellung analog Beispiel 2; Öl; MS: m/z 528 [(M+H)⁺].

### Beispiel 70: N-[2-[3-(2,6-Difluorphenyl)-propoxy]-2-(2,6-dimethylphenyl)-ethyl]-N-(2-methyl-but-1-yl)-N-ethyl-amin

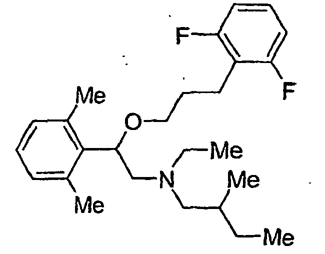

Herstellung analog Beispiel 5; Öl; MS: m/z 418 [(M+H)⁺].

### Beispiel 71: N-[2-[3-(2,6-Difluorphenyl)-propoxy]-2-(2,6-dimethylphenyl)-ethyl]-N-(3,3-dimethyl-but-1-yl)-N-ethyl-amin

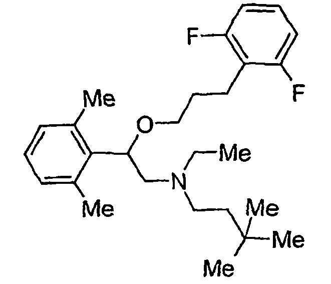

Herstellung analog Beispiel 5; Öl; MS: m/z 432 [(M+H)⁺].

### Beispiel 72: N-[2-[3-(2,6-Difluorphenyl)-propoxy]-2-(2,6-dimethylphenyl)-ethyl]-N-benzyl-N-ethyl-amin

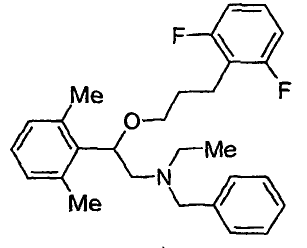

Herstellung analog Beispiel 5; Öl; MS: m/z 437 [(M+H)⁺].

### Beispiel 73: N-[2-[3-(2,6-Difluophenyl)-propoxy]-2-(2,6-dimethylphenyl)-ethyl]-N-isobutyl-N-ethyl-amin

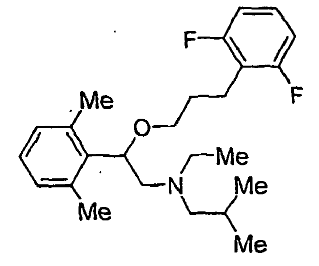

Herstellung analog Beispiel 5; Öl; MS: m/z 404 [(M+H)⁺].

### Beispiel 74: N-[2[3-(2,6-Difluorphenyl)-propoxy]-2-(2,6-dimethylphenyl)-ethyl]-N-(2,2-dimethyl-prop-1-yl)-N-ethyl-amin

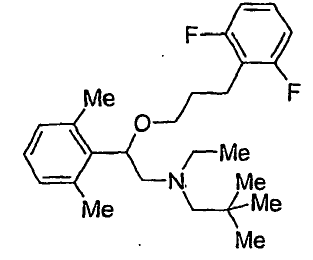

Herstellung analog Beispiel 5; Öl; MS: m/z 418 [(M+H)⁺].

### Beispiel 75:N-[2-[3-(2,6-Difluorphenyl)-propoxy]-2-(2,6-dimethylphenyl)-ethyl]-N-(2,2-dimethyl-prop-1-yl)-N,N-dimethyl-ammoniumiodid

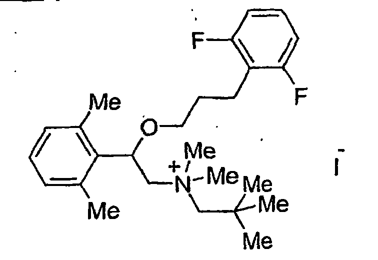

Herstellung analog Beispiel 5; Öl; MS: m/z 418 [(M+H)⁺].

### Beispiel 76: N-[2-[3-(2,6-Difluorphenyl)-propoxy]-2-(2,6-dimethytphenyl)-ethyl]-N-cyclohexyl-N,N-dimethyl-ammonlumiodid

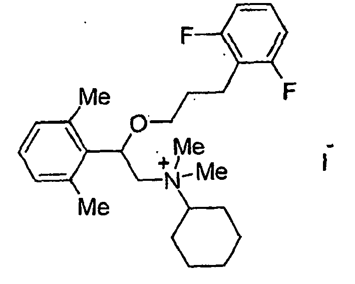

Herstellung analog Beispiel 5; Öl; MS: m/z 430 [M⁺].

### Beispiel 77: N-[2-[3-(2,6-Difluorphenyl)-propoxy]-2-(2,6-dimethyphenyl)-ethyl]-N-(2-trifluormethyl-ethyl)-N,N-dimethyl-ammoniumiodid

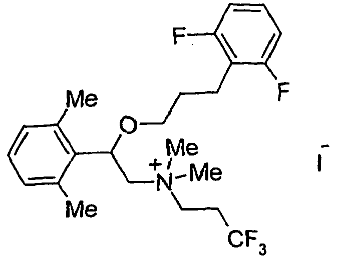

Herstellung analog Beispiel 5; Öl; MS: m/z 444 [(M+H)⁺].

### Beispiel 78: N-[2-[3-(2,6 Difluorphenyl)-propoxy]-2-(2,6-dimethylphenyl)-ethyl]-N-(4-isopropenyl-cyclohexen-1-yl-methyl)-N,N-dimethyl-ammoniumiodid

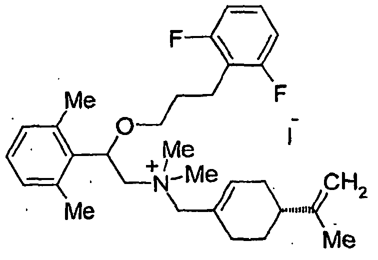

Herstellung analog Beispiel 5; Öl; MS: m/z 482 [M⁺].

### Beispiel 79: N-[2-[3-(2,6-Difluorphenyl)-propoxy]-2-(2,6-dimethylphenyl-ethyl]-N-ethyl-amin

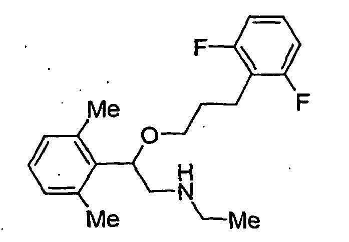

Herstellung analog Beispiel 3; Öl; MS: m/z 348 [(M+H)⁺].

### Beispiel 80: N-[2-[3-(2,6-Difluorphenyl)-propoxy]-2-(2,6-dimethylphenyl)-ethyl]-N-(3-pheynl-propyl)-amin

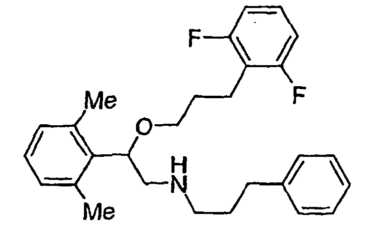

Herstellung analog Beispiel 3; ÖI; MS: m/z 438 [(M+H)⁺].

### Beispiel 81: N-[2-[3-(2,6-Difluorphenyl)-propoxy]-2-(2,6-dimethylphenyl)-ethyl]-N-(4-penten-1-yl)-amin

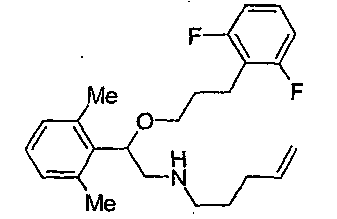

Herstellung analog Beispiel 3; Schmp.: 116°C; MS: m/z 338 [(M+H)⁺].

### Beispiel 82: 1-[2-[3(2,6-Difluorphenyl)-propoxy]-2-(2,6-dimethilphenyl)-ethyl]-1-methyl-piperidiniumiodid

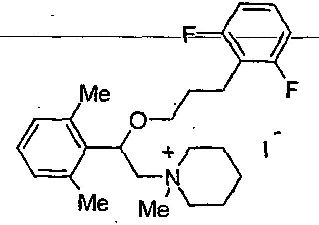

Herstellung analog Beispiel 7; Schmp.: 130 (Zers.); MS: m/z 402 [M⁺].

Die erfindungsgemäßen Verbindungen können oral, transdermal, intrathecal, inhalativ oder parenteral verabreicht werden und liegen hierbei als aktive Bestandteile in üblichen Darreichungsformen vor. Die erfindungsgemäßen Verbindungen können allein oder in Kombination mit anderen erfindungsgemäßen Wirkstoffen, gegebenenfalls auch in Kombination mit weiteren pharmakologisch aktiven Wirkstoffen, zur Anwendung gelangen. Geeignete Anwendungsformen sind beispielsweise Tabletten, Kapseln, Zäpfchen, Lösungen, Säfte, Emulsionen oder dispersible Pulver. Entsprechende Tabletten können beispielsweise durch Mischen des oder der Wirkstoffe mit bekannten Hilfsstoffen, beispielsweise inerten Verdünnungsmitteln, wie Calciumcarbonat, ,Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren Schichten bestehen.

Im folgenden sind einige Beispiele für pharmazeutische Zubereitungen mit dem Wirkstoff angegeben:

**Tabletten:**

| | |
|---|---|
| Wirkstoff gemäß allgemeiner Formel **1** oder **1-Y** | 20 mg |
| Magnesiumstearat | 1 mg |
| Laktose | 190 mg |

**Lösung zur Injektion**

| | |
|---|---|
| Wirkstoff gemäß allgemeiner Formel **1** oder **1-Y** | 0,3 mg |
| Natriumchlorid | 0,8 mg |
| Benzalkoniumchlorid | 0,01 mg |
| Aqua ad injectionem ad 100 ml | |

Eine ähnliche Lösung zu der oben aufgeführten ist geeignet für die nasale Applikation in einem Spray, oder in Kombination mit einem Gerät, das ein Aerosol mit einer Partikelgröße vorzugsweise zwischen 2 und 6 µM, zur Anwendung über die Lunge, erzeugt.

### Lösung zur Infusion

Eine 5 gewichtsprozentige Xylit- oder eine Kochsalzlösung, die beispielseise den Wirkstoff in einer Konzentration von 2 mg/ml enthält, wird mit einem Natriumacetat-Puffer auf einen pH-Wert von ca. 4 eingestellt.

Derartige Infusionslösungen können einen Gehalt an Wirkstoff gemäß der allgemeinen Formel 1 bezogen auf die Gesamtmasse der pharmazeutischen Zubereitung in einem Bereich von 0,001 bis 5 Gew.-%, vorzugsweise in einem Bereich von 0,001 bis 3 Gew.-% und besonders bevorzugt in einem Bereich von 0,01 bis 1 Gew.-% aufweisen.

### Kapseln für die Inhalation

Der Wirkstoff gemäß der allgemeinen Formel wird in mikronisierter. Form (Partikelgröße im wesentlichen zwischen 2 und 6 µM), gegebenfalls unter Zusatz von mikronisierten Trägersubstanzen, etwa Laktose, in Hartgelatinekapseln gefüllt. Zur Inhalation dienen übliche Geräte für die Pulverinhalation. In jede Kapsel werden z.B. zwischen 0,2 und 20 mg Wirkstoff und 0 bis 40 mg Laktose eingefüllt.

### Inhalationsaerosol

| | |
|---|---|
| Wirkstoff gemäß allgemeiner Formel **1** oder **1-Y** | 1 Teil |
| Sojalecithin | 0,2 Teile |
| Treibgasmischung ad | 100 Teile |

## Patentansprüche

1. Verbindungen der allgemeinen Formel **1**, 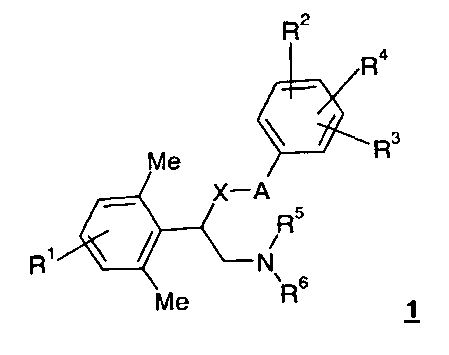 worin
R¹ Wasserstoff, Hydroxy, CF₃, NO₂, CN, Halogen, C₁-C₈-Alkyl oder C₁-C₈-Alkoxy;
R², R³ und R⁴ unabhängig voneinander Wasserstoff, C₁-C₈-Alkyl, Hydroxy, NO₂, CN, C₁-C₈-Alkyloxy, CF₃ oder Halogen;
R⁵ und R⁶ unabhängig voneinander Wasserstoff oder ein Rest ausgewählt aus der Gruppe bestehend aus C₁-C₈-Alkyl, C₂-C₈-Alkenyl, C₃-C₈-Alkinyl, C₃-C₈-Cycloalkyl, C₃-C₈-Cycloalkyl-C₁-C₆-alkylen, C₅-C₈-Cycloalkenyl, C₅-C₈-Cycloalkenyl-C₁-C₆-alkylen, C₆-C₁₀-Aryl und
C₆-C₁₀-Aryl-C₁-C₆-alkylen, der gegebenenfalls durch einen Rest ausgewählt aus der Gruppe bestehend aus C₁-C₆-Alkyl, C₂-C₆-Alkenyl, Halogen, C₁-C₆-Alkyloxy, -NH₂, -NH(C₁-C₄-Alkyl), -N(C₁-C₄-Alkyl)₂, Hydroxy, =O, -COOH, -CO-OC₁-C₄-alkyl, -CONH₂, -CONH(C₁-C₄-Alkyl), -CON(C₁-C₄-alkyl)₂ und CF₃ substituiert sein kann, oder
R⁵ und R⁶ gemeinsam mit dem Stickstoffatom einen gesättigten oder ungesättigten 5-, 6-, 7- oder 8-gliedrigen Heterocyclus, der gegebenenfalls ein oder zwei weitere Heteroatome ausgewählt aus Schwefel, Sauerstoff und Stickstoff enthält und gegebenenfalls ein-, zwei- oder dreifach durch einen Rest ausgewählt aus C₁-C₄-Alkyl, Hydroxy, =O, -COOH, -CO-OC₁-C₄-alkyl, -CONH₂, -CONH(C₁-C₄-Alkyl), -CON(C₁-C₄-alkyl)₂, Halogen und Benzyl substituiert sein kann;
X Sauerstoff, -NH-, -N(CHO)-, -N(CO-C₁-C₆-Alkyl)-, -N(C₁-C₆-Alkyl)- oder -N(C₃-C₆-Cycloalkyl-C₁-C₄-alkylen)-, bevorzugt Sauerstoff oder -NH-;
A ein Rest ausgewählt aus C₁-C₆-Alkylen, C₂-C₆-Alkenylen und C₃-C₆-Alkinylen, bedeuten.

2. Verbindungen der allgemeinen Formel **1** nach Anspruch 1,
worin
R¹ Wasserstoff, Halogen, C₁-C₆-Alkyl, CF₃ oder Methoxy;
R², R³ und R⁴ unabhängig voneinander Wasserstoff, C₁-C₆-Alkyl, C₁-C₆-Alkyloxy, CF₃ oder Halogen;
R⁵ und R⁶ unabhängig voneinander Wasserstoff oder ein Rest ausgewählt aus der Gruppe bestehend aus C₁-C₆-Alkyl, C₂-C₆-Alkenyl, C₃-C₆-Alkinyl, C₃-C₆-Cycloalkyl, C₃-C₆-Cycloalkyl-C₁-C₆-alkylen, C₅-C₆-Cycloalkenyl, C₅-C₆-Cycloalkenyl-C₁-C₆-alkylen, Phenyl und Phenyl-C₁-C₆-alkylen, der gegebenenfalls durch einen Rest ausgewählt aus der Gruppe C₁-C₄-Alkyl, C₂-C₄-Alkenyl, Halogen, C₁-C₄-Alkyloxy, Hydroxy, -CONH₂, =O und CF₃ substituiert sein kann
oder
R⁵ und R⁶ gemeinsam mit dem Stickstoffatom einen gesättigten oder ungesättigten 5-, 6- oder 7-gliedrigen Heterocyclus, der gegebenenfalls ein oder zwei weitere Heteroatome ausgewählt aus Schwefel, Sauerstoff und Stickstoff enthält und gegebenenfalls ein-, zwei- oder dreifach durch C₁-C₄-Alkyl, -CONH₂ oder Hydroxy substituiert sein kann;
X Sauerstoff, -NH-, -N(CHO)-, -N(CO-C₁-C₅-Alkyl)-, -N(C₁-C₅-Alkyl)- oder -N(C₃-C₆-Cycloalkyl-C₁-C₄-alkylen)-, bevorzugt Sauerstoff oder -NH-;
A C₁-C₅-Alkylen, C₂-C₄-Alkenylen oder C₃-C₄-Alkinylen, bevorzugt C₁-C₅-Alkylen, bedeuten.

3. Verbindungen der allgemeinen Formel **1** gemäß einem der Ansprüche oder 2,
worin
R¹ Wasserstoff, C₁-C₄-Alkyl oder CF₃;
R², R³ und R⁴ unabhängig voneinander Wasserstoff, C₁-C₄-Alkyl, CF₃ oder Halogen;
R⁵ und R⁶ unabhängig voneinander Wasserstoff, C₁-C₆-Alkyl, CF₃-C₁-C₆-Alkylen, bevorzugt ausgewählt aus -CH₂-CF₃ und -CH₂-CH₂-CF₃, C₂-C₆-Alkenyl, C₃-C₆-Alkinyl, C₃-C₆-Cycloalkyl, C₃-C₆-Cycloalkyl-C₁-C₆-alkylen, bevorzugt Cyclopropylmethyl oder Cyclohexenmethyl, Cyclohexenyl, Cyclohexenyl-C₁-C₆-alkylen, Propenyl-cyclohexenylen-C₁-C₆-alkylen, Phenyl, Phenyl-C₁-C₆-alkylen oder
R⁵ und R⁶ gemeinsam mit dem Stickstoffatom einen gesättigten oder ungesättigten 5-, 6- oder 7-gliedrigen Heterocyclus, der gegebenenfalls ein weiteres Stickstoffatom enthält und gegebenenfalls ein-, zwei- oder dreifach durch C₁-C₄-Alkyl, -CONH₂ oder Hydroxy substituiert sein kann;
X Sauerstoff, -NH-, -N(CHO)-, -N(CO-Methyl)-, -N(CO-Ethyl)-, -N(C₁-C₅-Alkyl)- oder -N(C₃-C₆-Cycloalkyl-methylen)-, bevorzugt Sauerstoff oder -NH-;
A -CH₂-, -CH₂-CH₂- oder -CH₂-CH₂-CH₂- bedeuten.

4. Verbindungen der allgemeinen Formel **1** gemäß einem der Ansprüche 1 bis 3,
worin
R¹ Wasserstoff oder Methyl;
R² und R³ unabhängig voneinander Wasserstoff, Methyl, Fluor, Chlor oder Brom;
R⁴ Wasserstoff, Fluor, Chlor oder Brom;
R⁵ und R⁶ unabhängig voneinander Wasserstoff, C₁-C₆-Alkyl, CF₃-C₁-C₆-Alkylen, bevorzugt -CH₂-CH₂-CF₃, C₂-C₆-Alkenyl, bevorzugt Butenyl und Pentenyl, C₃-C₆-Cycloalkyl, bevorzugt Cyclohexyl, C₃-C₆-Cycloalkyl-C₁-C₆-alkylen, bevorzugt Cyclopropylmethyl oder Cyclohexenmethyl, Cyclohexenyl, Cyclohexenyl-C₁-C₆-alkylen, bevorzugt Cyclohexenyl-CH₂-,
oder
R⁵ und R⁶ gemeinsam mit dem Stickstoffatom einen Heterocyclus ausgewählt aus der Gruppe bestehend aus Pyrrolidin, Piperidin, 1.2.3.6-Tetrahydropyridin und Azepan;
X Sauerstoff, -NH-, -N(CHO)-, -N(CO-Methyl)-, -N(CO-Ethyl)-, -N(Methyl)-, -N(Ethyl)-, -N(Propyl)-, -N(Butyl)-, -N(Pentyl)- oder -N(Cyclopropylmethyl)-, bevorzugt Sauerstoff oder -NH-;
A -CH₂-, -CH₂-CH₂- oder -CH₂-CH₂-CH₂- bedeuten.

5. Verbindungen der allgemeinen Formel **1** gemäß einem der ansprüche 1 bis 4,
worin
R¹ Wasserstoff oder Methyl;
R² und R³ unabhängig voneinander Wasserstoff, Methyl, Fluor, Chlor oder Brom;
R⁴ Wasserstoff, Fluor, Chlor oder Brom;
R⁵ und R⁶ unabhängig voneinander Wasserstoff, Methyl, Propyl, Butyl, Hexyl, Cyclopropylmethyl oder Cyclohexenmethyl, oder
R⁵ und R⁶ gemeinsam mit dem Stickstoffatom einen Heterocyclus ausgewählt aus der Gruppe bestehend aus Pyrrolidin, Piperidin, 1.2.3.6-Tetrahydropyridin und Azepan;
X Sauerstoff, -NH-, -N(CHO)-, -N(CO-Methyl)-, -N(CO-Ethyl)-, -N(Ethyl)-, -N(Propyl)-, -N(Butyl)-, -N(Pentyl)- oder -N(Cyclopropylmethyl)-, bevorzugt Sauerstoff oder -NH-;
A -CH₂-, -CH₂-CH₂- oder -CH₂-CH₂-CH₂- bedeuten.

6. Verbindungen der allgemeinen Formel **1** gemäß einem der Ansprüche 1 bis 5,
worin
R¹ Wasserstoff oder Methyl;
R² und R³ unabhängig voneinander Wasserstoff oder Fluor;
R⁴ Wasserstoff;
R⁵ und R⁶ unabhängig voneinander Wasserstoff, Methyl, Butyl, Hexyl oder Cyclohexenmethyl, oder
R⁵ und R⁶ gemeinsam mit dem Stickstoffatom Piperidin und 1.2.3.6-Tetrahydropyridin;
X Sauerstoff oder -NH-;
A -CH₂-CH₂- oder -CH₂-CH₂-CH₂- bedeuten.

7. Verbindungen der allgemeinen Formel **1** gemäß einem der Ansprüche 1 bis 6, in denen R¹ Wasserstoff bedeutet, in denen R² und R³ in ortho-Position angeordnet sind.

8. Verbindungen der allgemeinen Formel **1** gemäß einem der Ansprüche 1 bis 6, in denen R¹ Methyl bedeutet und in para-Position steht, sowie in denen R² und R³ in ortho-Position angeordnet sind.

9. Verbindungen der Formel **1** gemäß einem der Ansprüche 1 bis 8, in Form der einzelnen optischen Isomeren, Mischungen der einzelnen Enantiomeren oder Racemate sowie in Form der freien Basen oder der entsprechenden Säureadditionssalze mit pharmakologisch unbedenklichen Säuren.

10. Quartäre Ammoniumverbindungen der Formel **1-Y** 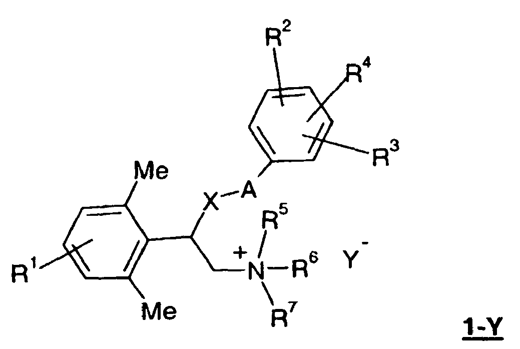 worin die Reste A, X, R¹, R², R³ und R⁴ die in den Ansprüchen 1 bis 8 genannten Bedeutungen haben können, R⁵ und R⁶ die die in den Ansprüchen 1 bis 8 genannten Bedeutungen, aber nicht Wasserstoff, haben können, R⁷ C₁-C₄-Alkyl, bedeutet und Y für ein Halogenid steht.

11. Verbindungen der allgemeinen Formel **1-Y** nach Anspruch 10, in denen R¹ Wasserstoff bedeutet, in denen R² und R³ in ortho-Position angeordnet sind.

12. Verbindungen der allgemeinen Formel **1-Y** nach Anspruch 10, in denen R¹ Methyl bedeutet und in para-Position steht und in denen R² und R³ in ortho-Position angeordnet sind.

13. Pharmazeutische Zubereitung **gekennzeichnet durch** einen Gehalt an einer der Verbindungen nach einem der Ansprüche 1 bis 12 neben üblichen Hilfs- und Trägerstoffen.

14. Pharmazeutische Zubereitung nach Anspruch 13, **dadurch gekennzeichnet, daß** sie als Infusionslösung formuliert ist.

15. Verwendung von Verbindungen nach einem der Ansprüche1 bis 12 zur Herstellung eines Arzneimittels zur therapeutischen Behandlung von durch Übererregung bedingten Funktionsstörungen.

16. Verwendung von Verbindungen nach einem der Ansprüche 1 bis 12 zur Herstellung eines Arzneimittels zur therapeutischen Behandlung von Arrhythmien, Spasmen, kardiale und Gehirnischämien, Schmerzen sowie neurodegenerative Erkrankungen verschiedener Genese.

17. Verwendung nach Anspruch 15 oder 16 zur Herstellung eines Arzneimittels zur therapeutischen Behandlung von Epilepsie, Hypoglykämie, Hypoxie, Anoxie, Gehirntrauma, Gehirnoedem, Gehirn-Schlaganfall, perinatale Asphyxie, , Degenerationen des Cerebellums, amyotrophe laterale Skler.ose, Morbus . Huntington, Morbus Alzheimer, Morbus Parkinson, Zyklophrenie, Hypotonie, . Herzinfakt, Herzrhythmusstörungen, Angina pectoris, chronischem Schmerz, neuropathischer Schmerz sowie Lokalanaesthesie.

18. Verfahren zur Herstellung einer Verbindung der Formel **1**, 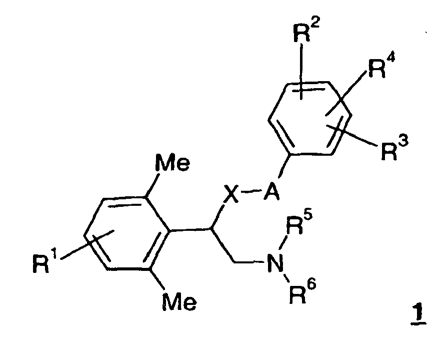 worin die Reste A, R¹, R², R³, R⁴, R⁵ und R⁶ die in den Ansprüchen 1-8 genannten Bedeutungen aufweisen können und in denen X für Sauerstoff steht, **dadurch gekennzeichnet, daß** eine Verbindung der Formel **6** 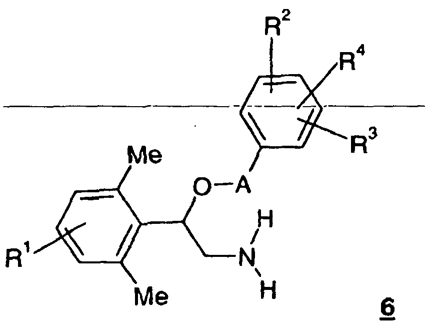 worin die Reste A, R¹, R², R³ und R⁴ die in den Ansprüchen 1-8 genannten Bedeutungen aufweisen können, in einem organischen Lösemittel in Gegenwart einer anorganischen oder organischen Base mit einem geeigneten Alkylierungsmittel, in dem die Alkylgruppe die in den Ansprüchen 1 bis 8 genannten Definitionen für R⁵ und R⁶ aufweist, zu einer Verbindung der Formel **1** umgesetzt wird oder indem ein Amin der Formel **6** durch reduktive Aminierung mit einer geeigneten Carbonylverbindungen in Gegenwart eines Reduktionsmittels zu einer Verbindung der Formel **1** erfolgt.

19. Verfahren nach Anspruch 18, **dadurch gekennzeichnet, daß** die Verbindung der Formel **6** 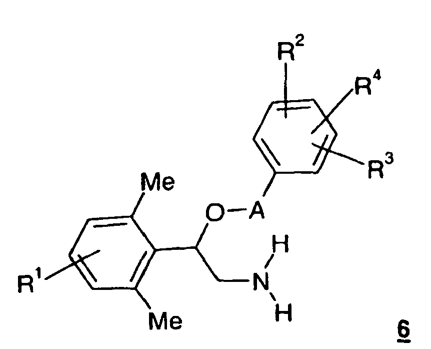 worin die Reste A, R¹, R², R³ und R⁴ die in den Ansprüchen 1-8 genannten Bedeutungen aufweisen können, **dadurch** erhalten wird, daß eine Verbindung der Formel **2** 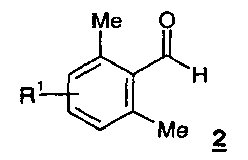 worin R¹ die in den Ansprüchen 1 bis 8 genannten Bedeutungen aufweisen kann, in einem ersten Schritt in Gegenwart einer Lewissäure in Trimethylsilylcyanid aufgenommen wird, die erhaltene Mischung mittels eines geeigneten wasserfreien organischen Lösemittel verdünnt und anschließend mittels eines geeigneten Reduktionsmittels zu einer Verbindung der Formel **3** 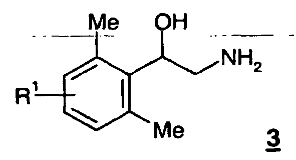 reduziert wird, diese in einem zweiten Schritt, gegebenenfalls nach Enantiomerentrennung durch Aufnehmen in einem geeigneten organischen Lösemittel in Gegenwart einer geeigneten organischen oder anorganischen Base mittels Trifluoressigsäureanhydrid zu einer Verbindung der Formel **4** 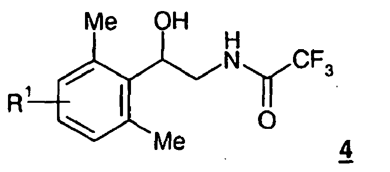 umgesetzt wird und diese abschließend in einem geeigneten organischen Lösemittel gelöst und in Gegenwart einer geeigneten organischen Base mit einer gegebenenfalls in einem geeigneten organischen Lösemittel gelösten Verbindung der Formel **5** 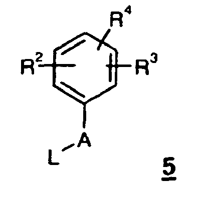 worin die Reste R², R³ und R⁴ die in den Ansprüchen 1-8 genannten Bedeutungen aufweisen können, zu einer Verbindung der Formel **6** umgesetzt wird.

20. Verfahren nach Anspruch 18, **dadurch gekennzeichnet, daß** die Verbindung der Formel **6** 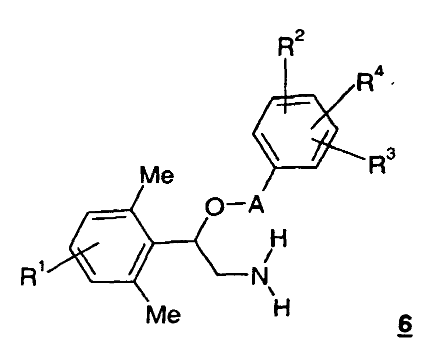 worin die Reste A, R¹, R², R³ und R⁴ die in den Ansprüchen 1-8 genannten Bedeutungen aufweisen können, **dadurch** erhalten wird, daß eine Verbindung der Formel **2** 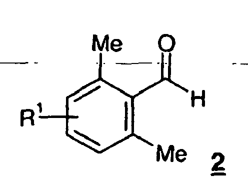 worin R¹ die in den Ansprüchen 1 bis 8 genannten Bedeutungen aufweisen kann, in einem ersten Schritt mittels Nitromethan in Eisessig bei erhöhter Temperatur, zu einer Verbindung der Formel **7** 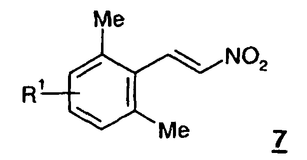 umgesetzt wird, diese in einem geeigneten organischen Lösemittel mittels eines Alkohols **8** 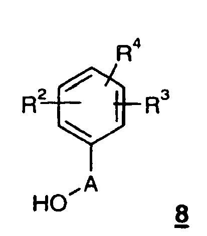 worin die Reste R², R³ und R⁴ die in den Ansprüchen 1-8 genannten Bedeutungen aufweisen können, in Gegenwart einer geeigneten Base zu einem Ether der Formel **9** 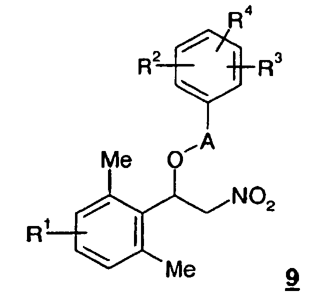 umgesetzt wird aus welchem sich reduktiv, vorzugsweise mittels Metall-katalysierter Reduktion die Verbindung der Formel **6** erhalten läßt.

21. Verfahren zur Herstellung von Verbindungen der Formel **1**, 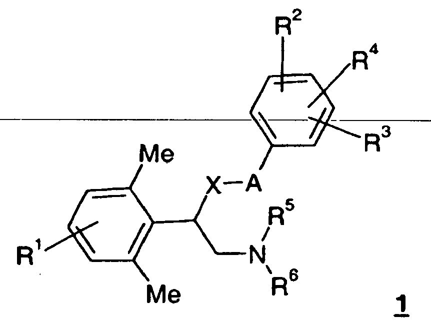 worin die Reste A, R¹, R², R³, R⁴, R⁵ und R⁶ die in den Ansprüchen 1-8 genannten Bedeutungen aufweisen können und in denen X für -NH- steht, **dadurch gekennzeichnet, daß** eine Verbindung der Formel **3** 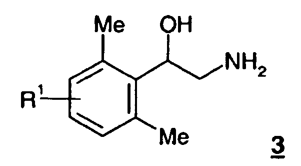 worin der Rest R¹ die in den Ansprüchen 1-8 genannten Bedeutungen aufweisen kann, in einem geeigneten organischen Lösemittel in Gegenwart einer geeigneten anorganischen oder organischen Base mittels eines geeigneten Alkylierungsmittel, in dem die Alkylgruppe die in den Ansprüchen 1 bis 8 genannten Definitionen für R⁵ und R⁶ aufweist, zu einer Verbindung der Formel **16** 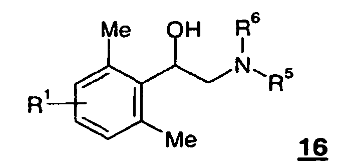 umgesetzt wird, diese im Falle von R⁵ oder R⁶ gleich Wasserstoff unter Verwendung geeigneter Schutzgruppen, mittels geeigneter Halogenierungsreabenzien, geeignet Sulfonsäurechloride oder geeigneter Sulfonsäureanhydride in Gegenwart geeignete Basen in inerten, geeigneten Lösemitteln zu einer Verbindung der Formel **17** 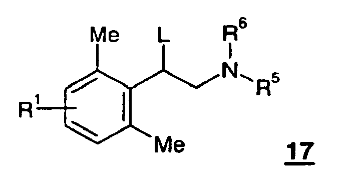 worin L für eine Abgangsgruppe, ausgewählt aus Chlor, Brom, lod, Methansulfonat, Trifluormethansulfonat und para-Toluolsulfonat steht, umgesetzt wird und diese in einem geeigneten organischen Lösemittel in Gegenwart einer geeigneten anorganischen oder organischen Base mittels einer Verbindung der Formel **18** 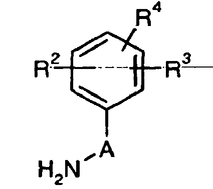 worin die Reste R², R³ und R⁴ die in den Ansprüchen 1-8 genannten Bedeutungen aufweisen können, zu einer Verbindung der Formel **1** umgesetzt wird.

22. Verfahren zur Herstellung einer Verbindungen der Formel **1**, 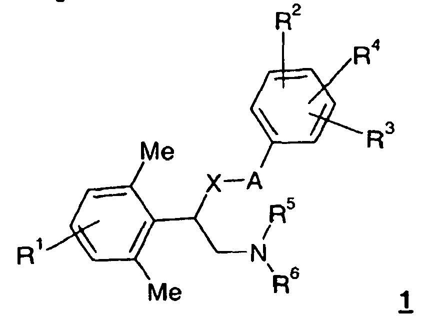 worin die Reste A, R¹, R², R³, R⁴, R⁵ und R⁶ die in den Ansprüchen 1-8 genannten Bedeutungen aufweisen können und in denen X für eine Gruppe ausgewählt aus -N(CHO)-, -N(CO-C₁-C₆-Alkyl)-, -N(C₁-C₆-Alkyl)- und -N(C₃-C₆-Cycloalkyl-C₁-C₄₋alkylen)- steht, **dadurch gekennzeichnet, daß** eine Verbindung der Formel **1**, in der X -NH-bedeutet in einem geeigneten organischen Lösemittel in Gegenwart einer geeigneten anorganischen oder organischen Base mittels eines geeigneten Alkylierungs-, Formylierungs- oder Acylierungsmittels umgesetzt wird.

## Claims

1. Compounds of general formula **1,** 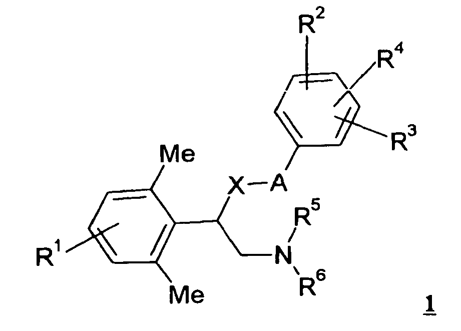 wherein
R¹ denotes hydrogen, hydroxy, CF₃, NO₂, CN, halogen, C₁-C₈₋alkyl or C₁-C₈-alkoxy;
R², R³ and R⁴ independently of one another denote hydrogen, C₁-C₈-alkyl, hydroxy, NO₂, CN, C₁-C₈-alkyloxy, CF₃ or halogen;
R⁵ and R⁶ independently of one another denote hydrogen or a group selected from among C₁-C₈-alkyl, C₂-C₈-alkenyl, C₃-C₈₋alkynyl,C₃-C₈-cycloalkyl, C₃-C₈-cycloalkyl-C₁-C₆-alkylene, C₅-C₈-cycloalkenyl, C₅-C₈-cycloalkenyl-C₁-C₆-alkylene, C₆-C₁₀-aryl and C₆-C₁₀-aryl-C₁-C₆-alkylene, which may optionally be substituted by a group selected from among C₁-C₆-alkyl, C₂-C₆-alkenyl, halogen, C₁-C₆-alkyloxy, -NH₂, -NH(C₁-C₄-alkyl), -N(C₁-C₄-alkyl)₂, hydroxy, =O, -COOH, -CO-OC₁-C₄-alkyl, -CONH₂, -CONH(C₁-C₄-alkyl), -CON(C₁-C₄-alkyl) ₂ and CF₃, or
R⁵ and R⁶ together with the nitrogen atom denote a saturated or unsaturated 5-, 6-, 7- or 8-membered heterocyclic group which optionally contains one or two further heteroatoms selected from sulphur, oxygen and nitrogen and may optionally be mono-, di- or trisubstituted by a group selected from C₁-C₄-alkyl, hydroxy, =O, -COOH, -CO-OC₁-C₄-alkyl, -CONH₂, -CONH(C₁-C₄-alkyl), -CON(C₁-C₄-alkyl)₂, halogen and benzyl;
X denotes oxygen, -NH-, -N(CHO)-, -N(CO-C₁-C₆-alkyl), -N(C₁-C₆-alkyl) or -N(C₃-C₆-cycloalkyl-C₁-C₄-alkylene), preferably oxygen or -NH-;
A denotes a group selected from C₁-C₆-alkylene, C₂-C₆₋alkenylene and C₃-C₆-alkynyiene.

2. Compounds of general formula **1** according to claim 1, wherein
R¹ denotes hydrogen, halogen, C₁-C₆-alkyl, CF₃ or methoxy;
R², R³ and R⁴ independently of one another denote hydrogen, C₁-C₆-alkyl, C₁-C₆-alkyloxy, CF₃ or halogen;
R⁵ and R⁶ independently of one another denote hydrogen or a group selected from among C₁-C₆-alkyl, C₂-C₆-alkenyl, C₃-C₆₋alkynyl, C₃-C₆-cycloalkyl, C₃-C₆-cycloalkyl-C₁-C₆-alkylene, C₅-C₆-cycloalkenyl, C₅-C₆-cycloalkenyl-C₁-C₆-alkylene, phenyl and phenyl-C₁-C₆-alkylene, which may optionally be substituted by a group selected from among C₁-C₄-alkyl, C₂-C₄-alkenyl, halogen, C₁-C₄-alkyloxy, hydroxy, -CONH₂, =O and CF₃
or
R⁵ and R⁶ together with the nitrogen atom denote a saturated or unsaturated 5-, 6- or 7-membered heterocyclic group which optionally contains one or two further heteroatoms selected from sulphur, oxygen and nitrogen and may optionally be mono-, di- or trisubstituted by C₁-C₄-alkyl, -CONH₂ or hydroxy;
X denotes oxygen, -NH-, -N(CHO)-, -N(CO-C₁-C₅-alkyl)-, -N(C₁-C₅-alkyl)- or -N(C₃-C₆-cycloalkyl-C₁-C₄-alkylene), preferably oxygen or -NH-;
A denotes C₁-C₅-alkylene, C₂-C₄-alkenylene or C₃-C₄-alkynylene, preferably C₁-C₅-alkylene.

3. Compounds of general formula **1** according to claim 1 or 2, wherein
R¹ denotes hydrogen, C₁-C₄-alkyl or CF₃;
R², R³ and R⁴ independently of one another denote hydrogen, C₁-C₄-alkyl, CF₃ or halogen,
R⁵ and R⁶ independently of one another denote hydrogen, C₁-C₆-alkyl, CF₃-C₁-C₆-alkylene, preferably selected from -CH₂-CF₃ and -CH₂-CH₂-CF₃, C₂-C₆-alkenyl, C₃-C₆-alkynyl, C₃-C₆₋cycloalkyl, C₃-C₆-cycloalkyl-C₁-C₆-alkylene, preferably cyclopropylmethyl or cyclohexenemethyl, cyclohexenyl, cyclohexenyl-C₁-C₆-alkylene, propenyl-cyclohexenylene-C₁-C₆₋alkylene, phenyl or phenyl-C₁-C₆-alkylene
or
R⁵ and R⁶ together with the nitrogen atom denote a saturated or unsaturated 5-, 6- or 7-membered heterocyclic group, which optionally contains another nitrogen atom and may optionally be mono-, di- or trisubstituted by C₁-C₄-alkyl, -CONH₂ or hydroxy;
X denotes oxygen, -NH-, -N(CHO)-, -N(CO-methyl)-, -N(CO-ethyl)-, -N(C₁-C₅-alkyl)- or -N(C₃-C₆-cycloalkyl-methylene)-, preferably oxygen or -NH;
A denotes -CH₂-, -CH₂-CH₂- or -CH₂-CH₂-CH₂-.

4. Compounds of general formula **1** according to one of claims 1 to 3, wherein
R¹ denotes hydrogen or methyl;
R² and R³ independently of one another denote hydrogen, methyl, fluorine, chlorine or bromine;
R⁴ denotes hydrogen, fluorine, chlorine or bromine;
R⁵ and R⁶ independently of one another denote hydrogen, C₁-C₆-alkyl, CF₃-C₁-C₆-alkylene, preferably -CH₂-CH₂-CF₃, C₂-C₆-alkenyl, preferably butenyl and pentenyl, C₃-C₆-cycloalkyl, preferably cyclohexyl, C₃-C₆-cycloalkyl-C₁-C₆-alkylene, preferably cyclopropylmethyl or cyclohexenemethyl, cyclohexenyl, cyclohexenyl-C₁-C₆-alkylene, preferably cyclohexenyl-CH₂-,
or
R⁵ and R⁶ together with the nitrogen atom denote a heterocyclic group selected from among pyrrolidine, piperidine, 1,2,3,6-tetrahydropyridine and azepan;
X denotes oxygen, -NH-, -N(CHO)-, -N(CO-methyl)-, -N(CO-ethyl)-, -N(methyl)-, -N(ethyl)-, -N(propyl)-, -N(butyl)-, -N(pentyl)- or -N(cyclopropylmethyl)-, preferably oxygen or -NH-;
A denotes -CH₂-, -CH₂-CH₂- or -CH₂-CH₂-CH₂-.

5. Compounds of general formula **1** according to one of claims 1 to 4, wherein
R¹ denotes hydrogen or methyl;
R² and R³ independently of one another denote hydrogen, methyl, fluorine, chlorine or bromine;
R⁴ denotes hydrogen, fluorine, chlorine or bromine;
R⁵ and R⁶ independently of one another denote hydrogen, methyl, propyl, butyl, hexyl, cyclopropylmethyl or cyclohexenemethyl, or
R⁵ and R⁶ together with the nitrogen atom denote a heterocyclic group selected from among pyrrolidine, piperidine, 1,2,3,6-tetrahydropyridine and azepan;
X denotes oxygen, -NH-, -N(CHO)-, -N(CO-methyl)-, -N(CO-ethyl)-, -N(ethyl)-, -N(propyl)-, -N(butyl)-, -N(pentyl)- or -N(cyclopropylmethyl)-, preferably oxygen or -NH-;
A denotes -CH₂-, -CH₂-CH₂- or -CH₂-CH₂-CH₂-.

6. Compounds of general formula **1** according to one of claims 1 to 5, wherein
R¹ denotes hydrogen or methyl;
R² and R³ independently of one another denote hydrogen or fluorine;
R⁴ denotes hydrogen;
R⁵ and R⁶ independently of one another denote hydrogen, methyl, butyl, hexyl or cyclohexenemethyl, or
R⁵ and R⁶ together with the nitrogen atom denote piperidine and 1,2,3,6-tetrahydropyridine;
X denotes oxygen or -NH-;
A denotes -CH₂-CH₂- or -CH₂-CH₂-CH₂-.

7. Compounds of general formula **1** according to one of claims 1 to 6, wherein R¹ denotes hydrogen, wherein R² and R³ are in the *ortho* position.

8. Compounds of formula **1** according to one of claims 1 to 6, wherein R¹ denotes methyl and is in the para position and wherein R² and R³ are in the *ortho* position.

9. Compounds of formula **1** according to one of claims 1 to 8, in the form of the individual optical isomers, mixtures of the individual enantiomers or racemates and in the form of the free bases or the corresponding acid addition salts thereof with pharmacologically acceptable acids.

10. Quaternary ammonium compounds of formula **1-Y** 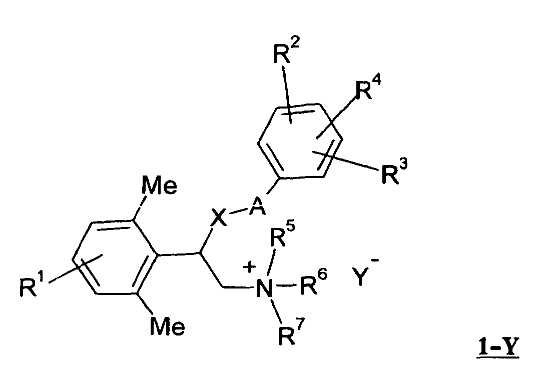 wherein the groups A, X, R¹, R², R³ and R⁴ may have the meanings given in claims 1 to 8, R⁵ and R⁶ may have the meanings given in claims 1 to 8, but not hydrogen, R⁷ denotes C₁-C₄-alkyl and Y denotes a halide.

11. Compounds of general formula **1-Y** according to claim 10 wherein R¹ denotes hydrogen, wherein R² and R³ are in the *ortho* position.

12. Compounds of general formula **1-Y** according to claim 10 wherein R¹ denotes methyl and is in the *para* position and wherein R² and R³ are in the *ortho* position.

13. Pharmaceutical preparation, **characterised in that** it contains one of the compounds according to one of claims 1 to 12 in addition to conventional excipients and carriers.

14. Pharmaceutical preparation according to claim 13, **characterised in that** it is formulated as a solution for infusion.

15. Use of compounds according to one of claims 1 to 12 for preparing a pharmaceutical composition for the therapeutic treatment of functional disorders caused by overstimulation.

16. Use of compounds according to one of claims 1 to 12 for preparing a pharmaceutical composition for the therapeutic treatment of arrhythmias, spasms, cardiac and cerebral ischaemias, pain and neurodegenerative disorders of various origins.

17. Use according to claim 15 or 16 for preparing a pharmaceutical composition for the therapeutic treatment of epilepsy, hypoglycaemia, hypoxia, anoxia, brain trauma, brain oedema, cerebral stroke, perinatal asphyxia, degeneration of the cerebellum, amyotropic lateral sclerosis, Huntington's disease, Alzheimer's disease, Parkinson's disease, cyclophrenia, hypotonia, cardiac infarct, heart rhythm disorders, angina pectoris, chronic pain, neuropathic pain and local anaesthesia.

18. Process for preparing a compound of formula **1**, 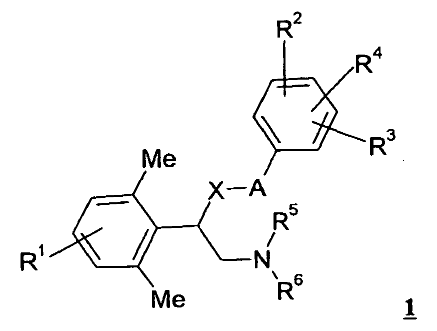 wherein the groups A, R¹, R², R³, R⁴, R⁵ and R⁶ may have the meanings given in claims 1 to 8 and wherein X denotes oxygen, **characterised in that** a compound of formula **6** 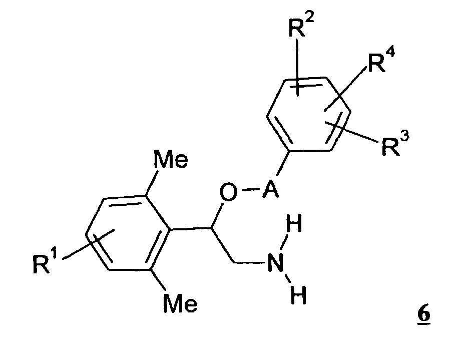 wherein the groups A, R¹, R², R³ and R⁴ may have the meanings given in claims 1 to 8, is reacted in an organic solvent in the presence of an inorganic or organic base with a suitable alkylating agent in which the alkyl group has the definitions given for R⁵ and R⁶ in claims 1 to 8, to obtain a compound of formula **1** or wherein an amine of formula **6** is converted into a compound of formula **1** by reductive amination with a suitable carbonyl compound in the presence of a reducing agent.

19. Process according to claim 18, **characterised in that** the compound of formula **6** 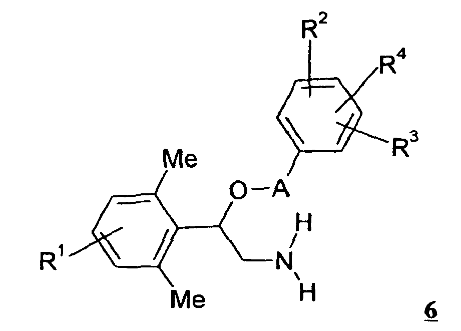 wherein the groups A, R¹, R², R³ and R⁴ may have the meanings given in claims 1 to 8, is obtained by taking up a compound of formula **2** 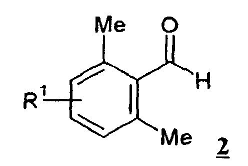 wherein R¹ may have the meanings given in claims 1 to 8, in trimethylsilylcyanide in a first step in the presence of a Lewis acid, the resulting mixture is diluted using a suitable anhydrous organic solvent and then reduced by means of a suitable reducing agent to form a compound of formula **3** 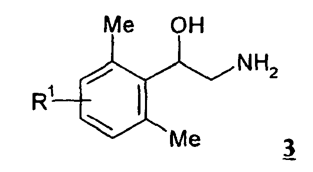 which is reacted with trifluoroacetic acid anhydride in a second step, optionally after separation of the enantiomers by taking up in a suitable organic solvent in the presence of a suitable organic or inorganic base, to form a compound of formula **4** 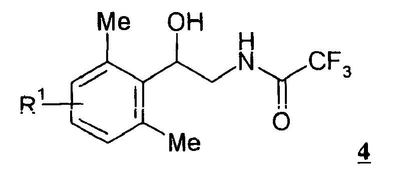 and this is finally dissolved in a suitable organic solvent and reacted in the presence of a suitable organic base with a compound of formula **5** 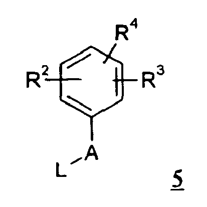 optionally dissolved in a suitable organic solvent
wherein the groups R², R³ and R⁴ may have the meanings given in claims 1 to 8, to form a compound of formula **6**.

20. Process according to claim 18, **characterised in that** the compound of formula **6** 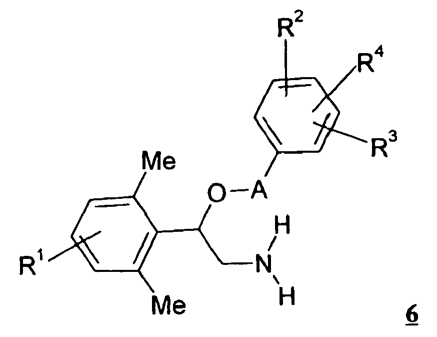 wherein the groups A, R¹, R², R³ and R⁴ may have the meanings given in claims 1 to 8, is obtained by reacting a compound of formula **2** 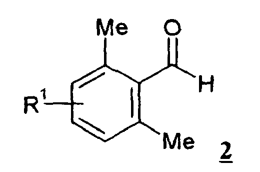 wherein R¹ may have the meanings given in claims 1 to 8, in a first step, using nitromethane in glacial acetic acid at elevated temperature, to obtain a compound of formula 7 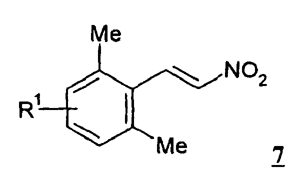 which is reacted in a suitable organic solvent by means of an alcohol **8** 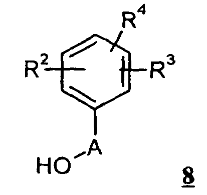 wherein the groups R², R³ and R⁴ may have the meanings given in claims 1 to 8, in the presence of a suitable base, to obtain an ether of formula **9** 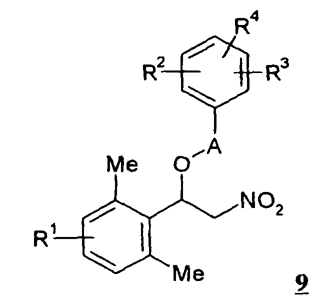 from which the compound of formula **6** may be obtained reductively, preferably by metal-catalysed reduction.

21. Process for preparing compounds of formula **1**, 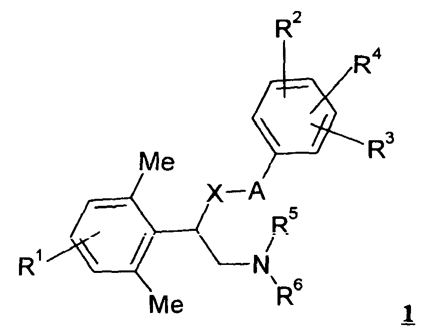 wherein the groups A, R¹, R², R³, R⁴, R⁵ and R⁶ may have the meanings given in claims 1 to 8 and wherein X denotes -NH-, **characterised in that** a compound of formula **3** 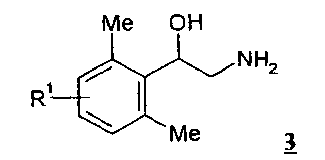 wherein the group R¹ may have the meanings given in claims 1-8, is reacted in a suitable organic solvent in the presence of a suitable inorganic or organic base using a suitable alkylating agent wherein the alkyl group has the definitions given for R⁵ and R⁶ in claims 1 to 8, to obtain a compound of formula **16** 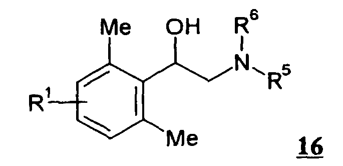 which is reacted, if R⁵ or R⁶ denotes hydrogen, using suitable protecting groups, by means of suitable halogenating reagents, suitable sulphonic acid chlorides or suitable sulphonic acid anhydrides in the presence of suitable bases in suitable inert solvents to obtain a compound of formula **17** 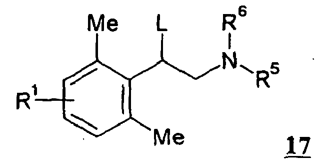 wherein L denotes a leaving group selected from among chlorine, bromine, iodine, methanesulphonate, trifluoromethanesulphonate and *para-*toluenesutphonate and this is reacted in a suitable organic solvent in the presence of a suitable inorganic or organic base using a compound of formula **18** 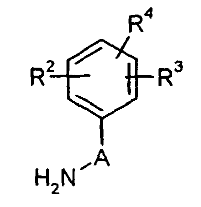 wherein the groups R², R³ and R⁴ may have the meanings given in claims 1 to 8, to obtain a compound of formula **1**.

22. Process for preparing a compound of formula **1**, 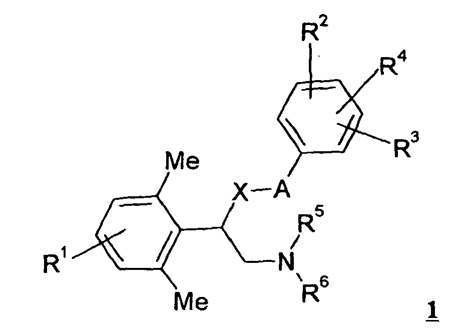 wherein the groups A, R¹, R², R³, R⁴, R⁵ and R⁶ may have the meanings given in claims 1 to 8 and wherein X denotes a group selected from -N(CHO)-, -N(CO-C₁-C₆-alkyl)-, -N(C₁-C₆-alkyl)- and -N(C₃-C₆-cycloalkyl-C₁-C₄₋alkylene), **characterised in that** a compound of formula 1 wherein X denotes -NH- is reacted in a suitable organic solvent in the presence of a suitable inorganic or organic base by means of a suitable alkylating, formylating or acylating agent.

## Revendications

1. Composés de formule générale **1** 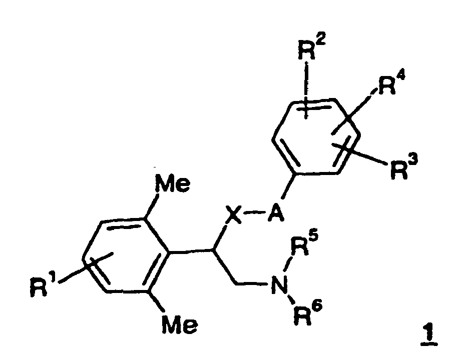 où
R¹ représente l'hydrogène, hydroxy, CF₃, NO₂, CN, halogène, C₁-C₈-alkyle ou C₁₋C₈-alcoxy ;
R², R³ et R⁴ représentent indépendamment les uns des autres l'hydrogène, C₁-C₈₋alkyle, hydroxy, NO₂, CN, C₁-C₈-alkyloxy, CF₃ ou halogène ;
R⁵ et R⁶ représentent indépendamment l'un de l'autre l'hydrogène ou un groupement choisi dans le groupe consistant en C₁-C₈-alkyle, C₂-C₈-alcényle, C₃₋Cg-alcynyle, C₃-C₈-cycloalkyle, C₃-C₈-cycloalkyl-C₁-C₆-alkylène, C₅-C₈₋cycloalcényle, C₅-C₈-cycloalcényl-C₁-C₆-alkylène, C₆-C₁₀-aryle et C₆-C₁₀-aryl-C₁₋C₆-alkylène, qui peut éventuellement être substitué par un groupement choisi dans le groupe consistant en C₁-C₆-alkyle, C₂-C₆-alcényle, halogène, C₁-C₆-alkyloxy, -NH₂, -NH(C₁-C₄-alkyle), -N(C₁-C₄-alkyle)₂, hydroxy, =O, -COOH, -CO-OC₁-C₄₋alkyle, -CONH₂, -CONH(C₁-C₄-alkyle), -CON(C₁-C₄-alkyle)₂ et CF₃, ou
R⁵ et R⁶ représentent avec l'atome d'azote un hétérocycle à 5, 6, 7 ou 8 chaînons saturé ou insaturé, qui contient éventuellement un ou deux hétéroatomes supplémentaires choisis parmi le soufre, l'oxygène et l'azote et peut éventuellement être substitué une, deux ou trois fois par un groupement choisi parmi C₁-C₄-alkyle, hydroxy, =O, -COOH, -CO-OC₁-C₄-alkyle, -CONH₂, -CONH(C₁-C₄-alkyle), -CON(C₁-C₄-alkyle)₂, halogène et benzyle ;
X représente l'oxygène, -NH-, -N(CHO)-, -N(CO-C₁-C₆-alkyle)-, -N(C₁-C₆₋alkyle)- ou -N(C₃-C₆-cycloalkyl-C₁-C₄-alkylène)-, de préférence l'oxygène ou -NH- ;
A représente un groupement choisi parmi C₁C₆-alkylène, C₂-C₆-alcénylène et C₃₋C₆-alcynylène.

2. Composés de formule générale **1** selon la revendication 1
où
R¹ représente l'hydrogène, halogène, C₁-C₆-alkyle, CF₃ ou méthoxy;
R², R³ et R⁴ représentent indépendamment les uns des autres l'hydrogène, C₁-C₆₋alkyle, C₁-C₆-alkyloxy, CF₃ ou halogène;
R⁵ et R⁶ représentent indépendamment l'un de l'autre l'hydrogène ou un groupement choisi dans le groupe consistant en C₁-C₆-alkyle, C₂-C₆-alcényle, C₃₋C₆-alcynyle, C₃-C₆-cycloalkyle, C₃-C₆-cycloalkyl-C₁-C₆-alkylène, C₅-C₆₋cycloalcényle, C₅-C₆-cycloalcényl-C₁-C₆-alkylène, phényle et phényl-C₁-C₆₋alkylène, qui peut éventuellement être substitué par un groupement choisi dans le groupe C₁-C₄-alkyle, C₂-C₄-alcényle, halogène, C₁-C₄-alkyloxy, hydroxy, -CONH₂, =O et CF₃, ou
R⁵ et R⁶ représentent avec l'atome d'azote un hétérocycle à 5, 6 ou 7 chaînons ; saturé ou insaturé, qui contient éventuellement un ou deux hétéroatomes supplémentaires choisis parmi le soufre, l'oxygène et l'azote et peut éventuellement être substitué une, deux ou trois fois par C₁-C₄-alkyle, -CONH₂ ou hydroxy ;
X représente l'oxygène, -NH-, -N(CHO)-, -N(CO-C₁-C₅-alkyle)-, -N(C₁-C₅₋alkyle)- ou N(C₃-C₆-cycloalkyl-C₁-C₄-alkylène)-, de préférence l'oxygène ou -NH- ;
A représente C₁-C₅-alkylène, C₂-C₄-alcénylène ou C₃-C₄-alcynylène, de préférence C₁-C₅-alkylène.

3. Composés de formule générale **1** selon l'une des revendications 1 ou 2
où
R¹ représente l'hydrogène, C₁-C₄-alkyle ou CF₃ ;
R², R³ et R⁴ représentent indépendamment les uns des autres l'hydrogène, C₁-C₄₋alkyle, CF₃ ou halogène ;
R⁵ et R⁶ représentent indépendamment l'un de l'autre l'hydrogène, C₁-C₆-alkyle, CF₃-C₁-C₆-alkylène, de préférence choisi parmi -CH₂-CF₃ et -CH₂-CH₂-CF₃, C₂₋C₆-alcényle, C₃-C₆-alcynyle, C₃-C₆-cycloalkyle, C₃-C₆-cycloalkyl-C₁-C₆-alkylène, de préférence cyclopropylméthyle ou cyclohexèneméthyle, cyclohexényle, cyclohexényl-C₁-C₆-alkylène, propényl-cyclohexénylène-C₁-C₆-alkylène, phényle, phényl-C₁-C₆-alkylène ou
R⁵ et R⁶ représentent avec l'atome d'azote un hétérocycle à 5, 6 ou 7 chaînons saturé ou insaturé, qui contient éventuellement un atome d'azote supplémentaire et qui peut éventuellement être substitué une, deux ou trois fois par C₁-C₄-alkyle, -CONH₂ ou hydroxy ;
X représente l'oxygène, -NH-, -N(CHO)-, -N(CO-méthyle)-, -N(CO-éthyle)-, -NH(C₁-C₅-alkyle)- ou -N(C₃-C₆-cycloalkyl-méthylène)-, de préférence l'oxygène
ou -NH- ;
A représente -CH₂-, -CH₂-CH₂- ou -CH₂-CH₂-CH₂-.

4. Composés de formule générale **1** selon l'une des revendications 1 à 3
où
R¹ représente l'hydrogène ou méthyle ;
R² et R³ représentent indépendamment l'un de l'autre l'hydrogène, méthyle, le fluor, le chlore ou le brome ;
R⁴ représente l'hydrogène, le fluor, le chlore ou le brome ;
R⁵ et R⁶ représentent indépendamment l'un de l'autre l'hydrogène, C₁-C₆-alkyle, CF₃-C₁-C₆-alkylène, de préférence -CH₂-CH₂-CF₃, C₂-C₆-alcényle, de préférence butényle et pentényle, C₃-C₆-cycloalkyle, de préférence cyclohexyle, C₃-C₆₋cycloalkyl-C₁-C₆-alkylène, de préférence cyclopropylméthyle ou cyclohexèneméthyle, cyclohexényle, cyclohexényl-C₁-C₆-alkylène, de préférence cyclohexényl-CH₂-,
ou
R⁵ et R⁶ représentent avec l'atome d'azote un hétérocycle choisi dans le groupe consistant en pyrrolidine, pipéridine, 1.2.3.6-tétrahydropyridine et azépane ;
X représente l'oxygène, -NH-, -N(CHO)-, -N(CO-méthyle)-, -N(CO-éthyle)-, -N(méthyle)-, -N(éthyle)-, -N(propyle)-, -N(butyle)-, -N(pentyle)- ou -N(cyclopropylméthyle), de préférence l'oxygène ou NH- ;
A représente -CH₂-, -CH₂-CH₂- ou -CH₂-CH₂-CH₂-.

5. Composés de formule générale **1** selon l'une des revendications 1 à 4
où
R¹ représente l'hydrogène ou méthyle ;
R² et R³ représentent indépendamment l'un de l'autre l'hydrogène, méthyle, le fluor, le chlore ou le brome ;
R⁴ représente l'hydrogène, le fluor, le chlore ou le brome ;
R⁵ et R⁶ représentent indépendamment l'un de l'autre l'hydrogène, méthyle, propyle, butyle, hexyle, cyclopropylméthyle ou cyclohexèneméthyle,
ou
R⁵ et R⁶ représentent avec l'atome d'azote un hétérocycle choisi dans le groupe consistant en pyrrolidine, pipéridine, 1.2.3.6-tétrahydropyridine et azépane ;
X représente l'oxygène, -NH-, -N(CHO)-, -N(CO-méthyle)-, -N(CO-éthyle)-, -N(éthyle)-, -N(propyle)-, -N(butyle)-, -N(pentyle)- ou -N(cyclopropylméthyle), de préférence l'oxygène ou -NH- ;
A représente -CH₂-, -CH₂-CH₂- ou -CH₂-CH₂-CH₂-.

6. Composés de formule générale **1** selon l'une des revendications 1 à 5
où
R¹ représente l'hydrogène ou méthyle ; ,
R² et R³ représentent indépendamment l'un de l'autre l'hydrogène ou le fluor ;
R⁴ représente l'hydrogène ;
R⁵ et R⁶ représentent indépendamment l'un de l'autre l'hydrogène, méthyle, butyle, hexyle ou cyclohexèneméthyle,
ou
R⁵ et R⁶ représentent avec l'atome d'azote pipéridine ou 1.2.3.6-tétrahydropyridine ;
X représente l'oxygène ou -NH- ;
A représente -CH₂-CH₂- ou -CH₂-CH₂-CH₂-.

7. Composés de formule générale **1** selon l'une des revendications 1 à 6 où R¹ représente l'hydrogène, où R² et R³ sont disposés en position ortho.

8. Composés de formule générale **1** selon l'une des revendications 1 à 6 où R¹ représente méthyle et est situé en position para, et où R² et R³ sont disposés en position ortho.

9. Composés de formule générale **1** selon l'une des revendications 1 à 8 sous forme des différents isomères optiques, de mélanges des différents énantiomères ou de racémates ainsi que sous forme des bases libres ou des sels d'addition d'acide correspondants avec des acides pharmacologiquement acceptables.

10. Composés d'ammonium quaternaire de formule **1-Y** 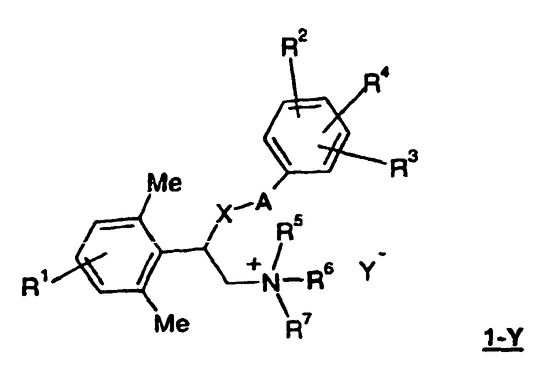 où les groupements A, X, R¹, R², R³ et R⁴ peuvent avoir les significations citées dans les revendications 1 à 8, R⁵ et R⁶ peuvent avoir les significations citées dans les revendications 1 à 8, mais pas l'hydrogène, R⁷ représente C₁-C₄-alkyle et Y représente un halogénure.

11. Composés de formule générale **1-Y** selon la revendication 10 où R¹ représente l'hydrogène, où R² et R³ sont disposés en position ortho.

12. Composés de formule générale **1-Y** selon la revendication 10 où R¹ représente méthyle et est situé en position para et où R² et R³ sont disposés en position ortho.

13. Préparation pharmaceutique **caractérisée par** une teneur en l'un des composés selon l'une des revendications 1 à 12 outre des adjuvants et supports habituels.

14. Préparation pharmaceutique selon la revendication 13 **caractérisée en ce qu'**elle est formulée sous forme de solution pour perfusion.

15. Utilisation de composés selon l'une des revendications 1 à 12 pour la production d'un médicament pour le traitement thérapeutique de troubles fonctionnels dus à une surexcitation.

16. Utilisation de composés selon l'une des revendications 1 à 12 pour la production d'un médicament pour le traitement thérapeutique des arythmies, des spasmes, des ischémies cardiaques et cérébrales, des douleurs et des maladies neurodégénératives de genèse diverse.

17. Utilisation selon la revendication 15 ou 16 pour la production d'un médicament pour le traitement thérapeutique de l'épilepsie, de l'hypoglycémie, de l'hypoxie, de l'anoxie, des traumatismes cérébraux, de l'oedème cérébral, de l'attaque cérébrale, de l'asphyxie périnatale, des dégénérescences du cervelet, de la sclérose latérale amyotrophique, de la maladie de Huntington, de la maladie d'Alzheimer, de la maladie de Parkinson, de la cyclothymie, de l'hypotonie, de l'infarctus cardiaque, des troubles du rythme cardiaque, de l'angine de poitrine, de la douleur chronique, de la douleur neuropathique ainsi que de l'anesthésie locale.

18. Procédé de préparation d'un composé de formule **1** 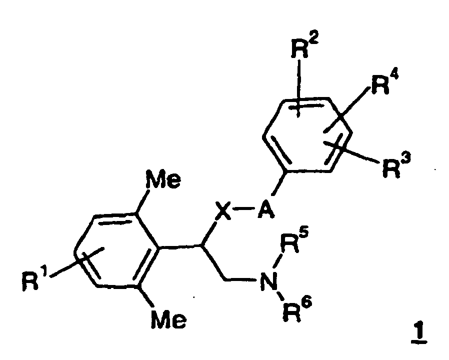 où les groupements A, R¹, R², R³, R⁴, R⁵ et R⁶ peuvent avoir les significations citées dans les revendications 1-8 et où X représente l'oxygène, **caractérisé en ce qu'**un composé de formule **6** 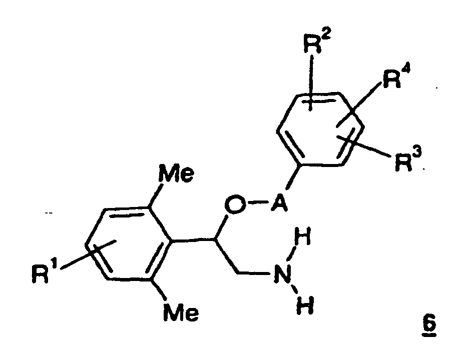 où les groupements A, R¹, R², R³ et R⁴ peuvent avoir les significations citées dans les revendications 1-8 est converti en un composé de formule **1** dans un solvant organique en présence d'une base inorganique ou organique avec un agent d'alkylation approprié, où le groupe alkyle présente les définitions citées dans les revendications 1 à 8 pour R⁵ et R⁶ ou bien où une amine de formule **6** est convertie en un composé de formule **1** par amination réductrice avec un composé carbonylé approprié en présence d'un réducteur.

19. Procédé selon la revendication 18 **caractérisé en ce que** le composé de formule **6** 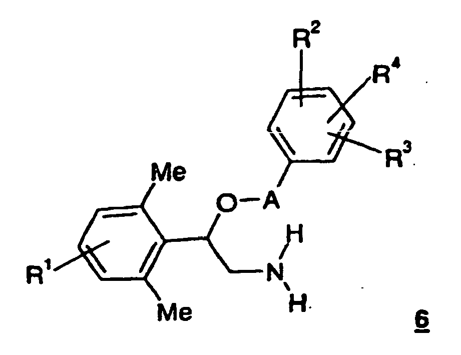 où les groupements A, R¹, R², R³ et R⁴ peuvent avoir les significations citées dans les revendications 1-8 est obtenu par le fait qu'un composé de formule **2** 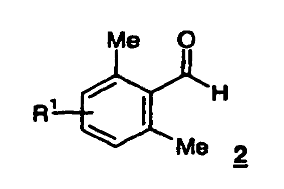 où R¹ peut avoir les significations citées dans les revendications 1 à 8, dans une première étape est repris dans le cyanure de triméthylsilyle en présence d'un acide de Lewis, le mélange obtenu est dilué au moyen d'un solvant organique anhydre approprié puis réduit au moyen d'un réducteur approprié en un composé de **3** 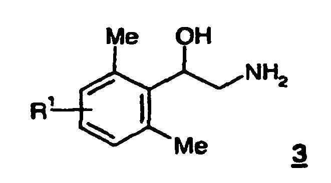 celui-ci, dans une seconde étape, éventuellement après séparation des énantiomères par reprise dans un solvant organique approprié en présence d'une base organique ou inorganique appropriée est converti au moyen de l'anhydride d'acide trifluoroacétique en un composé de formule **4** 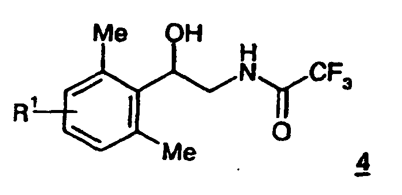 et celui-ci est enfin dissous dans un solvant organique approprié et converti en un composé de formule 6 en présence d'une base organique appropriée avec un composé de formule **5** éventuellement dissous dans un solvant organique approprié 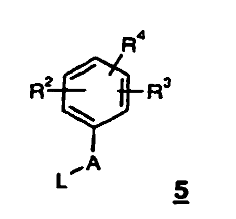 où les groupements R², R³ et R⁴ peuvent avoir les significations citées dans les revendications 1-8.

20. Procédé selon la revendication 18 **caractérisé en ce que** le composé de formule **6** 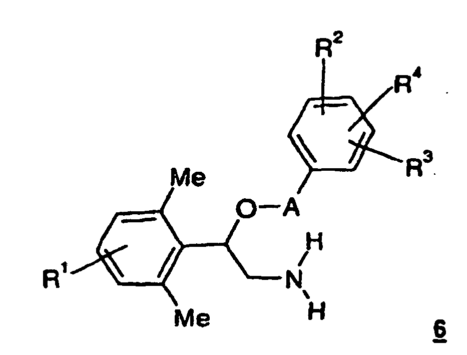 où les groupements A, R¹, R ² R³ et R⁴ peuvent avoir les significations citées dans les revendications 1-8 est obtenu par le fait qu'un composé de formule **2** 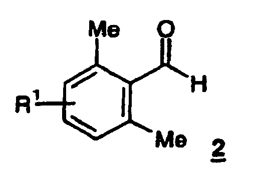 où R¹ peut avoir les significations citées dans les revendications 1 à 8, dans une première étape est converti au moyen du nitrométhane dans l'acide acétique glacial à haute température en un composé de formule **7** 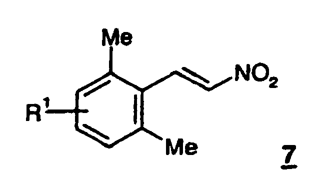 celui-ci est converti dans un solvant organique approprié au moyen d'un alcool **8** 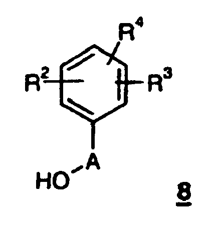 où les groupements R², R³ et R⁴ peuvent avoir les significations citées dans les revendications 1-8 en présence d'une base appropriée en un éther de formule **9** 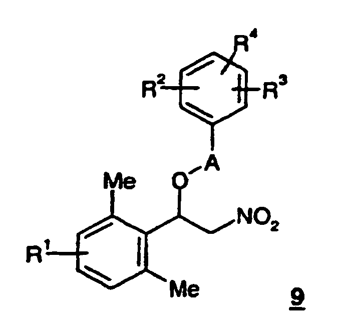 à partir duquel le composé de formule **6** peut être obtenu par réduction, de préférence par réduction catalysée par un métal.

21. Procédé de préparation de composés de formule **1** 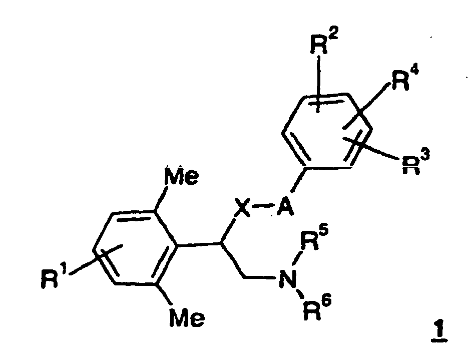 où les groupements A, R¹, R², R³, R⁴, R⁵ et R⁶ peuvent avoir les significations citées dans les revendications 1 à 8 et où X représente -NH-, **caractérisé en ce qu'**un composé de formule **3** 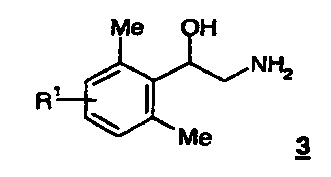 où le groupement R¹ peut avoir les significations citées dans les revendications 1-8 est converti dans un solvant organique approprié en présence d'une base inorganique ou organique appropriée au moyen d'un agent d'alkylation approprié dans lequel le groupe alkyle présente les définitions citées pour R⁵ dans les revendications 1 à 8 en un composé de formule **16** 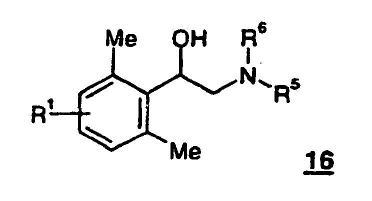 celui-ci, dans le cas où R⁵ ou R⁶ est l'hydrogène, est converti avec des groupes protecteurs appropriés, au moyen de réactifs d'halogénation appropriés, de chlorures d'acide sulfonique appropriés ou d'anhydrides d'acide sulfonique appropriés en présence de bases appropriées dans des solvants inertes appropriés en un composé de formule **17** 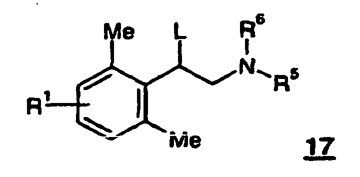 où L représente un groupe partant choisi parmi le chlore; le brome, l'iode, le méthanesulfonate, le trifluorométhanesulfonate et le para-toluènesulfonate, et celui-ci est converti en un composé de formule **1** dans un solvant organique approprié en présence d'une base inorganique ou organique appropriée au moyen d'un composé de formule **18** 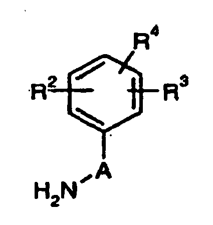 où les groupements R², R³ et R⁴ peuvent avoir les significations citées dans les revendications 1-8.

22. Procédé de préparation de composés de formule **1** 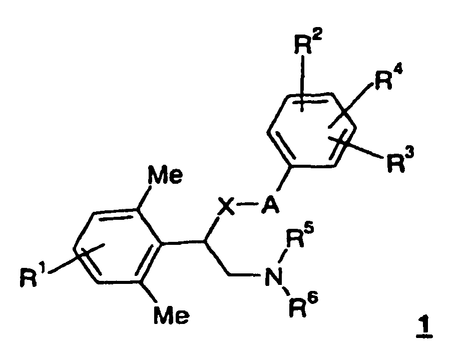 où les groupements A, R¹, R², R³, R⁴, R⁵ et R⁶ peuvent avoir les significations citées dans les revendications 1-8 et où X représente un groupe choisi parmi -N(CHO)-, -N(CO-C₁-C₆-alkyle)-, -N(C₁-C₆-alkyle)- et -N(C₃-C₆-cycloalkyl-C₁₋C₄-alkylène)-, **caractérisé en ce qu'**un composé de formule **1** où X représente -NH- est mis à réagir dans un solvant organique approprié en présence d'une base inorganique ou organique appropriée au moyen d'un agent d'alkylation, de formylation ou d'acylation approprié.
